# EUROPEAN PATENT APPLICATION

(11) **EP 1 972 631 A1**
(43) Date of publication of application: **24.09.2008**
(21) Application number: 07104782.3
(22) Date of filing: 23.03.2007
(51) Int. Cl.: C07D 487/04, A61K 31/5025, A61P 35/00, A61P 29/00

(54) **Imidazopyridazines as PI3K lipid kinase inhibitors**

(71) Applicant: Novartis AG, 4056 Basel (CH)
(72) Inventor: The designation of the inventor has not yet been filed
(74) Representative: Graff, Alan

(57) **Abstract**

The invention relates to novel compounds of the formula 1, 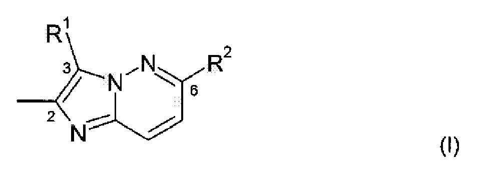 as well as other invention embodiments related to these compounds. The compounds are e.g. useful in the treatment of the animal or human body in view of their ability to inhibit protein kinases such as especially P13 kinase.

## Description

The invention relates to novel 3,6-disubstituted 2-methyl-imidazo[1,2-b]pyridazines, processes for the preparation thereof, these compounds for use in the treatment of the human or animal body, the use thereof - alone or in combination with one or more other pharmaceutically active compounds - for the treatment (this term including prophylactic and/or therapeutic treatment) of an inflammatory or obstructive airway disease, such as asthma, disorders commonly occurring in connection with transplantation, or a proliferative disease, such as a tumor disease, which may be solid or liquid, especially one or more of the mentioned diseases which respond to an inhibition of kinases of the PI3-kinase-related protein kinase family, especially lipid kinases and/or PI3 kinase (PI3K) and/or mTOR and/or DNA protein kinase and/or ATM and/or ATR and/or hSMG-1 activity; a method for the treatment of such a disease in animals, especially a human, and the use of such a compound - alone or in combination with one or more other pharmaceutically active compounds - for the manufacture of a pharmaceutical preparation for the treatment of said diseases in animals, especially a human.

The 3,6-disubstituted 2-methyl-imidazo[1,2-b]pyridazines preferably are compounds of the formula I, 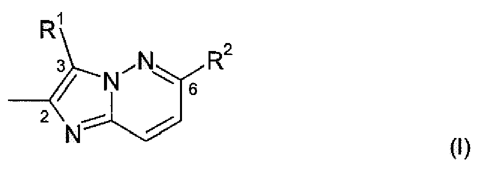 wherein
R¹ is unsubstituted or substituted aryl; especially substituted phenyl or substituted naphthyl; and
R² is substituted phenyl or substituted naphthyl;
or N-oxides thereof, solvates and/or (preferably pharmaceutically acceptable) salts thereof.

The general terms used hereinbefore and hereinafter preferably have within the context of this disclosure the following meanings, unless otherwise indicated, where more general terms whereever used may, independently of each other, be replaced by more specific definitions or remain, thus defining more preferred embodiments of the invention:

The prefix "lower" or "C₁-C₇-" denotes a radical having up to and including a maximum of 7, especially up to and including a maximum of 4 carbon atoms, the radicals in question being either linear or branched with single or multiple branching.

Lower alkyl (or C₁-C₇-alkyl) is preferably alkyl with from and including 1 up to and including 7, preferably from and including 1 to and including 4, and is linear or branched; preferably, lower alkyl is butyl, such as n-butyl, sec-butyl, isobutyl, tert-butyl, propyl, such as n-propyl or isopropyl, ethyl or preferably methyl.

The numbering of the positions of substituents at the central 2-methyl-imidazo[1,2-b]pyridazine ring system given in the present disclosure (e.g. in the Examples) is provided in formula 1 above by the small numbers 2, 3 and 6.

Halogen, halogeno (or halo) is especially fluoro, chloro, bromo, or iodo, especially fluoro, chloro or bromo.

In unsubstituted or substituted alkyl, alkyl preferably has up to 20, more preferably up to 12, carbon atoms (also in alkyloxy) and is especially C₁-C₇-alkyl; is linear or branched one or more times; and is unsubstituted or substituted (in any, e.g. the terminal position) by one or more moieties selected from the substituents mentioned below for aryl, especially from halo and cyano.

Mono- or disubstituted amino is preferably amino substituted by unsubstituted or substituted alkyl as defined above, by unsubstituted or substituted cycloalkyl as defined below or by acyl (then preferably only with one acyl), such as C₁-C₇-alkanoyl, C₁-C₇-alkyloxycarbonyl, phenyl-and/or naphtyl-C₁-C₇-alkoxycarbonyl; preferred is N-mono- or N,N-di-(C₁-C₇-alkyl, hydroxyl-C₁-C₇-alkyl, C₁-C₇-alkoxy-C₁-C₇-alkyl, phenyl-C₁-C₇-alkyl, naphthyl-C₁-C₇-alkyl, C₃-C₈-cycloalkyl, C₃-C₈-cycloalkyl-C₁-C₇-alkyl, C₃-C₇-alkanoyl, C₁-C₇-alkyloxycarbonyl, phenyl-and/or naphtyl-C₁-C₇-alkoxycarbonyl)-amino.

Mono- or di-substituted carbamoyl is preferably carbamoyl that is substituted by unsubstituted or substituted alkyl as defined above or by unsubstituted or substituted cycloalkyl as defined below; preferred is N-mono- or N,N-di-(C₁-C₇-alkyl, hydroxyl-C₁-C₇-alkyl, C₁-C₇-alkoxy-C₁-C₇-alkyl, phenyl-C₁-C₇-alkyl, naphthyl-C₁-C₇-alkyl, C₃-C₈-cycloalkyl and/or C₃-C₈-cycloalkyl-C₁-C₇-alkyl)-carbamoyl.

In unsubstituted or substituted heterocyclyl (also in unsubstituted or substituted heterocyclylcarbonyl), heterocyclyl is preferably a heterocyclic radical that is unsaturated (= carrying the largest possible number of conjugated double bonds in the ring(s), then heterocyclyl being heteroaryl), saturated or partially saturated and is preferably a monocyclic or in a broader aspect of the invention bicyclic or tricyclic ring; and has 3 to 24, more preferably 4 to 16, most preferably 4 to 10 and most preferably 6 ring atoms; wherein one or more, preferably one to four, especially one or two carbon ring atoms are replaced by a heteroatom selected from the group consisting of nitrogen, oxygen and sulfur, the bonding ring preferably having 4 to 12, especially 5 to 7 ring atoms; which heterocyclic radical (heterocyclyl) is unsubstituted or substituted by one or more, especially 1 to 3, substituents independently selected from the group consisting of the substituents defined below for substituted aryl; and where heterocyclyl is especially a heterocyclyl radical selected from the group consisting of oxiranyl, azirinyl, aziridinyl, 1,2-oxathiolanyl, thienyl (= thiophenyl), furanyl, tetrahydrofuryl, pyranyl, thiopyranyl, thianthrenyl, isobenzofuranyl, benzofuranyl, chromenyl, 2H-pyrrolyl, pyrrolyl, pyrrolinyl, pyrrolidinyl, imidazolyl, imidazolidinyl, benzimidazolyl, pyrazolyl, pyrazinyl, pyrazolidinyl, thiazolyl, isothiazolyl, dithiazolyl, oxazolyl, isoxazolyl, pyridyl, pyrazinyl, pyrimidinyl, piperidinyl, piperazinyl, pyridazinyl, morpholinyl, thiomorpholinyl, (S-oxo or S,S-dioxo)-thiomorpholinyl, indolizinyl, azepanyl, diazepanyl, especially 1,4-diazepanyl, isoindolyl, 3H-indolyl, indolyl, benzimidazolyl, cumaryl, indazolyl, triazolyl, tetrazolyl, purinyl, 4H-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, pyrrolo-pyrimidinyl, especially pyrrolo[2,3-d]pyrimidin-(e.g.1-)yl, 1H,4H,5H-trihydropyrazolo[2,3-c]piperidin-1-yl, pyrrolo-pyridinyl, e.g. pyrrolo[2,3-c]pyridine-1-yl (meaning 5-aza-indol-1-yl), quinoxalyl, quinazolinyl, quinazolinyl, cinnolinyl, pteridinyl, carbazolyl, beta-carbolinyl, phenanthridinyl, acridinyl, perimidinyl, phenanthrolinyl, furazanyl, phenazinyl, phenothiazinyl, phenoxazinyl, chromenyl, isochromanyl, chromanyl, benzo[1 ,3]dioxol-5-yl and 2,3-dihydro-benzo[1,4]dioxin-6-yl, each of these radicals being unsubstituted or substituted by one or more, preferably up to three, substituents selected from those mentioned above for substituted aryl and from oxo, especially from the group consisting of C₁-C₇-alkyl, hydroxy-C₁-C₇-alkyl, such as hydroxymethyl, C₁-C₇-alkoxy-C₁-C₇-alkyl, such as methoxymethyl, amino- or C₁-C₇-alkylamino-C₁-C₇-alkyl, halo, oxo, hydroxyl, C₁-C₇-alkoxy, amino, mono- or di-(C₁-C₇-alkyl and/or hydroxyl-C₁-C₇-alkyl)-amino, benzoylamino, aminobenzoylamino, C₁-C₇-alkoxycarbonylamino, (phenyl or naphthyl)-C₁-C₇-alkoxycarbonylamino, N-mono- or N,N-di-(C₁-C₇-alkyl and/or phenyl-C₁-C₇-alkyl)aminocarbonyl, pyridine-2-, -3- or -4-ylaminocarbonyl, phenylaminocarbonyl, thiazolylaminocarbonyl, N-[N'-mono- or N',N'-di-(C₁-C₇alkyl)-amino-C₁-C₇-alkyl]-aminocarbonyl and mono- or di-[C₁-C₇-alkoxy, pyrrolidino, piperidino, piperazino, thiazolyl (e.g. thiazol-5-yl), hydroxyl-C₁-C₇-alkylamino and/or N'-mono- or N',N'-di-(C₁-C₇-alkyl)-amino]-substituted phenyl-aminocarbonyl (especially in the case of unsubstituted or substituted heteocyclyl R¹ where the heterocyclyl substituents are preferably in the meta or para position relative to the binding aminocarbonyl group).

Unsubstituted or substituted heterocyclyl bound via an N-atom is preferably unsubstituted or substituted heterocyclyl as defined in the preceding paragraph which contains at least one nitrogen atom (which is preferably not charged without further protonation or N-oxide-formation) via which the respective moiety is bound to the rest of the molecule, especially one of the specific heterocyclyl moieties mentioned in the preceding paragraph wherein in the heterocyclic compound from which the moiety is formed by removal of a hydrogen from a ring NH a ring NH is present.

N-mono- or N,N-disubstituted sulfamoyl is preferably sulfmoyl that is substituted by unsubstituted or substituted alkyl as defined above or by unsubstituted or substituted cycloalkyl as defined below; preferred is N-mono- or N,N-di-(C₁-C₇)-alkyl, hydroxyl-C₁-C₇-alkyl, C₁-C₇-alkoxy-C₁-C₇-alkyl, phenyl-C₁-C₇-alkyl, naphthyl-C₁-C₇-alkyl, C₃-C₈-cycloalkyl, azetidine- and/or C₃-C₈-cycloalkyl-C₁-C₇-alkyl)-sulfamoyl.

Unsubstituted or substituted cycloalkyl is preferably a cycloalkyl which has 3 to 18, more preferably 3 to 10, most preferably 3 to 8 ring carbon atoms and is unsubstituted or substituted by one or more, especially up to 3, more preferably one or two, substitutents independently selected from those given below for substituted aryl.

In unsubstituted or substituted aryl, aryl preferably has 6 to 18 carbon atoms and is a mono-, di- or polycyclic (preferably up to tricyclic, more preferably up to bicyclic) unsaturated carbocyclic moiety with conjugated double bonds in the ring, especially phenyl, naphthyl, biphenylenyl, indacenyl, acenaphthylenyl, fluorenyl, phenalehyl, phenanthrenyl or anthracenyl. Naphthyl and preferably phenyl are especially preferred. Aryl is unsubstituted or (in the case of substituted aryl) substituted by one or more, e.g. one to three, substitutents preferably independently selected from the group consisting of C₁-C₇-alkyl, such as methyl, ethyl, n-propyl, isopropyl, n-butyl, isobutyl, sec-butyl or *tert-butyl;* C₂-C₇-alkenyl; C₂-C₇-alkinyl; C₆-C₁₈-aryl-C₁-C₇-alkyl in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), morpholino, thiomorpholino, pyridinyl, pyrimidinyl, pyrazinyl, pyridazinyl, oxazolyl or thiazolyl)-C₁-C₇-alkyl wherein pyrrolidinyl, piperidinyl, piperazinyl, pyridinyl, pyrimidinyl, pyrazinyl, pyridazinyl, oxazolyl or thiazolyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl, for example pyrrolidino-C₁-C₇-alkyl, piperidino-C₁-C₇-alkyl, morpholino-C₁-C₇-alkyl, thiomorpholino-C₁-C₇-alkyl, N-C₁-C₇-alkyl-piperazino-C₁-C₇-alkyl, or N-mono- or N,N-di-(C₁-C₇-alkyl)-amino-substituted or unsubstituted pyrrolidino-C₁-C₇-alkyl; (pyrrolidinyl (especially pyrrolidino), piperidinyl (especially piperidino), piperazinyl (especially piperazino), pyridinyl, pyrimidinyl, pyrazinyl, pyridazinyl, oxazolyl or thiazolyl)-oxy-C₁-C₇-alkyl wherein pyrrolidinyl, piperidinyl, piperazinyl, pyridinyl, pyrimidinyl, pyrazinyl, pyridazinyl, oxazolyl and thiazolyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl; such as trifluoromethyl; (pyrrolidin (especially pyrrolidino), piperidin (especially piperidino), piperazin (especially piperazino), pyridin, pyrimidin, pyrazin, pyridazin, oxazoly or thiazol)-carbonyl-C₁-C₇-alkyl wherein pyrrolidin, piperidin, piperazin, pyridin, pyrimidin, pyridazin, oxazol or pyridazin are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; halo-C₁-C₇-alkyl, such as trifluoromethyl; hydroxy-C₁-C₇-alkyl, such as hydroxymethyl; C₁-C₇-alkoxy-C₁-C₇-alkyl, such as 3-methoxypropyl or 2-methoxyethyl; C₁-C₇-alkoxy-C₁-C₇-alkoxy-C₁-C₇-alkyl; phenyloxy- or naphthyloxy-C₁-C₇-alkyl; phenyl-C₁-C₇-alkoxy-or naphthyl-C₁-C₇-alkoxy-C₁-C₇-alkyl; amino-C₁-C₇-alkyl, such as aminomethyl; N-mono- or N,N-di-(C₁-C₇-alkyl, C₁-C₇-alkoxy-C₁-C₇-alkyl and/or (mono- or di-(C₁-C₇-alkyl)-amino)-C₁-C₇-alkyl)-amino-C₁-C₇-alkyl; C₁-C₇-alkoxy-C₁-C₇-alkylamino-C₁-C₇-alkyl; mono- or di-[C₆-C₁₈-aryl-C₁-C₇-alkyl in which aryl is preferably phenyl, naphthyl, biphenylenyl, indacenyl, acenaphthylenyl, fluorenyl, phenalenyl, phenanthrenyl or anthracenyl and unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; naphthyl- or phenyl-C₁-C₇-alkyl]-amino-C₁-C₇-alkyl; C₁-C₇-alkanoylamino-C₁-C₇-alkyl; carboxy-C₁-C₇-alkyl; benzoyl- or naphthoylamino-C₁-C₇-alkyl; C₁-C₇-alkylsulfonylamino-C₁-C₇-alkyl; phenyl- or naphthylsulfonylamino-C₁-C₇-alkyl wherein phenyl or naphthyl is unsubstituted or substituted by one or more, especially one to three, C₁-C₇-alkyl moieties; phenyl- or naphthyl-C₁-C₇-alkylsulfonylamino-C₁-C₇-alkyl; cyano-C₁-C₇-alkyl; halo, especially fluoro (preferred), chloro (preferred) or bromo; hydroxy; C₁-C₇-alkoxy; C₆-C₁₈-aryl-C₁-C₇-alkoxy in which aryl is preferably phenyl, naphthyl, biphenylenyl, indacenyl, acenaphthylenyl, fluorenyl, phenalenyl, phenanthrenyl or anthraxcenyl and unsubstituted or substituted by C₁-C₇-alkyl, such as methyl or ethyl, by C₁-C₇-alkoxy, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; such as phenyl-C₁-C₇-alkoxy wherein phenyl is unsubstituted or substituted by C₁-C₇-alkoxy and/or halo; halo-C₁-C₇-alkoxy, such as trifluoromethoxy; hydroxy-C₁-C₇-alkoxy; C₁-C₇-alkoxy-C₁-C₇-alkoxy, such as 2-(methoxy)-ethoxy; amino-C₁-C₇-alkoxy, N-C₁-C₇-alkanoylamino-C₁-C₇-alkoxy; N-unsubstituted-, N-mono- or N,N-di-(C₁-C₇-alkyl)carbamoyl-C₁-C₇-alkoxy; phenyl- or naphthyloxy; phenyl- or naphthyl-C₁-C₇-alkyloxy; (pyrrolidinyl (especially pyrrolidino), piperidinyl (especially piperidino), piperazinyl (especially piperazino), pyridinyl, pyrimidinyl, pyrazinyl, pyridazinyl, oxazolyl or thiazolyl)-C₁-C₇-alkoxy wherein pyrrolidinyl, piperidinyl, piperazinyl, pyridinyl, pyrimidinyl, pyrazinyl, pyridazinyl, oxazolyl and thiazolyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; (pyrrolidinyl (especially pyrrolidino), piperidinyl (especially piperidino), piperazinyl (especially piperazino), pyridinyl, pyrimidinyl, pyrazinyl, pyridazinyl, oxazolyl or thiazolyl)-oxy-C₁-C₇-alkoxy wherein pyrrolidinyl, piperidinyl, piperazinyl, pyridinyl, pyrimidinyl, pyrazinyl, pyridazinyl, oxazolyl and thiazolyl are unsubstituted or substituted by C₁-C₇-alkyl, such as methyl or ethyl, by pyrrolidinyl, especially pyrrolidino, by piperazinyl, especially piperazino, by amino, by N-mono- and/or N,N-di-C₁-C₇-alkylamino, by halo, by C₁-C₇-alkoxy, such as methoxy, and/or by halo-C₁-C₇-alkyl, such as trifluoromethyl; C₁-C₇-alkanoyloxy; benzoyl- or naphthoyloxy; C₁-C₇-alkylthio; halo-C₁-C₇-alkylthio, such as trifluoromethylthio; C₁-C₇-alkoxy-C₁-C₇-alkylthio; phenyl- or naphthylthio; phenyl- or naphthyl-C₁-C₇-alkylthio; C₁-C₇-alkanoylthio; benzoyl- or naphthaylthio; nitro; amino; mono- or di-(C₁-C₇-alkyl, C₃-C₈-cyloalkyl and/or hydroxyl-C₁-C₇-alkyl)-amino; mono- or di-(naphthyl- or phenyl-C₁-C₇-alkyl)-amino; C₁-C₇-alkanoylamino; unsubstituted or amino-, N-mono- or N,N-di-(C₁-C₇-alkyl and/or phenyl- or naphthyl-C₁-C₇alkyl)amino-substituted benzoyl- or naphthoylamino; C₁-C₇-alkoxycarbonylamino; (phenyl or naphthyl)-C₁-C₇-alkoxycarbonylamino; C₁-C₇-alkylsulfonylamino; phenyl- or naphthylsulfonylamino wherein phenyl or naphthyl is unsubstituted or substituted by one or more, especially one to three, C₁-C₇-alkyl moieties; phenyl-or naphthyl-C₁-C₇-alkylsulfonylamino; C₁-C₇-alkanoyl; C₁-C₇-alkoxy-C₁-C₇-alkanoyl; carboxyl (-COOH); C₁-C₇-alkoxy-carbonyl; phenoxy- or naphthoxycarbonyl; phenyl- or naphthyl-C₁-C₇-alkoxycarbonyl; C₁-C₁₀- especially C₁-C₄-alkylendioxy, such as methylendioxy or 1,2-ethylendioxy; carbamoyl; N-mono- or N,N-di-[C₁-C₇-alkyl, naphthyl-C₁-C₇-alkyl, phenyl-C₁-C₇-alkyl, N'-mono- or N',N'-di-(C₁-C₇alkyl)amino-C₁-C₇-alkyl, pyrrolidinyl(especially pyrrolidino)-C₁-C₇-alkyl, piperidinyl (especially piperidino)-C₁-C₇-alkyl, piperazinyl- or N-(C₁-C₇-alkyl)piperazinyl(especially piperazino or 4-C₁-C₇-alkylpiperazino)-C₁-C₇-alkyl, mono-C₁-C₇-alkoxy-C₁-C₇-alkyl, (N'-mono- or N',N'-di-(C₁-C₇-alkyl)-amino); phenyl, pyridinyl, oxazolyl or thiazolyl each of which is unsubstituted or substituted by C₁-C₇-alkoxy, by halo, especially fluoro, by pyrrolidino, by piperidino, by piperazino, by hydroxyl-C₁-C₇-alkylamino, by hydroxyl-C₁-C₇-alkyl, by amino or by N-mono- or N,N-di-(C₁-C₇-alkyl)amino; C₃-C₈-cyloalkyl, pyrrolidinyl, piperidinyl, morpholinyl, piperazinyl, pyrimidinyl, pyrazinyl and/or pyridazinyl]-amino-carbonyl, such as N- mono- or N,N-di-(C₁-C₇-alkyl)-aminocarbonyl; N-C₁-C₇-alkoxy-C₁-C₇-alkylcarbamoyl; pyrrolidin-1-carbonyl; amino-N-pyrrolidin-1-carbonyl; N-mono- or N,N-di(C₁-C₇-alkyl)amino-pyrrolidin-1-carbonyl; piperidin-1-carbonylmorpholin-4-carbonyl; morpholinocarbonyl, thiomorpholinocarbonyl, S-oxo- or S,S-dioxo-thiomorpholino-carbonyl, thiomorpholin-4-carbonyl; S-oxo-thiomorpholin-4-carbonyl; S,S-dioxothiomorpholin-4-carbonyl; piperazin-1-carbonyl; N-C₁-C₇-alkyl-piperazin-1-carbonyl; N-C₁-C₇-alkoxycarbonyl-piperazin-1-carbonyl; N-mono- or N,N-di-(C₁-C₇-alkyl)-amino-substituted or unsubstituted pyrrolidinyl-C₁-C₇-alkyl-carbonyl; cyano; C₁-C₇-alkenylene or -alkinylene; C₁-C₇-alkylsulfonyl (= C₁-C₇-lakane-sulfonyl); phenyl- or naphthylsulfonyl wherein phenyl or naphthyl that is unsubstituted or substituted by one or more, especially one to three, C₁-C₇-alkyl moieties; phenyl- or naphthyl-C₁-C₇-alkylsulfonyl; sulfamoyl; N-mono or N,N-di-(C₁-C₇-alkyl, phenyl-, naphthyl-, phenyl-C₁-C₇-alkyl-, pyrrolidinyl(especially pyrrolidino)-C₁-C₇-alkyl, piperidinyl(especially piperidino)-C₁-C₇-alkyl, piperazinyl (especially piperazino)-C₁-C₇-alkyl, N-C₁-C₇-alkyl-piperazinyl(especially 4-C₁-C₇-alkylpiperazino)-C₁-C₇-alkyl, naphthyl-C₁-C₇-alkyl, phenyl which is unsubstituted or substituted by C₁-C₇-alkoxy, by halo, especially fluoro, by pyrrolidino, by piperidino, by piperazino, by hydroxyl-C₁-C₇-alkyl or by N-mono- or N,N-di-(C₁-C₇-alkyl)-C₁-C₇-alkyl; pyrrolidinyl (especially pyrrolidino), piperidinyl (especially piperidino), piperazinyl (especially piperazino), pyridinyl, pyrimidinyl, pyrazinyl, pyridazinyl, oxazolyl and/or thiazolyl)-aminosulfonyl, pyrazolyl, pyrazolidinyl, pyrrolyl, pyridyl that is unsubstituted or substituted by C₁-C₇-alkoxy, such as methoxy, and/or by halo-C₁-C₇-alkyl, such as trifluoromethyl, pyrrolidinyl, such as pyrrolidin-1-yl, oxo-pyrrolidinyl, such as 2-oxo-pyrrolidin-1-yl, piperidinyl, oxo-piperidinyl, such as 2-oxopiperidin-1-yl , morpholinyl, such as morpholino, thiomorpholinyl, such as thiomorpholino, S-oxo-thiomorpholinyl, such as S-oxo-thiomorpholino, S,S-dioxothiomorpholinyl, such as S,S-dioxo-thiomorpholino, piperazinyl, N-C₁-C₇-alkyl-piperazinyl, 4-(phenyl-C₁-C₇-alkyl)-piperazinyl, 4-(naphthyl-C₁-C₇-alkyl)-piperazinyl, 4-(C₁-C₇-alkoxycarbonyl)-piperazinyl, 4-(phenyl-C₁-C₇-alkoxycarbonyl)-piperazinyl, 4-(naphthyl-C₁-C₇-alkoxycarbonyl)-piperazinyl, oxazolyl and thiazolyl, triazolyl, e.g. 1,2,4-triazol-1-yl, carbamoyl-triazolyl, e.g. carbamoyl-1,2,4-triazol-1-yl, such as 3-carbamoyl-1,2,4-triazol-1-yl, pyrazolyl, such as pyrazol-1-yl, halo-C₁-C₇alkyl-pyrazolyl, such as 3-trifluoromethyl-pyrazol-1-yl, halophenyl-pyrazolyl, such as 3-(halophenyl)-pyrazol-1-yl, e.g. 3-(4-chlorophenyl)-pyrazol-1-yl, pyridine-(2-, 3- or 4-)yl, pyrimidin-(2-, 4- or 5-)yl, benzimidazol(especially -1-)y, (e.g. 5-)C₁-C₇-alkoxy-substituted benzimidazol(especially-1-)yl, pyrrolo-pyrimidinyl, especially pyrrolo[2,3-d]pyrimidin-(e.g.1-)yl, C₁-C₇-alkyl-substituted pyrrolo-pyrimidinyl, e.g. 2-C₁-C₇-alkyl-pyrrolo[2,3-d]pyrimidin-(e.g.1-)yl (meaning 2-C₁-C₇-alkyl-5,7-diazaindol-1-yl), 1H,4H,5H-trihydropyrazolo[2,3-c]piperidin-1-yl (meaning 5-aza-3,4,5,6-tetrahydroindazol-1-yl) which is unsubstituted or substituted by 1 or 2 substituents independently selected from C₁-C₇-alkyl (e.g. methyl, especially in 5-position) and halo-C₁-C₇-alkyl (e.g. trifluoromethyl, especially in 3-position), and further from C₃-C₈-cycloalkyl, phenyl or naphthyl each of which is unsubstituted or substituted by one or more, e.g. up to 2, moieties independently selected from the group consisting of halo, C₁-C₇-alkoxy, C₁-C₇-alkanesulfonyl, nitro and cyano; tetrazolyl, e.g. tetrazol-5-yl, indol-(e.g.5-)yl, indazolyl, e.g. indazol-5-yl, (e.g. 3-) C₁-C₇-alkyl-indazoyl-(e.g. 5-)yl and pyrrolo-pyridinyl, e.g. pyrrolo[2,3-c]pyridine-1-yl (meaning 5-aza-indol-1-yl); especially preferably aryl is phenyl or naphthyl, each of which is unsubstituted or substituted as just described, more preferably by one or more, e.g. up to three, substituents independently selected from those mentioned above.

Substituted phenyl or substituted naphthyl (especially as R²) is especially phenyl or naphthyl, more preferably phenyl, each of which is substituted by one or more moieties mentioned above for aryl, more preferably by one to three, more preferably one or two or further three, moieties independently selected from the group consisting of halo, especially fluoro or further chloro, C₁-C₇-alkoxy (very preferred), especially methoxy, hydroxyl, C₁-C₇-alkoxy-C₁-C₇-alkoxy, especially 2-methoxyethoxy, 2-ethoxyethoxy, 2- or 3-methoxypropoxy, 2- or 3-ethoxypropoxy or 2- or 3-propoxypropoxy, C₁-C₇-alkoxy-C₁-C₇-alkoxy-C₁-C₇-alkoxy, such as 2-(2-methoxyethoxy or 2-ethoxyethoxy)-ethoxy, amino-C₁-C₇-alkoxy, N-mono- or N,N-di-(C₁-C₇-alkyl)-amino-C₁-C₇-alkoxy, e.g. 2-dimethyl- or 2-diethyl-amino-ethoxy or 2- or 3-dimethyl- or 2- or 3-diethyl-amino-propoxy, pyrrolidinyl-C₁-C₇-alkoxy, piperidinyl-C₁-C₇-alkoxy, e.g. piperidino-C₁-C₇-alkxoy, morpholinyl-C₁-C₇-alkoxy, e.g. morpholino-C₁-C₇-alkoxy, thiomorpholinyl-C₁-C₇-alkoxy, e.g. thiomorpholino-C₁-C₇-alkoxy, S-oxo-thiomorpholinyl-C₁-C₇-alkoxy, e.g. S-oxothiomorpholino-C₁-C₇-alkoxy, S,S-dioxothiomorpholinyl-C₁-C₇-alkoxy, e.g. S,S-dioxo-thiomorpholino-C₁-C₇-alkoxy, piperazinyl-C₁-C₇-alkoxy, e.g. piperazino-C₁-C₇-alkoxy, N'-C₁-C₇-alkyl-piperazino-C₁-C₇-alkoxy, C₃-C₈-cyloalkoxy, C₁-C₇-alkane-sulfonyl, e.g. methane- or ethanesulfonyl, and C₃-C₈-cyloalkyl-sulfonyl, more especially in the meta- and/or (most especially) the para-position.

Preferably, aryl R¹ carries at least one substituent (especially as defined in the last paragraph) in p-position and a methoxy in meta-position.

Generally, in the case of R¹ substituents in substituted aryl, substituted heterocyclyl and substituted cycloalkyl can be in the ortho- or preferably the meta- or para-position in the case of six-membered cycles, generally in position 2 or preferably 3 or (especially) 4 relative to the atom binding to the rest of the molecule.

An N-oxide derivative or pharmaceutically acceptable salt of each of the compounds of the formula I is also within the scope of this invention. For example, a nitrogen ring atom of a nitrogen-containing heterocyclic (e.g. heteroaryl) can form an N-oxide in the presence of a suitable oxidizing agent, e.g. a peroxide, such as m-chloro-perbenzoic acid or hydrogen peroxide.

Wherever a compound or compounds of the formula I are mentioned, this is further also intended to include one or more N-oxides of such compounds, also where not stated explicitly.

The term "an N-oxide thereof, a solvate thereof and/or a pharmaceutically acceptable salt thereof' especially means that a compound of the formula I may be present as such or in mixture with its N-oxide or as essentially pure N-oxide, as a solvate of the compound or the N-oxide, or as a salt of the compound of the formula I or an N-oxide thereof, or as a solvate of such salt and/or N-oxide, either each of these forms in essentially pure form or as a mixture with one or more of the other forms.

Compounds of the formula I can also be modified by appending appropriate functionalities to enhance selective biological properties. Modifications of this kind are known in the art and include those that increase penetration into a given biological system (e.g. blood, lymphatic system, central nervous system, testis), increase bioavailability, increase solubility to allow parenteral administration (e.g. injection, infusion), alter metabolism and/or alter the rate of secretion. Examples of this type of modifications include but are not limited to esterification, e.g. with polyethylene glycols, derivatisation with pivaloyloxy or fatty acid substituents, conversion to carbamates, hydroxylation of aromatic rings and heteroatom substitution in aromatic rings. Whereever compounds of the formula I, N-oxides, solvates and/or (especially pharmaceutically acceptable) salts thereof are mentioned, this comprises such modified formulae, while preferably the molecules of the formula I, N-oxides, solvates and/or (especially pharmaceutical acceptable) salts thereof as such are meant.

In view of the close relationship between the novel compounds of the formula I in free form and those in the form of their salts, including those salts that can be used as intermediates, for example in the purification or identification of the novel compounds, any reference to the compounds or a compound of the formula I hereinbefore and hereinafter is to be understood as referring also to one or more salts, as appropriate and expedient, as well as to one or more solvates, e.g. hydrates.

Solvate means a (at least partially) crystalline compound of the formula I or a salt thereof in crystalline form with solvent molecules included in the crystal structure - the term solvate here includes hydrates (crystals including, water molecules) and/or any other (preferably pharmaceutically acceptable) solvates with one or more other solvents.

Salts are formed, for example, as acid addition salts, preferably with organic or inorganic acids, from compounds of formula I with a basic nitrogen atom, especially the pharmaceutically acceptable salts. Suitable inorganic acids are, for example, halogen acids, such as hydrochloric acid, sulfuric acid, or phosphoric acid. Suitable organic acids are, for example, carboxylic, phosphonic, sulfonic or sulfamic acids, for example acetic acid, propionic acid, octanoic acid, decanoic acid, dodecanoic acid, glycolic acid, lactic acid, fumaric acid, succinic acid, malonic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantanecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 4-toluenesulfonic acid, 2-naphthalenesulfonic acid, 1,5-naphthalene-disulfonic acid, 2- or 3-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

For isolation or purification purposes it is also possible to use pharmaceutically unacceptable salts, for example picrates or perchlorates. For therapeutic use, only pharmaceutically acceptable salts or free compounds are employed (where applicable in the form of pharmaceutical preparations), and these are therefore preferred.

Preferred is a compound of the formula I, wherein
R¹ is phenyl that is unsubstituted or substituted by by one or more, especially one or two or three, more preferably by two, moieties selected from the group consisting of unsubstituted or substituted alkyl, such as C₁-C₇-alkyl that is unsubstituted or substituted by hydroxyl or cyano-C₁-C₇-alkyl, halo, hydroxyl, alkyloxy, especially C₁-C₇-alkoxy, especially methoxy, amino, mono- or disubstituted amino, preferably N-mono- or N,N-di-(C₁-C₇-alkyl and/or C₃-C₈-cyloalkyl)-amino, especially N-methylamino, C₁-C₇-alkanoylamino, C₁-C₇-alkoxycarbonylamino, phenyl- or naphthyl-C₁-C₇-alkoxycarbonyl-amino, carbamoyl, mono- or disubstituted carbamoyl, preferably N-mvno- or N,N-di-(C₁-C₇-alkyl and/or C₃-C₈-cycloalkyl)-carbamoyl, pyrrolidine-2-carbonyl-amino, heterocyclylcarbonyl where heterocyclyl is bound via a ring nitrogen to the carbonyl, especially piperidinocarbonyl, morpholino-carbonyl, thiomorpholino-carbonyl or S-oxo- or S,S-dioxothiomorpholinocarbonyl, C₁-C₇-alkanesulfonyl, sulfamoyl, N-mono- or N,N-disubstituted sulfamoyl, preferably N-mono- or N,N-di-(C₁-C₇-alkyl)-sulfamoyl, C₃-C₈-cycloalkyl-sulfamoyl, azetidine-sulfamoyl, hydroxy-(C₁-C₇-alkyl)-sulfamoyl, cyano, nitro, unsubstituted or substituted heterocyclyl bound via a ring carbon atom or preferably a ring nitrogen atom, especially 1,2,4-triazol-1-yl, carbamoyl-1,2,4-triazol-1-yl, pyrazol-1-yl, 3-trifluoromethyl-pyrazol-1-yl, 3-(halophenyl)-pyrazol-1-yl, e.g. 3-(4-chlorophenyl)-pyrazol-1-yl, pyrrofidin-1-yl, 2-oxo-pyrrolidin-1-yl, piperidin-1-yl, 2-oxopiperidin-1-yl, morpholino, pyridinyl which is unsubstituted or substituted by cyano, C₁-C₇-alkyl or amino, pyrimidinyl, pyrazinyl, benzimidazolyl, C₁-C₇-alkoxy-substituted benzimidazolyl, benzothiazolyl, amino-substituted benzothiazolyl, pyrrolo-pyrimidinyl, especially pyrroio[2,3-d]pyrimidinyl, C₁-C₇-alkylsubstituted pyrrolo-pyrimidinyl, e.g. 2-C₁-C₇-alkyl-pyrrolo[2,3-d]pyrimidin-yl, and 1H,4H,5H-trihydropyrazolo[2,3-c]piperidin-1-yl which is unsubstituted or substituted by 1 or 2 substituents independently selected from C₁-C₇-alkyl and halo-C₁-C₇-alkyl, and/or further from unsubstituted or substituted aryl, from unsubstituted or substituted cycloalkyl and from unsubstituted or substituted heterocyclyl, especially from phenyl that is unsubstituted or substituted by one or more, e.g. up to 2, moieties independently selected from the group consisting of halo, C₁-C₇-alkoxy and C₁-C₇-alkanesulfonyl, from tetrazol-5-yl, from indolyl, from indazolyl, from C₁-C₇-alkyl-indazoylyl from pyrrolo-pyridinyl and from azetidin-2-one; and
R² is phenyl that is substituted by 1 to 3, preferably 1 or 2 (especially in meta- and/or para-position) substituents selected from the group consisting of halo, especially fluoro, C₁-C₇-alkoxy (very preferred), especially methoxy, hydroxyl, C₁-C₇-alkoxyphenyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, C₁-C₇-alkoxycarbonyl, amino-C₁-C₇-alkoxy, pyrrolidinyl-C₁-C₇-alkoxy, piperidinyl-C₁-C₇-alkoxy, morpholinyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl and C₃-C₈-cyloalkyl-sulfonyl;
   preferably with the proviso that phenyl R² is substituted in meta position by C₁-C₇-alkoxy, especially methoxy, and in para-position by C₁-C₇-alkoxy, especially methoxy, hydroxyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, pyrrolidinyl-C₁-C₇-alkoxy, piperidinyl-C₁-C₇-alkoxy, morpholinyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl or C₃-C₈-cyloalkyl-sulfonyl; where in one more preferred embodiment R² is 3,4-dimethoxyphenyl;
or an N-oxide thereof, a solvate and/or a (preferably pharmaceutically acceptable) salt thereof.

More preferred is a compound of the formula I, wherein
R¹ is phenyl that is unsubstituted or substituted (preferably in the 3- (meta) and/or 4- (para) position of the phenyl) by one or more, especially one or two or further three, more preferably by one or two, moieties selected from the group consisting of hydroxyl-C₁-C₇-alkyl, cyano-C₁-C₇-alkyl, halo, C₁-C₇-alkoxy, amino, N-mono- or N,N-di-(C₁-C₇-alkyl and/or C₃-C₈-cyloalkyl)-amino, C₁-C₇-alkanoylamino, C₁-C₇-alkoxycarbonyl-amino, phenyl- or naphthyl-C₁-C₇-alkoxycarbonyl-amino, carbamoyl, N-mono- or N,N-di-(C₁-C₇-alkyl and/or C₃-C₈-cycloalkyl)-carbamoyl, pyrrolidine-2-carbonyl-amino, piperidinocarbonyl, morpholino-carbonyl, thiomorpholino-carbonyl, S-oxo- or S,S-dioxothiomorpholinocarbonyl, C₁-C₇-alkanesulfonyl, sulfamoyl, N-mono- or N,N-di-(C₁-C₇-alkyl)-sulfamoyl, C₃-C₈-cycloalkyl-sulfamoyl, azetidine-sulfamoyl, hydroxy-(C₁-C₇-alkyl)-sulfamoyl, cyano, nitro, 1,2,4-triazol-1-yl, carbamoyl-1,2,4-triazol-1-yl, such as 3-carbamoyl-1,2,4-triazol-1-yl, pyrazol-1-yl, 3-trifluoromethyl-pyrazol-1-yl, 3-(halophenyl)-pyrazol-1-yl, e.g. 3-(4-chlorophenyl)-pyrazol-1-yl, pyrrolidin-1-yl, 2-oxo-pyrrolidin-1-yl, piperidin-1-yl, 2-oxopiperidin-1-yl, morpholino, pyridine-(2-, 3- or 4-)yl which is unsubstituted or substituted by cyano, C₁-C₇-alkyl or amino, pyrimidin-(2-, 4- or 5-)yl, pyrazinyl, benzimidazolyl, C₁-C₇-alkoxy-substituted benzimidazolyl, benzothiazolyl, amino-substituted benzothiazolyl, pyrrolo-pyrimidinyl, especially pyrrolo[2,3-d]pyrimidin-yl, C₁-C₇-alkyl-substituted pyrrolo-pyrimidinyl and 1H,4H,5H-trihydropyrazolo[2,3-c]piperidin-1-yl which is unsubstituted or substituted by 1 or 2 substituents independently selected from C₁-C₇-alkyl and halo-C₁-C₇-alkyl, or further from phenyl that is unsubstituted or substituted by one or more moieties independently selected from the group consisting of halo, C₁-C₇-alkoxy and C₁-C₇-alkanesulfonyl, from tetrazol-5-yl, from indolyl, from indazolyl, from C₁-C₇-alkyl-indazoylyl, from pyrrolo-pyridinyl and from azetidin-2-one; and
R² is phenyl that is substituted by 1 to 3, preferably 1 or 2 substituents selected from the group consisting of halo, C₁-C₇-alkoxy, hydroxyl, C₁-C₇-alkoxyphenyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, C₁-C₇-alkoxycarbonyl, amino-C₁-C₇-alkoxy, pyrrolidinyl-C₁-C₇-alkoxy, piperidinyl-C₁-C₇-alkoxy, morpholinyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇ alkoxy, N'-C₁-C₇-alkyl-piperazino-C₁-C₇-alkoxy, C₃-C₈-cyloalkoxy, C₁-C₇-alkane-sulfonyl and C₃-C₈-cyloalkyl-sulfonyl;
   preferably with the proviso that phenyl R² is substituted in meta position by C₁-C₇-alkoxy, especially methoxy, and in para-position by C₁-C₇-alkoxy, hydroxyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, pyrrolidinyl-C₁-C₇-alkoxy, piperidinyl-C₁-C₇-alkoxy, morpholihyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl or C₃-C₈-cyloalkyl-sulfonyl; where in one preferred embodiment R² is 3,4-dimethoxyphenyl;
   or an N-oxide thereof, a solvate and/or a (preferably pharmaceutically acceptable) salt thereof.

Even more preferred is a compound of the formula I, wherein
R¹ is phenyl, (especially 3- or 4-) halophenyl, (especially 3- or 4-) C₁-C₇-alkoxyphenyl, halo- and C₁-C₇-alkoxy-substituted phenyl, especially 3-halo-4-C₁-C₇-alkoxyphenyl or 4-halo-3-C₁-C₇-alkoxyphenyl, halo-, C₁-C₇-alkoxy- and C₁-C₇-alkyl-substituted phenyl, such as 2-halo-4-C₁-C₇-alkoxy-5-C₁-C₇-alkyl-phenyl, (especially 3- or 4-) aminophenyl, pyrrolidin-2-carbonylamino-phenyl, (especially 3- or 4-) mono- or di-(C₁-C₇-alkyl)phenyl, (especially 3- or 4-) C₁-C₇-alkanesulfonyl-phenyl, azetidine-1-sulfonyl-phenyl, (especially 3- or 4-) sulfamoyl-phenyl, (especially 3- or 4-) N-mono-(C₁-C₇-alkyl)-sulfamoyl-phenyl, cyclopropyl-sulfamoyl-phenyl, 2-hydroxy-ethyl-sulfamoyl-phenyl, (especially 3-or 4-) cyanophenyl, (especially 3- or 4-) nitrophenyl, (especially 4-) 1,2,4-triazol-1-yl-phenyl, (especially 4-) pyrazol-1-yl-phenyl, (especially 4-) (3-trifluaromethyl-pyrazol-1-yl)-phenyl, (especially 4-) 2-oxo-pyrrolidin-1-yl-phenyl, (especially 4-) 2-oxopipeddin-1-yl-phenyl, (especially 4-) morpholino-phenyl, (especially 3- or 4-)piperazin-1-yl-phenyl, (especially 3- or 4-) 4-(C₁-C₇-alkyl)-piperazin-1-yl-phenyl, (especially 4-) pyridine-(2-, 3- or 4-)yl-phenyl, cyano-pyridyl-phenyl, methyl-pyridyl-phenyl, amino-pyridyl-phenyl, (especially 4-) pyrimidin-(2-, 4- or 5-)yl-phenyl, pyrazinyl-phenyl, azetidin-2-one-phenyl, or (especially 3- or 4-)benzimidazol-1-yl-phenyl, and
R² is 4-methane-sulfonylphenyl, 4'-methoxy-biphenyl, 4-(3-amino-propoxy)-3-methoxyphenyl, 3,4-di-C₁-C₇-alkoxy-phenyl, especially 3,4-dimethoxyphenyl or 4-ethoxy-3-methoxyphenyl,
or an N-oxide thereof, a solvate and/or a (preferably pharmaceutically acceptable) salt thereof.

Preferred is also an embodiment of the invention that relates to a compound of the formula I, wherein
R¹ is phenyl that is unsubstituted or substituted by by one or more, especially one or two or further three, moieties selected from the group consisting of unsubstituted or substituted alkyl, such as C₁-C₇-alkyl that is unsubstituted or substituted by hydroxyl or cyano-C₁-C₇-alkyl, halo, hydroxyl, alkyloxy, especially C₁-C₇-alkoxy, especially methoxy, amino, mono- or disubstituted amino, preferably N-mono- or N,N-di-(C₁-C₇-alkyl and/or C₃-C₈-cyloalkyl)-amino, especially N-methylamino, C₁-C₇-alkanoylamino, C₁-C₇-alkoxycarbonyl-amino, phenyl- or naphthyl-C₁-C₇-alkoxycarbonyl-amino, carbamoyl, mono- or disubstituted carbamoyl, preferably N-mono- or N,N-di-(C₁-C₇-alkyl and/or C₃-C₈-cycloalkyl)-carbamoyl, heterocyclylcarbonyl (= heterocyclyl-C(=O)-) where heterocyclyl is bound via a ring nitrogen to the carbonyl, especially piperidihocarbonyl, morpholino-carbonyl, thiomorpholino-carbonyl or S-oxo- or S,S-dioxothiomorpholinocarbonyl, C₁-C₇-alkanesulfonyl, such as methanesulfonyl, sulfamoyl, N-mono- or N,N-disubstituted sulfamoyl, preferably N-mono- or N,N-di-(C₁-C₇-alkyl)-sulfamoyl, cyano, nitro, unsubstituted or substituted heterocyclyl bound via a ring carbon atom or preferably a ring nitrogen atom, especially 1,2,4-triazol-1-yl, carbamoyl-1,2,4-triazol-1-yl, such as 3-carbamoyl-1,2,4-triazol-1-yl, pyrazol-1-yl, 3-trifluoromethylpyrazol-1-yl, 3-(halophenyl)-pyrazol-1-yl, e.g. 3-(4-chlorophenyl)-pyrazol-1-yl, pyrrolidin-1-yl, 2-oxo-pyrrolidin-1-yl, piperidin-1-yl, 2-oxopiperidin-1-yl, morpholino, pyridine-(2-, 3- or 4-)yl, pyrimidin-(2-, 4- or 5-)yl, benzimidazol(especially -1-)yl, (e.g. 5-)C₁-C₇-alkoxy-substituted benzimidazol(especially -1-)yl, pyrrolo-pyrimidinyl, especially pyrrolo[2,3-d]pyrimidin-(e.g.1-)-yl, C₁-C₇-alkyl-substituted pyrrolo-pyrimidinyl, e.g. 2-C₁-C₇-alkyl-pyrrolo[2,3-d]pyrimidin-(e.g.1-)yl (meaning 2-C₁-C₇-alkyl-5,7-diazaindol-1-yl), and 1H,4H,5H-trihydropyrazolo[2,3-c]piperidin-1-yl (meaning 5-aza-3,4,5,6-tetrahydroindazol-1-yl) which is unsubstituted or substituted by 1 or 2 substituents independently selected from C₁-C₇-alkyl (e.g. methyl, especially in 5-position) and halo-C₁-C₇-alkyl (e.g. trifluoromethyl, especially in 3-position), and/or further from unsubstituted or substituted aryl, from unsubstituted or substituted cycloalkyl and from unsubstituted or substituted heterocyclyl, especially from phenyl that is unsubstituted or substituted by one or more, e.g. up to 2, moieties independently selected from the group consisting of halo, C₁-C₇-alkoxy and C₁-C₇-alkanesulfonyl, from tetrazol-5-yl, from indol-(e.g.5-)yl, from indazolyl, e.g. indazol-5-yl, from (e.g. 3-) C₁-C₇-alkyl-indazoyl-(e.g. 5-)yl and from pyrrolo-pyridinyl, e.g. pyrrolo[2,3-c]pyridine-1-yl (meaning 5-aza-indol-1-yl);
R² is phenyl that is substituted by 1 to 3, preferably 1 or 2 (especially in meta- and/or para-position) substituents selected from the group consisting of halo, especially fluoro, C₁-C₇-alkoxy (very preferred), especially methoxy, hydroxyl, C₁-C₇-alkoxy-C₁-C₇-alkoxy, especially 2-methoxyethoxy, 2-ethoxyethoxy, 2- or 3-methoxypropoxy, 2- or 3-ethoxypropoxy or 2- or 3-propoxypropoxy, C₁-C₇-alkoxy-C₁-C₇-alkoxy-C₁-C₇-alkoxy, such as 2-(2-methoxyethoxy or 2-ethoxyethoxy)-ethoxy, amino-C₁-C₇-alkoxy, N-mono- or N,N-di-(C₁-C₇-alkyl)-amino-C₁-C₇-alkoxy, e.g. 2-dimethyl- or 2-diethyl-amino-ethoxy or 2- or 3-dimethyl- or 2- or 3-diethyl-amino-propoxy, pyrrolidinyl-C₁-C₇-alkoxy, piperidinyl-C₁-C₇-alkoxy, e.g. piperidino-C₁-C₇-alkxoy, morpholinyl-C₁-C₇-alkoxy, e.g. morpholino-C₁-C₇-alkoxy, thiomorpholinyl-C₁-C₇-alkoxy, e.g. thiomorpholino-C₁-C₇-alkoxy, S-oxo-thiomorpholinyl-C₁-C₇-alkoxy, e.g. S-oxothiomorpholino-C₁-C₇-alkoxy, S,S-dioxothiomorpholinyl-C₁-C₇-alkoxy, e.g. S,S-dioxothiomorpholino-C₁-C₇-alkoxy, piperazinyl-C₁-C₇-alkoxy, e.g. piperazino-C₁-C₇-alkoxy, N'-C₁-C₇-alkyl-piperazino-C₁-C₇-alkoxy, C₃-C₈-cyloalkoxy, C₁-C₇-alkane-sulfonyl, e.g. methane- or ethanesulfonyl, and C₃-C₈-cyloalkyl-sulfonyl;
   preferably with the proviso that phenyl R² is substituted in meta position by C₁-C₇-alkoxy, especially methoxy, and in para-position by C₁-C₇-alkoxy, especially methoxy, hydroxyl, C₁-C₇-alkoxy-C₁-C₇-alkoxy, especially 2-methoxyethoxy, 2-ethoxyethoxy, 2- or 3-methoxypropoxy, 2- or 3-ethoxypropoxy or 2- or 3-propoxypropoxy, C₁-C₇-alkoxy-C₁-C₇-alkoxy-C₁-C₇-alkoxy, such as 2-(2-methoxyethoxy or 2-ethoxyethoxy)-ethoxy, amino-C₁-C₇-alkoxy, N-mono- or N,N-di-(C₁-C₇-alkyl)-amino-C₁-C₇-alkoxy, e.g. 2-dimethyl- or 2-diethyl-amino-ethoxy or 2- or 3-dimethyl- or 2- or 3-diethyl-amino-propoxy, pyrrolidinyl-C₁-C₇-alkoxy, piperidinyl-C₁-C₇-alkoxy, e.g. piperidino-C₁-C₇-alkxoy, morpholinyl-C₁-C₇-alkoxy, e.g. morpholino-C₁-C₇-alkoxy, thiomorpholinyl-C₁-C₇-alkoxy, e.g. thiomorpholino-C₁-C₇-alkoxy, S-oxo-thiomorpholinyl-C₁-C₇-alkoxy, e.g. S-oxothiomorpholino-C₁-C₇-alkoxy, S,S-dioxothiomorpholinyl-C₁-C₇-alkoxy, e.g. S,S-dioxothiomorpholino-C₁-C₇-alkoxy, piperazinyl-C₁-C₇-alkoxy, e.g. piperazino-C₁-C₇-alkoxy, N'-C₁-C₇-alkyl-piperazino-C₁-C₇-alkoxy, C₃-C₈-cyloalkoxy, C₁-C₇-alkanesulfonyl, e.g. methane- or ethanesulfonyl, or C₃-C₈-cyloalkyl-sulfonyl; where in one more preferred embodiment R² is 3,4-dimethoxyphenyl;
or an N-oxide thereof, a solvate and/or a (preferably pharmaceutically acceptable) salt thereof.

Preferred is also an embodiment of the invention that relates to a compound of the formula I, wherein
R¹ is phenyl that is unsubstituted or substituted (preferably in the 3- (meta) and/or 4- (para) position of the phenyl) by one or more, especially one or two, or further three, moieties selected from the group consisting of hydroxyl-C₁-C₇-alkyl, such as 3-hydroxypropyl, cyano-C₁-C₇-alkyl, such as cyanomethyl, halo, especially fluoro, chloro, bromo or iodo, C₁-C₇-alkoxy, especially methoxy, amino, N-mono- or N,N-di-(C₁-C₇-alkyl and/or C₃-C₈-cyloalkyl)-amino, especially N-methylamino, C₁-C₇-alkanoylamino, C₁-C₇-alkoxycarbonyl-amino, phenyl- or naphthyl-C₁-C₇-alkoxycarbonyl-amino, carbamoyl, N-mono- or N,N-di-(C₁-C₇-alkyl and/or C₃-C₈-cycloalkyl)-carbamoyl, piperidinocarbonyl, morpholino-carbonyl, thiomorpholino-carbonyl, S-oxo- or S,S-dioxothiomorpholinocarbonyl, C₁-C₇-alkanesulfonyl, such as methanesulfonyl, sulfamoyl, N-mono- or N,N-di-(C₁-C₇-alkyl)-sulfamoyl, cyano, nitro, 1,2,4-triazol-1-yl, carbamoyl-1,2,4-triazol-1-yl, such as 3-carbamoyl-1,2,4-triazol-1-yl, pyrazol-1-yl, 3-trifluoromethyl-pyrazol-1-yl, 3-(halophenyl)-pyrazol-1-yl, e.g. 3-(4-chlorophenyl)-pyrazol-1-yl, pyrrofidin-1-yl, 2-oxo-pyrrolidin-1-yl, piperidin-1-yl, 2-oxopiperidin-1-yl, morpholino, pyridine-(2-, 3- or 4-)yl, pyrimidin-(2-, 4- or 5-)yl, benzimidazol(especially -1-)yl, (e.g. 5-)C₁-C₇-alkoxy-substituted benzimidazol(especially -1-)yl, pyrrolo-pyrimidinyl, especially pyrrolo[2,3-d]pyrimidin-(e.g.1-)yl, C₁-C₇-alkyl-substituted pyrrolo-pyrimidinyl, e.g. 2-C₁-C₇-alkyl-pyrrolo[2,3-d]pyrimidin-(e.g.1-)yl (meaning 2-C₁-C₇-alkyl-5,7-diazaindol-1-yl), and *1H,4H,5H*-trihydropyrazolo[2,3-c]piperidin-1-yl (meaning 5-aza-*3,4,5,6*-tetrahydroindazol-1-yl) which is unsubstituted or substituted by 1 or 2 substituents independently selected from C₁-C₇-alkyl (e.g. methyl, especially in 5-position) and halo-C₁-C₇-alkyl (e.g. trifluoromethyl, especially in 3-position), or further from phenyl that is unsubstituted or substituted by one or more, e.g. up to 2, moieties independently selected from the group consisting of halo, C₁-C₇-alkoxy and C₁-C₇-alkanesulfonyl, from tetrazol-5-yl, from indol-(e.g.5-)yl, from indazolyl, from (e.g. 3-) C₁-C₇-alkyl-indazoyl-(e.g. 5-)yl and from pyrrolo-pyridinyl, e.g. pyrrolo[2,3-c]pyridine-1-yl (meaning 5-aza-indol-1-yl); and
R² is phenyl that is substituted by 1 to 3, preferably 1 or 2 (especially in meta- and/or para-position) substituents selected from the group consisting of halo, especially fluoro, C₁-C₇-alkoxy (very preferred), especially methoxy, hydroxyl, C₁-C₇-alkoxy-C₁-C₇-alkoxy, especially 2-methoxyethoxy, 2-ethoxyethoxy, 2- or 3-methoxypropoxy, 2- or 3-ethoxypropoxy or 2- or 3-propoxypropoxy, C₁-C₇-alkoxy-C₁C₇-alkoxy-C₁-C₇-alkoxy, such as 2-(2-methoxyethoxy or 2-ethoxyethoxy)-ethoxy, amino-C₁-C₇-alkoxy, N-mono- or N,N-di-(C₁-C₇-alkyl)-amino-C₁-C₇-alkoxy, e.g. 2-dimethyl- or 2-diethyl-amino-ethoxy or 2- or 3-dimethyl- or 2- or 3-diethyl-amino-propoxy, pyrrolidinyl-C₁-C₇-alkoxy, piperidinyl-C₁-C₇-alkoxy, e.g. piperidino-C₁-C₇-alkxoy, morphnlinyl-C₁-C₇-alkoxy, e.g. morpholino-C₁-C₇-alkoxy, thiomorpholinyl-C₁-C₇-alkoxy, e.g. thiomorpholino-C₁-C₇-alkoxy, S-oxo-thiomorpholinyl-C₁-C₇-alkoxy, e.g. S-oxothiomorpholino-C₁-C₇-alkoxy, S,S-dioxothiomorpholinyl-C₁-C₇-alkoxy, e.g. S,S-dioxothiomorpholino-C₁-C₇-alkoxy, piperazinyl-C₁-C₇-alkoxy, e.g. piperazino-C₁-C₇-alkoxy, N'-C₁-C₇-alkyl-piperazino-C₁-C₇-alkoxy, C₃-C₈-cyloalkoxy, C₁-C₇-alkane-sulfonyl, e.g. methane- or ethanesulfonyl, and C₃-C₈-cyloalkyl-suifonyl;
   preferably with the proviso that phenyl is substituted in meta position by C₁-C₇-alkoxy, especially methoxy, and in para-position by C₁-C₇-alkoxy, especially methoxy, hydroxyl, C₁-C₇-alkoxy-C₁-C₇-alkoxy, especially 2-methoxyethoxy, 2-ethoxyethoxy, 2- or 3-methoxypropoxy, 2- or 3-ethoxypropoxy or 2- or 3-propoxypropoxy, C₁-C₇-alkoxy-C₁-C₇-alkoxy-C₁-C₇-alkoxy, such as 2-(2-methoxyethoxy or 2-ethoxyethoxy)-ethoxy, amino-C₁-C₇-alkoxy, N-mono- or N,N-di-(C₁-C₇-alkyl)-amino-C₁-C₇-alkoxy, e.g. 2-dimethyl- or 2-diethyl-amino-ethoxy or 2- or 3-dimethyl- or 2- or 3-diethyl-amino-propoxy, pyrrolidinyl-C₁-C₇-alkoxy, piperidinyl-C₁-C₇-alkoxy, e.g. piperidino-C₁-C₇-alkxoy, morpholinyl-C₁-C₇-alkoxy, e.g. morpholino-C₁-C₇-alkoxy, thiomorpholinyl-C₁-C₇-alkoxy, e.g. thiomorpholino-C₁-C₇-alkoxy, S-oxo-thiomorpholinyl-C₁-C₇-alkoxy, e.g. S-oxothiomorpholino-C₁-C₇-alkoxy, S,S-dioxothiomorpholinyl-C₁-C₇-alkoxy, e.g. S,S-dioxothiomorpholino-C₁-C₇-alkoxy, piperazinyl-C₁-C₇-alkoxy, e.g. piperazino-C₁-C₇-alkoxy, N'-C₁C₇-alkyl-piperazino-C₁-C₇-alkoxy, C₃-C₈-cyloalkaxy, C₁-C₇-alkane-sulfonyl, e.g. methane- or ethanesulfonyl, or C₃-C₈-cyloalkyl-sulfonyl; where in one preferred embodiment R² is 3,4-d'imethoxyphenyl;
or an N-oxide thereof, a solvate and/or a (preferably pharmaceutically acceptable) salt thereof.

Preferred is also an embodiment of the invention that relates to a compound of the formula I, wherein
R¹ is phenyl, (especially 3- or 4-) halophenyl, (especially 3- or 4-) C₁-C₇-alkoxyphenyl, halo- and C₁-C₇-alkoxy-substituted phenyl, especially 3-halo-4-C₁-C₇-alkoxyphenyl or 4-halo-3-C₁-C₇-alkoxyphenyl, halo-, C₁-C₇-alkoxy and C₁-C₇-alkyl-substituted phenyl, such as 2-halo-4-C₁-C₇-alkoxy-5-C₁-C₇-alkyl-phenyl, (especially 3- or 4-) aminophenyl, (especially 3- or 4-) mono- or di-(C₁-C₇-alkyl)phenyl, (especially 3- or 4-) C₁-C₇-alkanesulfonyl-phenyl, (especially 3-or 4-) cyanophenyl, (especially 3- or 4-) nitrophenyl, (especially 4-) 1,2,4-triazol-1-yl-phenyl, (especially 4-) pyrazol-1-yl-phenyl, (especially 4-) (3-trifluoromethyl-pyrazal-1-yl)-phenyl, (especially 4-) 2-oxo-pyrrolidin-1-yl-phenyl, (especially 4-) 2-oxopiperidin-1-yl-phenyl, (especially 4-) morpholino-phenyl, (especially 3- or 4-)piperazin-1-yl-phenyl, (especially 3- or 4-) 4-(C₁-C₇-alkyl)-piperazin-1-yl-phenyl, (especially 4-) pyridine-(2-, 3- or 4-)yl-phenyl, (especially 4-) pyrimidin-(2-, 4- or 5-)yl-phenyl or (especially 3- or 4-)benzimidazol-1-yl-phenyl, and
R² is 3,4-di-C₁-C₇-alkoxy-phenyl, especially 3,4-dimethoxyphenyl,
or an N-oxide thereof, a solvate and/or a (preferably pharmaceutically acceptable) salt thereof.

Very preferred are also embodiments of the invention represented in the claims which are therefore incorporated by reference herein.

The invention relates especially to a compound of the formula I as mentioned below in the examples by their names, preferably the isomers shown as formulae, respectively, or a pharmaceutically acceptable salt thereof, or its USE according to the invention.

Quite unexpectedly, it has now been found that the compounds of formula I have advantageous pharmacological properties and inhibit the activity of the lipid kinases, such as the PI3-kinase and/or members of the PI3-kinase-related protein kinase family (also called PIKK and include DNA-PK, ATM, ATR, hSMG-1 and mTOR), such as the DNA protein-kinase, and may be used to treat disease or disorders which depend on the activity of said kinases.

The phosphatidylinosital'-3'-OH kinase (Pl3K) pathway is one of the central signaling pathways that exerts its effect on numerous cellular functions including cell cycle progression, proliferation, motility, metabolism and survival. An activation of receptor tyrosine kinases causes PI3K to phosphorylate phosphatidylinositol-(4,5)-diphosphate, resulting in membrane-bound phosphatidylinositol-(3,4,5)-triphosphate. The latter promotes the transfer of a variety of protein kinases from the cytoplasm to the plasma membrane by binding of phosphatidylinositol-(3,4,5)-triphosphate to the pleckstrin-homology (PH) domain of the kinase. Kinases that are key downstream targets of PI3K include phosphoinositide-dependent kinase 1 (PDK1) and AKT (also known as Protein Kinase B). Phosphorylation of such kinases then allows for the activation or deactivation of numerous other pathways, involving mediators such as GSK3, mTOR, PRAS40, FKHD, NF-κB, BAD, Caspase-9, and the like. An important negative feedback mechanism for the P13K pathway is PTEN, a phosphatase that catalyses the dephosphorylation of phosphatidylinositol-(3,4,5)-triphosphate to phosphorylate phosphatidylinositol-(4,5)-diphosphate. In more than 60 % of all solid tumors, PTEN is mutated into an inactive form, permitting a constitutive activation of the PI3K pathway. As most cancers are solid tumors, such an observation provides evidence that a targeting of PI3k itself or individual downstream kinases in the PI3K pathway provide a promising approach to mitigate or even abolish the dysregulation in many cancers and thus restore normal cell function and behaviour. This, however, does not exclude that other mechanisms may be responsible for the beneficial effects of PI3K activity modifying agents such as those in the present invention.

Having regard to their inhibitory effect on phosphatidylinositol 3-kinase enzymes, compounds of formula (I) in free or pharmaceutically acceptable salt form, are useful in the treatment of conditions which are mediated by the activation (including normal activity or especially overactivity) of one or more of the members of the P13 kinase family, especially P13 kinase enzyme, such as proliferative, inflammatory or allergic conditions, obstructive airways diseases and/or disorders commonly occurring in connection with transplantation.

"Treatment" in accordance with the invention may be therapeutic, e.g. symptomatic, and/or prophylactic. Preferred is the treatment of warm-blooded animals, especially humans.

Preferred is a compound of formula I for use or the use thereof in the treatment of a proliferative disease selected from a benign or malignant tumor, carcinoma of the brain, kidney, liver, adrenal gland, bladder, breast, stomach, gastric tumors, ovaries, colon, rectum, prostate, pancreas, lung, vagina or thyroid, sarcoma, glioblastomas, multiple myeloma or gastrointestinal cancer, especially colon carcinoma or colorectal adenoma or a tumor of the neck and head, an epidermal hyperproliferation, psoriasis, prostate hyperplasia, a neoplasia, a neoplasia of epithelial character, lymphomas, a mammary carcinoma or a leukemia. Other diseases include Cowden syndrome, Lhermitte-Dudos disease and Bannayan-Zonana syndrome, or diseases in which the PI3K/PKB pathway is aberrantly activated.

Compounds according to the invention are also of use in the treatment of inflammatory or obstructive airways (respiratory tract) diseases, resulting, for example, in reduction of tissue damage, airways inflammation, bronchial hyperreactivity, remodeling or disease progresssion. Inflammatory or obstructive airways diseases to which the present invention is applicable include asthma of whatever type or genesis including both intrinsic (non-allergic) asthma and extrinsic (allergic) asthma, e.g. mild asthma, moderate asthma, severe asthma, bronchitic asthma, exercise-induced asthma, occupational asthma and asthma induced following bacterial infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma can be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Compounds of the formula I can be of use for other inflammatory or obstructive airways diseases and conditions to which the present invention is applicable and include acute lung injury (ALI), adult/acute respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy.

The invention also to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumo-coniosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to their anti-inflammatory activity, in particular in relation to inhibition of eosinophil activation, compounds of the invention are also of use in the treatment of eosinophil related disorders, e.g. eosinophilia, in particular eosinophil related disorders of the airways (e.g. involving morbid eosinophilic infiltration of pulmonary tissues) including hypereosino-philia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

Compounds of the invention are also of use in the treatment of inflammatory or allergic conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphigus, epidermolysis bullosa acquisita, and other inflammatory or allergic conditions of the skin.

Compounds of the invention may also be used for the treatment of other diseases or conditions, such as diseases or conditions having an inflammatory component, for example, treatment of diseases and conditions of the eye such as conjunctivitis, keratoconjunctivitis sicca, and vernal conjunctivitis, diseases affecting the nose including allergic rhinitis, and inflammatory disease in which autoimmune reactions are implicated or having an autoimmune component or aetiology, including autoimmune haematological disorders (e.g. haemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, Steven-Johnson syndrome, idiopathic sprue, autoimmune inflammatory bowel disease (e.g. ulcerative colitis and Crohn's disease), endocrine opthalmopathy, Grave's disease, sarcoidosis, alveolitis, chronic hypersensitivity pneumonitis, multiple sclerosis, primary billiary cirrhosis, uveitis (anterior and posterior), keratoconjunctivitis sicca and vernal keratoconjunctivitis, interstitial lung fibrosis, psoriatic arthritis and glomerulonephritis (with and without nephrotic syndrome, e.g. including idiopathic nephrotic syndrome or minimal change nephropathy).

Furthermore, the invention provides the use of a compound according to the definitions herein, or a pharmaceutically acceptable salt, or a hydrate or solvate thereof for the preparation of a medicament for the treatment of a proliferative disease, an inflammatory disease, an obstructive respiratory disease, or a disorder commonly occurring in connection with transplantation.

The invention expecially relates to the use of a compound of the formula I (or a pharmaceutical formulation comprising a compound of the formula I) in the treatment of one or more of the diseases mentioned above and below where the disease(s) respond or responds (in a beneficial way, e.g. by partial or complete removal of one or more of its symptoms up to complete cure or remission) to an inhibition of one or more kinases of the PI3-kinase-related protein kinase family, most especially PI3 kinase (PI3K), especially where the kinase shows (in the context of other regulatory mechanisms) inadequately high or more preferably higher than normal (e.g. constitutive) activity.

Whereever the term "use" or "used" is mentioned, this is intended to include a compound of the formula I (also the one excluded from the compound per se protection above and in the claims) for use in the prophylactic and/or therapeutic treatment of a disease of a warm-blooded animal, especially a human, preferably of one or more diseases mentioned above or below, a method of use or a method of treatment comprising administering a compound of the formula I to a person in need of such treatment in an effective amount for the prophylactic and/or therapeutic treatment of a disease as mentioned above and below, the preparation or a method or preparation of a pharmaceutical formulation/preparation for use in the prophylactic and therapeutic treatment of a disease mentioned above and below, especially involving mixing a compound of the formula I (as therapeutically active ingredient) with at least one pharmaceutically acceptable carrier material, including making it ready for use in such treatment (e.g. adding an instruction insert (e.g. package leaflet or the like), formulation, appropriate preparation, adaptation for specific uses, customizing and the like), and the use of a compound of the formula I for such preparation, and/or all other prophylactic or therapeutic uses mentioned hereinbefore or below. All these aspects are embodiments of the present invention.

The efficacy of the compounds of formula I and salts thereof as PI3 kinase inhibitors can be demonstrated as follows:

The kinase reaction is performed in a final volume of 50 µL per well of a half area COSTAR, 96 well plate. The final concentrations of ATP and phosphatidyl inositol in the assay are 5 µM and 6 µg/mL respectively. The reaction is started by the addition of P13 kinase, e.g. PI3 kinase p110β.

The components of the assay are added per well as follows:
- 10 µL test compound in 5% DMSO per well in columns 2-1.
- Total activity is determined by addition 10 µL of 5% vol/vol DMSO in the first 4 wells of column 1 and the last 4 wells of column 12.
- The background is determined by addition of 10 µM control compound to the last 4 wells of column 1 and the first 4 wells of column 12.
- 2 mL 'Assay mix' are prepared per plate:
   1.912 mL of HEPES assay buffer
   8.33 µL of 3 mM stock of ATP giving a final concentration of 5 µM per well
   1 µL of [³³P]ATP on the activity date giving 0.05 µCi per well
   30 µL of 1 mg/mL PI stock giving a final concentration of 6 µg/mL per well
   5 µL of 1 M stock MgCl₂ giving a final concentration of 1 mM per well
- 20 µL of the assay mix are added per well.
- 2 mL 'Enzyme mix' are prepared per plate (x* µL P13 kinase p110β in 2 mL of kinase buffer). The 'Enzyme mix' is kept on ice during addition to the assay plates.
- 20 µl 'Enzyme mix' are added/well to start the reaction.
- The plate is then incubated at room temperature for 90 minutes.
- The reaction is terminated by the addition of 50 µL WGA-SPA bead (wheat germ agglutinin-coated Scintillation Proximity Assay beads) suspension per well.
- The assay plate is sealed using TopSeal-S)heat seal for polystyrene microplates, PerkinElmer LAS (Deutschland) GmbH, Rodgau, Germany) and incubated at room temperature for at least 60 minutes.
- The assay plate is then centrifuged at 1500 rpm for 2 minutes using the Jouan bench top centrifuge (Jouan Inc., Nantes, France).
- The assay plate is counted using a Packard TopCount, each well being counted for 20 seconds.
   * The volume of enzyme is dependent on the enzymatic activity of the batch in use.

Some of the compounds show a certain level of selectivity against the different paralogs PI3K alpha, beta, gamma and delta.

The range of activity in these assays is preferably between 150 nM and about 5 uM.

### Description of biochemical assay for DNA-PK:

The assay is conducted using the kit V7870 from Promega (SignaTECT® DNA-Dependent Protein Kinase Syste, comprises DNA-PK, biotinylated peptide substrate end further ingredients, Promega, Madison, Wisconsin, USA), that quantitates DNA-dependent protein kinase activity, both in purified enzyme preparations and in cell nuclear extracts. DNA-PK is a nuclear serine/threonine protein kinase that requires double-stranded DNA (dsDNA) for activity. The binding of dsDNA to the enzyme results in the formation of the active enzyme and also brings the substrate closer to the enzyme, allowing the phosphorylation reaction to proceed.

DNA-PK X5 reaction buffer (250 mM HEPES, 500 mM KCl, 50 mM MgCl₂, 1 mM EGTA, 0.5 mM EDTA, 5 mM DTT, pH to 7.5 with KOH) is diluted 1/5 in deionised water and BSA (stock = 10 mg/ml) is added to a final concentration of 0.1 mg/ml.

The activation buffer is made from 100 µg/ml of calf thymus DNA in control buffer (10 mM Tris-HCl (pH 7.4), 1 mM EDTA (pH 8.0)). Per tube, the reaction mix is composed of: 2.5 µl of activation or control buffers, 5 µl of X5 reaction buffer, 2.5 µl of p53-derived biotinylated peptide substrate (stock= 4mM), 0.2 µl of BSA (stock at 10 mg/ml) and 5 µl of [γ-³²P] ATP (5 µl of 0.5 mM cold ATP + 0.05 µl of Redivue [γ-³²P] ATP = Amersham AA0068-250 µCi, 3000Ci/mmol, 10 µCi/µl (now GE Gealthoare Biosciences AB, Uppsala, Sweden).

The DNA-PK enzyme (Promega V5811, concentration=100 U/µL) is diluted 1/10 in X1 reaction buffer and kept on ice until imminent use. 10.8 µl of the diluted enzyme is incubated with 1.2 µl of 100 µM compounds (diluted 1/100 in water from 10 mM stock in neat DMSO) for 10 minutes, at room temperature. During that time, 15.2 µl of the reaction mix is added to screw-capped tubes, behind Perspex glass. 9.8 µl of the enzyme is then transferred to the tubes containing the reaction mix and after 5 minutes incubation, at 30°C, the reaction is stopped by adding 12.5 µl of termination buffer (7.5 M guanidine hydrochloride).

After mixing well, a 10 µl aliquot of each tube is spotted onto a SAM2^{®} biotin capture membrane (Promega, Madison, Wisconsin, USA), which is left to dry for a few minutes. The membrane is then washed extensively to remove the excess free [γ-³²P] ATP and nonbiotinylated proteins: once for 30 seconds in 200 ml of 2M NaCl, 3 times for 2 minutes each in 200 ml of 2M NaCl, 4 times for 2 minutes each in 2M NaCl in 1 % H₃PO₄ and twice for 30 seconds each in 100 ml of deionised water. The membrane is subsequently left to air-dry at room temperature for 30-60 minutes.

Each membrane square is separated using forceps and scissors and placed into a scintillation vial, after which 8 ml of scintillation liquid (Flo-Scint 6013547 from Perkin-Elmer) is added. The amount of ³²P incorporated into the DNA-PK biotinylated peptide substrate is then determined by liquid scintillation counting. In this test system, compounds of the formula I can be shown to have IC₅₀ values in the range from 10 nM to 50 µM, e.g. from 10 nM to 10 µM.

The efficacy of the compounds of the invention in blocking the activation of the PI3K/PKB pathway can be demonstrated in cellular settings as follows:

### Protocol for the detection of phospho-PKB in U87MG cells by Elisa:

U87MG cells (human glioblastoma, ATCC No. HTB-14) are trypsinized, counted in a CASY cell counter (Schärffe systems, Göttingen, Germany), diluted in fresh complete DMEM high glucose medium to load ,per well ,150µL cell suspension containing 4x10⁴ cells, and test plates incubated for 18 hours. In parallel, 50 µL of coating antibody, at the desired concentration in PBS/O is loaded in each well of the ELISA plates, and plates are kept for 2 h at room temperature. This ELISA assays is performed in black flat-bottom 96-well plates (Microtest^{™}, Falcon Becton-Dickinson, Ref: 353941) sealed with Plate Sealers (Costar-Corning, Ref: 3095). Medium in plates is discarded and replaced by complete DMEM high glucose medium containing either 0.1 % DMSO or 0.1 % inhibitor at titers (7) between 10 mM and 0.156 mM in DMSO. After 30 minutes of contact, the medium is quickly removed by aspiration, plates are then placed on ice and immediately cells lyzed with 70 µL of Lysis buffer. In parallel, the 96 wells plates prepared with the coating antibody (1/250 diluted (in PBS/O) Anti-Akt1 C-20, goat, Santa-Cruz-1618, Santa Cruz Biotechnology, Inc., Santa Cruz, California, USA) are washed 3 times 1 min with PBS/O containing 0.05% Tween 20 and 0.1% Top-Block^{®} (derivative of gelatine that blocks unspecific binding sites on surfaces; Sigma-Aldrich, Fluka, Buchs, Switzerland, Ref.: 37766), and remaining protein binding sites blocked to prevent non-specific interactions with 200 µL of PBS containing 3% Top Block®, for 2 h at room temperature. Well content is replaced with 50 µL of samples from treated cells, and plates are incubated for 3 h at 4°C. The ELISA assays are always done in parallel with the following controls, in 6 replicates: U87MG (untreated control) or Lysis buffer alone (LB). After 3 x 15 minutes washes, all wells received 50 µL of the secondary antibody (1/250 diluted (in 3% top block) Anti-S473P-PKB, rabbit, Cell Signaling-9271, Cell Signaling Technologies, Inc., Danvers, Massachusetts, USA)), and are incubated for 16 h at 4°C. After three washes, plates are incubated with the third and conjugated antibody (1/1000 diluted (in 3% top block) anti rabbit (HRP) Jackson Immuno Research 111-035-144) for 2 hours at room temperature. Finally, the immune-complexes are washed 2 times 15 seconds with PBS/O/tween20 /top block 1 time with 200 µl of water and finally 200µl of water are left in each test well before a for 45 min incubation in darkness. The plates are then assayed with (SuperSignal^{®} ELISA pico Chemiluminescent substrate, Pierce, Ref: 27070, Pierce Biotechnology, Inc., Rockford, Illinois, USA). 100 µL of substrate are added, and plates shacked for 1 min. The luminescence is read immediately on a Top-Count NXT (Packard Bioscience) luminometer. Using this test system, IC₅₀ values in the range from 10 µM to 5 nM, more preferably from 5 µM to 10 nM can be found for compounds of the formula I as test compounds.

There are also experiments that can demonstrate the antitumor activity of compounds of the formula (I) *in vivo.*

For example, female Harlan (Indianapolis, Indiana, USA) athymic nu/nu mice with s.c. transplanted human glioblastoms U87MG tumors can be used to determine the anti-tumor activity of PI3 kinase inhibitors. On day 0, with the animals under peroral Forene® (1-chloro-2,2,2-trifluoroethyldifluormethylether, Abbot, Wiesbaden, Germany) narcosis, a tumor fragment of approximately 25 mg is placed under the skin on the animals' left flank and the small incised wound is closed by means of suture clips. When tumors reach a volume of 100 mm³, the mice are divided at random into groups of 6-8 animals and treatment commences. The treatment is carried out for a 2-3 weeks period with peroral, intravenous or intra-peritoneal administration once daily (or less frequently) of a compound of formula (I) in a suitable vehicle at defined doses. The tumors are measured twice a week with a slide gauge and the volume of the tumors is calculated.

As an alternative to cell line U87MG, other cell lines may also be used in the same manner, for example,
- the MDA-MB 468 breast adenocarcinoma cell line (ATCC No. HTB 132; see also In Vitro 14, 911-15 [1978]);
- the MDA-MB 231 breast carcinoma cell line (ATCC No. HTB-26; see also In Vitro 12, 331 [1976]);
- the MDA-MB 453 breast carcinoma cell line (ATCC No.HTB-131);
- the Colo 205 colon carcinoma cell line (ATCC No. CCL 222; see also Cancer Res. 38, 1345-55 [1978]);
- the DU145 prostate carcinoma cell line DU 145 (ATCC No. HTB 81; see also Cancer Res. 37, 4049-58 [1978]),
- the PC-3 prostate carcinoma cell line PC-3 (especially preferred; ATCC No. CRL 1435; see also Cancer Res. 40, 524-34 [1980]) and the PC-3M prostate carcinoma cell line;
- the A549 human lung adenocarcinoma (ATCC No. CCL 185; see also Int. J. Cancer 17, 62-70 [1976]),
- the NCI-H596 cell line (ATCC No. HTB 178; see also Science 246, 491-4 [1989]);
- the pancreatic cancer cell line SUIT-2 (see Tomioka et al., Cancer Res. 61, 7518-24 [2001]).

Compounds of the invention exhibit T cell inhibiting activity. More particular the compounds of the invention prevent T cell activation and/or proliferation in e.g. aqueous solution, e.g. as demonstrated in accordance with the following test method. The two-way MLR is performed according to standard procedures (J. Immunol. Methods, 1973, 2, 279 and Meo T. et al., Immunological Methods, New York, Academic Press, 1979, 227-39). Briefly, spleen cells from CBA and BALB/c mice (1.6 x 105 cells from each strain per well in flat bottom tissue culture microtiter plates, 3.2 x 105 in total) are incubated in RPMI medium containing 10% FCS, 100 U/ml penicillin, 100 µg/ml streptomycin (Gibco BRL, Basel, Switzerland), 50 µM 2-mercaptoethanol (Fluka, Buchs, Switzerland) and serially diluted compounds. Seven threefold dilution steps in duplicates per test compound are performed. After four days of incubation, 1 µCi 3H'-thymidine is added. Cells are harvested after an additional five-hour incubation period, and incorporated 3H-thymidine is determined according to standard procedures. Background values (low control) of the MLR are the proliferation of BALB/c cells alone. Low controls are subtracted from all values. High controls without any sample are taken as 100% proliferation. Percent inhibition by the samples is calculated, and the concentrations required for 50% inhibition (IC50 values) are determined. In this assay, the compounds of the invention preferably have IC50 values in the range of 10 nM to 5 µM, preferably from 10 nM to 500 nM.

A compound of the formula (I) may also be used to advantage in combination with other antiproliferative compounds. Such antiproliferative compounds include, but are not limited to aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibittors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (TEMODAL®); kinesin spindle protein inhibitors, such as SB715992 or SB743921 from GlaxoSmithKline, or pentamidine/chlorpromazine from CombinatoRx; MEK inhibitors such as ARRY142886 from Array PioPharma, AZD6244 from AstraZeneca, PD181461 from Pfizer, leucovorin, EDG binders, antileukemia compounds, ribonucleotide reductase inhibittors, S-adenosylmethionine decarboxylase inhibitors, antiproliferative antibodies or other chemotherapeutic compounds. Further, alternatively or in addition they may be used in combination with other tumor treatment approaches, including surgery, ionizing radiation, photodynamic therapy, implants, e.g. with corticosteroids, hormones, or they may be used as radiosensitizers. Also, in anti-inflammatory and/or antiproliferative treatment, combination with anti-inflammatory drugs is included. Combination is also possible with antihistamine drug substances, bronchodilatatory drugs, NSAID or antagonists of chemokine receptors.

The term "aromatase inhibitor" as used herein relates to a compound which inhibits the estrogen production, i.e. the conversion of the substrates androstenedione and testosterone to estrone and estradiol, respectively. The term includes, but is not limited to steroids, especially atamestane, exemestane and formestane and, in particular, non-steroids, especially aminogiutethimide, roglethimide, pyridoglutethimide, trilostane, testolactone, ketokonazole, vorozole, fadrozole, anastrozole and letrozole. Exemestane can be administered, e.g., in the form as it is marketed, e.g. under the trademark AROMASIN. Formestane can be administered, e.g., in the form as it is marketed, e.g. under the trademark LENTARON. Fadrozole can be administered, e.g., in the form as it is marketed, e.g. under the trademark AFEMA. Anastrozole can be administered, e.g., in the form as it is marketed, e.g. under the trademark ARIMIDEX. Letrozole can be administered, e.g., in the form as it is marketed, e.g. under the trademark FEMARA or FEMAR. Aminoglutethimide can be administered, e.g., in the form as it is marketed, e.g. under the trademark ORIMETEN. A combination of the invention comprising a chemotherapeutic agent which is an aromatase inhibitor is particularly useful for the treatment of hormone receptor positive tumors, e.g. breast tumors.
The term "antiestrogen" as used herein relates to a compound which antagonizes the effect of estrogens at the estrogen receptor level. The term includes, but is not limited to tamoxifen, fulvestrant, raloxifene and raloxifene hydrochloride. Tamoxifen can be administered, e.g., in the form as it is marketed, e.g. under the trademark NOLVADEX. Raloxifene hydrochloride can be administered, e.g., in the form as it is marketed, e.g. under the trademark EVISTA. Fulvestrant can be formulated as disclosed in US 4,659,516 or it can be administered, e.g., in the form as it is marketed, e.g. under the trademark FASLODEX. A combination of the invention comprising a chemotherapeutic agent which is an antiestrogen is particularly useful for the treatment of estrogen receptor positive tumors, e.g. breast tumors.
The term "anti-androgen" as used herein relates to any substance which is capable of inhibiting the biological effects of androgenic hormones and includes, but is not limited to, bicalutamide (CASODEX), which can be formulated, e.g. as disclosed in US 4,636,505.
The term "gonadorelin agonist" as used herein includes, but is not limited to abarelix, goserelin and goserelin acetate. Goserelin is disclosed in US 4,100,274 and can be administered, e.g., in the form as it is marketed, e.g. under the trademark ZOLADEX. Abarelix can be formulated, e.g. as disclosed in US 5,843,901.
The term "topoisomerase I inhibitor" as used herein includes, but is not limited to topotecan, gimatecan, irinotecan, camptothecian and its analogues, 9-nitrocamptothecin and the macromolecular camptothecin conjugate PNU-1 66148 (compound A1 in WO99/17804). Irinotecan can be administered, e.g. in the form as it is marketed, e.g. under the trademark CAMPTOSAR. Topotecan can be administered, e.g., in the form as it is marketed, e.g. under the trademark HYCAMTIN.
The term "topoisomerase II inhibitor" as used herein includes, but is not limited to the anthracyclines such as doxorubicin (including liposomal formulation, e.g. CAELYX), daunorubicin, epirubicin, idarubicin and nemorubicin, the anthraquinones mitoxantrone and Iosoxantrone, and the podophillotoxines etoposide and teniposide. Etoposide can be administered, e.g. in the form as it is marketed, e.g. under the trademark ETOPOPHOS. Teniposide can be administered, e.g. in the form as it is marketed, e.g. under the trademark VM 26-BRISTOL. Doxorubicin can be administered, e.g. in the form as it is marketed, e.g. under the trademark ADRIBLASTIN or ADRIAMYCIN. Epirubicin can be administered, e.g. in the form as it is marketed, e.g. under the trademark FARMORUBICIN. Idarubicin can be administered, e.g. in the form as it is marketed, e.g. under the trademark ZAVEDOS. Mitoxantrone can be administered, e.g. in the form as it is marketed, e.g. under the trademark NOVANTRON.

The term "microtubule active compound" relates to microtubule stabilizing, microtubule destabilizing compounds and microtublin polymerization inhibitors including, but not limited to taxanes, e.g. paclitaxel and docetaxel, vinca alkaloids, e.g., vinblastine, especially vinblastine sulfate, vincristine especially vincristine sulfate, and vinorelbine, discodermolides, cochicine and epothilones and derivatives thereof, e.g. epothilone B or D or derivatives thereof. Paclitaxel may be administered e.g. in the form as it is marketed, e.g. TAXOL. Docetaxel can be administered, e.g., in the form as it is marketed, e.g. under the trademark TAXOTERE. Vinblastine sulfate can be administered, e.g., in the form as it is marketed, e.g. under the trademark VINBLASTIN R.P.. Vincristine sulfate can be administered, e.g., in the form as it is marketed, e.g. under the trademark FARMISTIN. Discodermolide can be obtained, e.g., as disclosed in US 5,010,099. Also included are Epothilone derivatives which are disclosed in WO 98/10121, US 6,194,181, WO 98/25929, WO 98/08849, WO 99/43653, WO 98/22461 and WO 00/31247. Especially preferred are Epothilone A and/or B.
The term "alkylating compound" as used herein includes, but is not limited to, cyclophosphamide, ifosfamide, melphalan or nitrosourea (BCNU or Gliadel). Cyclophosphamide can be administered, e.g., in the form as it is marketed, e.g. under the trademark CYCLOSTIN. Ifosfamide can be administered, e.g., in the form as it is marketed, e.g. under the trademark HOLOXAN.
The term "histone deacetylase inhibitors" or "HDAC inhibitors" relates to compounds which inhibit the histone deacetylase and which possess antiproliferative activity. This includes compounds disclosed in WO 02/22577, especially N-hydroxy-3-[4-[[(2-hydroxyethyl)[2-(1H-indol-3-yl)ethyl]-amino]methyl]phenyl]-2E-2-propenamide, N-hydroxy-3-[4-[[[2-(2-methyl-1*H*-indol-3-yl)-ethyl]-amino]methyl]phenyl]-2*E*-2-propenamide and pharmaceutically acceptable salts thereof. It further especially includes Suberoylanilide hydroxamic acid (SAHA).
The term "antineoplastic antimetabolite" includes, but is not limited to, 5-Fluorouracil or 5-FU, capecitabine, gemcitabine, DNA demethylating compounds, such as 5-azacytidine and decitabine, methotrexate and edatrexate, and folic acid antagonists such as pemetrexed. Capecitabine can be administered, e.g., in the form as it is marketed, e.g. under the trademark XELODA. Gemcitabine can be administered, e.g., in the form as it is marketed, e.g. under the trademark GEMZAR..
The term "platin compound" as used herein includes, but is not limited to, carboplatin, cis-platin, cisplatinum and oxaliplatin. Carboplatin can be administered, e.g., in the form as it is marketed, e.g. under the trademark CARBOPLAT. Oxaliplatin can be administered, e.g., in the form as it is marketed, e.g. under the trademark ELOXATIN.

The term "compounds targeting/decreasing a protein or lipid kinase activity"; or a "protein or lipid phosphatase activity"; or "further anti-angiogenic compounds" as used herein includes, but is not limited to, protein tyrosine kinase and/or serine and/or threonine kinase inhibitors or lipid kinase inhibitors, e.g.,
a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g. a N-phenyl-2-pyrimidine-amine derivative, e.g. imatinib, SU101, SU6668 and GFB-111;
b) compounds targeting, decreasing or inhibiting the activity of the fibroblast growth factor-receptors (FGFR);
c) compounds targeting, decreasing or inhibiting the activity of the insulin-like growth factor receptor I (IGF-IR), such as compounds which target, decrease or inhibit the activity of IGF-IR, especially compounds which inhibit the kinase activity of IGF-I receptor, such as those compounds disclosed in WO 02/092599, or antibodies that target the extracellular domain of IG'F-I receptor or its growth factors;
d) compounds targeting, decreasing or inhibiting the activity of the Trk receptor tyrosine kinase family, or ephrin B4 inhibitors;
e) compounds targeting, decreasing or inhibiting the activity of the Axl receptor tyrosine kinase family;
f) compounds targeting, decreasing or inhibiting the activity of the Ret receptor tyrosine kinase;
g) compounds targeting, decreasing or inhibiting the activity of the Kit/SCFR receptor tyrosine kinase, e.g. imatinib;
h) compounds targeting, decreasing or inhibiting the activity of the C-kit receptor tyrosine kinases - (part of the PDGFR family), such as compounds which target, decrease or inhibit the activity of the c-Kit receptor tyrosine kinase family, especially compounds which inhibit the c-Kit receptor, e.g. imatinib;
i) compounds targeting, decreasing or inhibiting the activity of members of the c-Abl family, their gene-fusion products (e.g. BCR-Abl kinase) and mutants, such as compounds which target decrease or inhibit the activity of c-Abl family members and their gene fusion products, e.g. a N-phenyl-2-pyrimidine-amine derivative, e.g. imatinib or nilotinib (AMN107); PD180970; AG957; NSC 680410; PD173955 from ParkeDavis; or dasatinib (BMS-354825)
j) compounds targeting, decreasing or inhibiting the activity of members of the protein kinase C (PKC) and Raf family of serine/threonine kinases, members of the MEK, SRC, JAK, FAK, PDK1, PKB/Akt, and Ras/MAPK family members, and/or members of the cyclin-dependent kinase family (CDK) and are especially those staurosporine derivatives disclosed in US 5,093,330, e.g. midostaurin; examples of further compounds include e.g. UCN-01, safingol, BAY 43-9006, Bryostatin 1, Perifosine; Ilmofosine; RO 318220 and RO 320432; GO 6976; Isis 3521; LY333531/LY379196; isochinoline compounds such as those disclosed in WO 00/09495; FTIs; PD184352 or QAN697 (a P13K inhibitor) or AT7519 (CDK inhibitor);
k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810; AG 99; Tyrphostin AG 213; Tyrphostin AG 1748; Tyrphostin AG 490; Tyrphostin B44; Tyrphostin B44 (+) enantiomer; Tyrphostin AG 555; AG 494; Tyrphostin AG 556, AG957 and adaphostin (4-{[(2,5-dihydroxyphenyl)methyl]amino}-benzoic acid adamantyl ester; NSC 680410, adaphostin);
l) compounds targeting, decreasing or inhibiting the activity of the epidermal growth factor family of receptor tyrosine kinases (EGFR, ErbB2, ErbB3, ErbB4 as homo- or heterodimers) and their mutants, such as compounds which target, decrease or inhibit the activity of the epidermal growth factor receptor family are especially compounds, proteins or antibodies which inhibit members of the EGF receptor tyrosine kinase family, e.g. EGF receptor, ErbB2, ErbB3 and ErbB4 or bind to EGF or EGF related ligands, and are in particular those compounds, proteins or monoclonal antibodies generically and specifically disclosed in WO 97/02266, e.g. the compound of ex. 39, or in EP 0 564 409, WO 99/03854, EP 0520722, EP 0 566 225, EP 0 787 722, EP 0 837 063, US 5,747,498, WO 98/10767, WO 97/30034, WO 97/49688, WO 97/38983 and, especially, WO 96/30347 (e.g. compound known as CP 358774), WO 96/33980 (e.g. compound ZD 1839) and WO 95/03283 (e.g. compound ZM105180); e.g. trastuzumab (Herceptin^{™}), cetuximab (Erbitux^{™}), Iressa, Tarceva, OSI-774, Cl-1033, EKB-569, GW-2016, E1.1, E2.4, E2.5, E6.2, E6.4, E2.11, E6.3 or E7.6.3, and 7H-pyrrolo-[2,3-d]pyrimidine derivatives which are disclosed in WO 03/013541; and
m) compounds targeting, decreasing or inhibiting the activity of the c-Met receptor, such as compounds which target, decrease or inhibit the activity of c-Met, especially compounds which inhibit the kinase activity of c-Met receptor, or antibodies that target the extracellular domain of c-Met or bind to HGF.

Further anti-angiogenic compounds include compounds having another mechanism for their activity, e.g. unrelated to protein or lipid kinase inhibition e.g. thalidomide (THALOMID) and TNP-470.
Compounds which target, decrease or inhibit the activity of a protein or lipid phosphatase are e.g. inhibitors of phosphatase 1, phosphatase 2A, or CDC25, e.g. okadaic acid or a derivative thereof.
Compounds which induce cell differentiation processes are e.g. retinoic acid, α- γ- or δ-tocopherol or α- γ- or δ-tocotrienol.
The term cyclooxygenase inhibitor as used herein includes, but is not limited to, e.g. Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g. 5-methyl-2-(2'-chloro-6'-filuoroanilino)phenyl acetic acid, lumiracoxib.
The term "bisphosphonates" as used herein includes, but is not limited to, etridonic, clodronic, tiludronic, pamidronic, alendronic, ibandronic, risedronic and zoledronic acid. "Etridonic acid" can be administered, e.g., in the form as it is marketed, e.g. under the trademark DIDRONEL. "Clodronic acid" can be administered, e.g., in the form as it is marketed, e.g. under the trademark BONEFOS. "Tiludronic acid" can be administered, e.g., in the form as it is marketed, e.g. under the trademark SKELID. "Pamidronic acid" can be administered, e.g. in the form as it is marketed, e.g. under the trademark AREDIA^{™}. "Alendronic acid" can be administered, e.g., in the form as it is marketed, e.g. under the trademark FOSAMAX. "Ibandronic acid" can be administered, e.g., in the form as it is marketed, e.g. under the trademark BONDRANAT. "Risedronic acid" can be administered, e.g., in the form as it is marketed, e.g. under the trademark ACTONEL. "Zoledronic acid" can be administered, e.g. in the form as it is marketed, e.g. under the trademark ZOMETA. The term "mTOR inhibitors" relates to compounds which inhibit the mammalian target of rapamycin (mTOR) and which possess antiproliferative activity such as sirolimus (Rapamune®), everolimus (Certican^{™}), CCI-779 and ABT578.

The term "heparanase inhibitor" as used herein refers to compounds which target, decrease or inhibit heparin sulfate degradation. The term includes, but is not limited to, PI-88.
The term "biological response modifier" as used herein refers to a lymphokine or interferons, e.g. interferon γ.
The term "inhibitor of Ras oncogenic isoforms", e.g. H-Ras, K-Ras, or N-Ras, as used herein refers to compounds which target, decrease or inhibit the oncogenic activity of Ras e.g. a "farnesyl transferase inhibitor" e.g. L-744832, DK8G557 or R115777 (Zamestra).
The term "telomerase inhibitor" as used herein refers to compounds which target, decrease or inhibit the activity of telomerase. Compounds which target, decrease or inhibit the activity of telomerase are especially compounds which inhibit the telomerase receptor, e.g. telomestatin.
The term "methionine aminopeptidase inhibitor" as used herein refers to compounds which target, decrease or inhibit the activity of methionine aminopeptidase. Compounds which target, decrease or inhibit the activity of methionine aminopeptidase are e.g. bengamide or a derivative thereof.
The term "proteasome inhibitor" as used herein refers to compounds which target, decrease or inhibit the activity of the proteasome. Compounds which target, decrease or inhibit the activity of the proteasome include e.g. Bortezomid (Velcade^{™})and MLN 341.
The term "matrix metalloproteinase inhibitor" or ("MMP" inhibitor) as used herein includes, but is not limited to, collagen peptidomimetic and nonpeptidomimetic inhibitors, tetracycline derivatives, e.g. hydroxamate peptidomimetic inhibitor batimastat and its orally bioavailable analogue marimastat (BB-2516), prinomastat (AG3340), metastat (NSC 683551) BMS-279251, BAY 12-9566, TAA211, MMI270B or AAJ996.
The term "compounds used in the treatment of hematologic malignancies" as used herein includes, but is not limited to, FMS-like tyrosine kinase inhibitors e.g. compounds targeting, decreasing or inhibiting the activity of FMS-like tyrosine kinase receptors (Flt-3R); interferon, 1-b-D-arabinofuransylcytosine (ara-c) and bisulfan; and ALK inhibitors e.g. compounds which target, decrease or inhibit anaplastic lymphoma kinase.
Compounds which target, decrease or inhibit the activity of FMS-like tyrosine kinase receptors (Flt-3R) are especially compounds, proteins or antibodies which inhibit members of the Flt-3R receptor kinase family, e.g. PKC412, midostaurin, a staurosporine derivative, SU11248 and MLN518.
The term "HSP90 inhibitors" as used herein includes, but is not limited to, compounds targeting, decreasing or inhibiting the intrinsic ATPase activity of HSP90; degrading, targeting, decreasing or inhibiting the HSP90 client proteins via the ubiquitin proteosome pathway. Compounds targeting, decreasing or inhibiting the intrinsic ATPase activity of HSP90 are especially compounds, proteins or antibodies which inhibit the ATPase activity of HSP90 e.g., 17-allylamino, 17-clemethoxygeldanamycin (17AAG), a geldanamycin derivative; other geldanamycin related compounds; radicicol and HDAC inhibitors.
The term "antiproliferative antibodies" as used herein includes, but is not limited to, trastuzumab (Herceptin^{™}), Trastuzumab-DM1,erbitux, bevacizumab (Avastin^{™}), rituximab (Rituxan^{®}), PRO64553 (anti-CD40) and 2C4 Antibody. By antibodies is meant e.g. intact monoclonal antibodies, polyclonal antibodies, multispecific antibodies formed from at least 2 intact antibodies, and antibodies fragments so long as they exhibit the desired biological activity.
For the treatment of acute myeloid leukemia (AML), compounds of formula (I) can be used in combination with standard leukemia therapies, especially in combination with therapies used for the treatment of AML. In particular, compounds of formula (I) can be administered in combination with, e.g., farnesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.
The term "antileukemic compounds" includes, for example, Ara-C, a pyrimidine analog, which is the 2'-alpha-hydroxy ribose (arabinoside) derivative of deoxycytidine. Also included is the purine analog of hypoxanthine, 6-mercaptopurine (6-MP) and fludarabine phosphate. Compounds which target, decrease or inhibit activity of histone deacetylase (HDAC) inhibitors such as sodium butyrate and suberoylanilide hydroxamic acid (SAHA) inhibit the activity of the enzymes known as histone deacetylases. Specific HDAC inhibitors include MS275, SAHA, FK228 (formerly FR901228), Trichostatin A and compounds disclosed in US 6,552,065, in particular, *N*-hydroxy-3-[4-[[[2-(2-methyl-1*H*-indol-3-yl)-ethyl]-amino]methyl]phenyl]-2*E*-2-propenamide, or a pharmaceutically acceptable salt thereof and *N*-hydroxy-3-[4-[(2-hydroxyethyl){2-(1*H*-indol-3-yl)ethyl]-amino]methyl]phenyl]-2*E*-2-propenamide, or a pharmaceutically acceptable salt thereof, especially the lactate salt. Somatostatin receptor antagonists as used herein refers to compounds which target, treat or inhibit the somatostatin receptor such as octreotide, and SOM230 (pasireotide). Tumor cell damaging approaches refer to approaches such as ionizing radiation. The term "ionizing radiation" referred to above and hereinafter means ionizing radiation that occurs as either electromagnetic rays (such as X-rays and gamma rays) or particles (such as alpha and beta particles). Ionizing radiation is provided in, but not limited to, radiation therapy and is known in the art. See Hellman, Principles of Radiation Therapy, Cancer, in Principles and Practice of Oncology, Devita et al., Eds., 4th Edition, Vol. 1, pp. 248-275 (1993). The term "EDG binders" as used herein refers a class of immunosuppressants that modulates lymphocyte recirculation, such as FTY720.
The term "ribonucleotide reductase inhibitors" refers to pyrimidine or purine nucleoside analogs including, but not limited to, fludarabine and/or cytosine arabinoside (ara-C), 6-thioguanine, 5-fluorouracil, cladribine, 6-mercaptopurine (especially in combination with ara-C against ALL) and/or pentastatin. Ribonucleotide reductase inhibitors are especially hydroxyurea or 2-hydroxy-1*H*-isoindole-1,3-dione derivatives, such as PL-1, PL-2, PL-3, PL-4, PL-5, PL-6, PL-7 or PL-8 mentioned in Nandy et al., Acta Oncologica, Vol. 33, Na. 8, pp. 953-961 (1994).
The term "S-adenosylmethionine decarboxylase inhibitors" as used herein includes, but is not limited to the compounds disclosed in US 5,461,076.
Also included are in particular those compounds, proteins or monoclonal antibodies of VEGF disclosed in WO 98/35958, e.g. 1-(4-chloroanilino)-4-(4-pyridyimethyl)phthalazine or a pharmaceutically acceptable salt thereof, e.g. the succinate, or in WO 00/09495,
WO 00/27820, WO 00/59509, WO 98111223, WO 00127819 and EP 0 769 947; those as described by Prewett et al, Cancer Res, Vol. 59, pp. 5209-5218 (1999); Yuan et al., Proc Natl Acad Sci U S A, Vol. 93, pp. 14765-14770 (1996); Zhu et al., Cancer Res, Vol. 58, pp. 3209-3214 (1998); and Mordenti et al., Toxicol Pathol, Vol. 27, No. 1, pp. 14-21 (1999); in WO 00/37502 and WO 94/10202; ANGIOSTATIN, described by O'Reilly et al., Cell, Vol. 79, pp. 315-328 (1994); ENDOSTATIN, described by O'Reilly et al., Cell, Vol. 88, pp. 277-285 (1997); anthranilic acid amides; ZD4190; ZD6474; SU5416; SU6668; bevacizumab; or anti-VEGF antibodies or anti-VEGF receptor antibodies, e.g. rhuMAb and RHUFab, VEGF aptamer e.g. Macugon; FLT-4 inhibitors, FLT-3 inhibitors, VEGFR-2 IgG1 antibody, Angiozyme (RPI 4610) and Bevacizumab (Avastin^{™}).

Photodynamic therapy as used herein refers to therapy which uses certain chemicals known as photosensitizing compounds to treat or prevent cancers. Examples of photodynamic therapy includes treatment with compounds, such as e.g. VISUDYNE and porfimer sodium. Angiostatic steroids as used herein refers to compounds which block or inhibit angiogenesis, such as, e.g., anecortave, triamcinolone. hydrocortisone, 11-α-epihydrocotisol, cortexolone, 17α-hydroxyprogesterone, corticosterone, desoxycorticosterone, testosterone, estrone and dexamethasone.

Implants containing corticosteroids refers to compounds, such as e.g. fluocinolone, dexamethasone.

"Other chemotherapeutic compounds" include, but are not limited to, plant alkaloids, hormonal compounds and antagonists; biological response modifiers, preferably lymphokines or interferons; antisense oligonucleotides or oligonucleotide derivatives; shRNA or siRNA; or miscellaneous compounds or compounds with other or unknown mechanism of action.

The compounds of the invention are also useful as co-therapeutic compounds for use in combination with other drug substances such as anti-inflammatory, bronchodilatory or antihistamine drug substances, particularly in the treatment of obstructive or inflammatory airways diseases such as those mentioned hereinbefore, for example as potentiators of therapeutic activity of such drugs or as a means of reducing required dosaging or potential side effects of such drugs. A compound of the invention may be mixed with the other drug substance in a fixed pharmaceutical composition or it may be administered separately, before, simultaneously with or after the other drug substance. Accordingly the invention includes a combination of a compound of the invention as hereinbefore described with an anti-inflammatory, bronchodilatory, antihistamine or anti-tussive drug substance, said compound of the invention and said drug substance being in the same or different pharmaceutical composition.
Suitable anti-inflammatory drugs include steroids, in particular glucocorticosteroids such as budesonide, beclamethasone dipropionate, fluticasone propionate, ciclesonide or mometasone furoate, or steroids described in WO 02/88167. WO 02/12266, WO 02/100879, WO 02/00679 (especially those of Examples 3, 11, 14, 17, 19, 26, 34, 37, 39, 51, 60, 67, 72, 73, 90, 99 and 101), WO 03/035668, WO 03/048181, WO 03/062259, WO 03/064445, WO 03/072592, non-steroidal glucocorticoid receptor agonists such as those described in WO 00/00531, WO 02/10143, WO 03/082280, WO 03/082787, WO 03/104195, WO 04/005229; LTB4 antagonists such LY293111, CGS025019C, CP-195543, SC-53228, BilL 284, ONO 4057, SB 209247 and those described in US 5451700; LTD4 antagonists such as montelukast and zafirlukast; PDE4 inhibitors such cilomilast (Ariflo® GlaxoSmithKline), Roflumilast (Byk Gulden),V-11294A (Napp), BAY19-8004 (Bayer), SCH-351591 (Schering-Plough), Arofylline (Almirall Prodesfarma), PD189659/ PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), and those disclosed in WO 92/19594, WO 93/19749, WO 93/19750, WO 93/19751, WO 98/18796, WO 99/16766, WO 01/13953, WO 03/104204, WO 03/104205. WO 03/39544, WO 04/000814, WO 04/000839, WO 04/005258, WO 04/018450, WO 04/018451, WO 04/018457, WO 04/018465, WO 04/ 018431, WO 04/018449, WO 04/018450, WO 04/018451, WO 04/018457, WO 04/018465, WO 04/019944, WO 04/019945, WO 04/045607 and WO 04/037805; A2a agonists such as those disclosed in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/ 039762, WO 04/039766, WO 04/045618 and WO 04/046083; A2b antagonists such as those described in WO 02/42298; and beta-2 adrenoceptor agonists such as albuterol (salbutamol), metaproterenol, terbutaline, salmeterol fenoterol, procaterol, and especially, formoterol and pharmaceutically acceptable salts thereof, and compounds (in free or salt or solvate form) of formula I of WO 0075114, which document is incorporated herein by reference, preferably compounds of the Examples thereof, especially a compound of formula 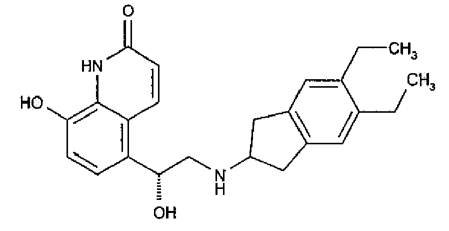 and pharmaceutically acceptable salts thereof, as well as compounds (in free or salt or solvate form) of formula I of WO 04/16601, and also compounds of WO 04/033412. Suitable bronchodilatory drugs include anticholinergic or antimuscarinic compounds, in particular ipratropium bromide, oxitropium bromide, tiotropium salts and CHF 4226 (Chiesi), and glycopyrrolate, but also those described in WO 01/04118, WO 02/51841, WO 02/53564, WO 03/00840, WO 03/87094, WO 04/05285, WO 02/00652, WO 03/53966, EP 424021, US 5171744, US 3714357, WO 03/33495 and WO 04/018422.
Suitable antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in WO 03/099807, WO 04/026841 and JP 2004107299.
Other useful combinations of compounds of the invention with anti-inflammatory drugs are those with antagonists of chemokine receptors, e.g. CCR-1, CCR-2, CCR-3, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzo-cyclohepten-8-yl]carbonyl]amino]phenyl]-methyl]tetrahydro-N,N-dimethyl-2H-pyran-4-amin-ium chloride (TAK-770), and CCR-5 antagonists described in US 6166037 (particularly claims 18 and 19), WO 00/66558 (particularly claim 8), WO 00/66559 (particularly claim 9), WO 04/018425 and WO 04/026873.
The structure of the active compounds identified by code nos., generic or trade names may be taken from the actual edition of the standard compendium "The Merck Index" or from databases, e.g. Patents International (e.g. IMS World Publications).

The above-mentioned compounds, which can be used in combination with a compound of the formula (I), can be prepared and administered as described in the art, such as in the documents cited above.

By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative, e.g. synergistic effect.

The invention also provides a pharmaceutical preparation, comprising a compound of formula I as defined herein, or an N-oxide or a tautomer thereof, or a pharmaceutically acceptable salt of such a compound, or a hydrate or solvate thereof, and at least one pharmaceutically acceptable carrier.

A compound of formula I can be administered alone or in combination with one or more other therapeutic compounds, possible combination therapy taking the form of fixed combinations or the administration of a compound of the invention and one or more other therapeutic (including prophylactic) compounds being staggered or given independently of one another, or the combined administration of fixed combinations and one or more other therapeutic compounds. A compound of formula I can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

The dosage of the active ingredient depends upon a variety of factors including type, species, age, weight, sex and medical condition of the patient; the severity of the condition to be treated; the route of administration; the renal and hepatic function of the patient; and the particular compound employed. A physician, clinician or veterinarian of ordinary skill can readily determine and prescribe the effective amount of the drug required to prevent, counter or arrest the progress of the condition. Optimal precision in achieving concentration of drug within the range that yields efficacy requires a regimen based on the kinetics of the drug's availability to target sites. This involves a consideration of the distribution, equilibrium, and elimination of a drug.

The dose of a compound of the formula I or a pharmaceutically acceptable salt thereof to be administered to warm-blooded animals, for example humans of approximately 70 kg body weight, is preferably from approximately 3 mg to approximately 5 g, more preferably from approximately 10 mg to approximately 1.5 g per person per day, divided preferably into 1 to 3 single doses which may, for example, be of the same size. Usually, children receive half of the adult dose.

The compounds of the invention may be administered by any conventional route, in particular parenterally, for example in the form of injectable solutions or suspensions, enterally, e.g. orally, for example in the form of tablets or capsules, topically, e.g. in the form of lotions, gels, ointments or creams, or in a nasal or a suppository form. Topical administration is e.g. to the skin. A further form of topical administration is to the eye. Pharmaceutical compositions comprising a compound of the invention in association with at least one pharmaceutical acceptable carrier or diluent may be manufactured in conventional manner by mixing with a pharmaceutically acceptable carrier or diluent.

The invention relates also to pharmaceutical compositions comprising an effective amount, especially an amount effective in the treatment of one of the above-mentioned disorders, of a compound of formula I or an N-oxide or a tautomer thereof together with one or more pharmaceutically acceptable carriers that are suitable for topical, enteral, for example oral or rectal, or parenteral administration and that may be inorganic or organic, solid or liquid. There can be used for oral administration especially tablets or gelatin capsules that comprise the active ingredient together with diluents, for example lactose, dextrose, mannitol, and/or glycerol, and/or lubricants and/or polyethylene glycol. Tablets may also comprise binders, for example magnesium aluminum silicate, starches, such as corn, wheat or rice starch, gelatin, methylcellulose, sodium carboxymethylcellulose and/or polyvinylpyrrolidone, and, if desired, disintegrators, for example starches, agar, alginic acid or a salt thereof, such as sodium alginate, and/or effervescent mixtures, or adsorbents, dyes, flavorings and sweeteners. It is also possible to use the pharmacologically active compounds of the present invention in the form of parenterally administrable compositions or in the form of infusion solutions. The pharmaceutical compositions may be sterilized and/or may comprise excipients, for example preservatives, stabilisers, wetting compounds and/or emulsifiers, solubilisers, salts for regulating the osmotic pressure and/or buffers. The present pharmaceutical compositions, which may, if desired, comprise other pharmacologically active substances are prepared in a manner known per se, for example by means of conventional mixing, granulating, confectionning, dissolving or lyophilising processes, and comprise approximately from 1% to 99% by weight, especially from approximately 1% to approximately 60%, active ingredient(s).

Additionally, the present invention provides a compound of formula I or an N-oxide or a tautomer thereof, or a pharmaceutically acceptable salt of such a compound, for use in a method for the treatment of the human or animal body, especially for the treatment of a disease mentioned herein, most especially in a patient requiring such treatment..

The present invention also relates to the use of a compound of formula I or a tautomer thereof, or a pharmaceutically acceptable salt of such a compound, for the preparation of a medicament for the treatment of a proliferative disease, an inflammatory disease, or an obstructtive airway disease, or disorders commonly occurring in connection with transplantation.

Furthermore, the invention relates to a method for the treatment of a proliferative disease which responds to an inhibition of lipid kinases and/or PI3-kinase-related protein kinases, in particular the PI3 kinase, and/or mTOR, and/or DNA protein kinase activity, which comprises administering a compound of formula I or a pharmaceutically acceptable salt thereof, wherein the radicals and symbols have the meanings as defined above, especially in a quantity effective against said disease, to a warm-blooded animal requiring such treatment.

Furthermore, the invention relates to a pharmaceutical composition for treatment of solid or liquid tumours in warm-blooded animals, including humans, comprising an antitumor effective dose of a compound of the formula I as described above or a pharmaceutically acceptable salt of such a compound together with a pharmaceutical carrier.

### Manufacturing Process:

The invention relates also to a process for the manufacture of a compound of the formula I, an N-oxide thereof, a solvate thereof and/or a salt thereof.

Compounds of the formula I can be prepared according to or in analogy to methods that, in principle but with other educts, intermediates and/or final products, are known in the art, especially and according to the invention by a novel process comprising
a) reacting a compound of the formula II, 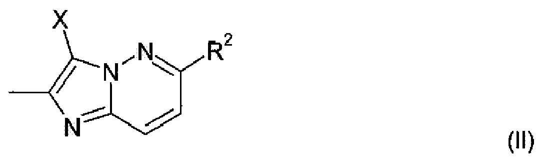 wherein R² is as defined for a compound of the formula I and X is halo, preferably chloro, bromo or iodo, or is trifluoromethansulfonyloxy, under cross-coupling conditions with a boronic acid or boronic acid ester of the formula III,

   R¹-D (III)

   wherein R¹ is as defined for a compound of the formula I and is bound via a carbon atom to D and D is -B(OH₂) in free form or in esterified form, e.g. as dialkoxy ester or as a group of the formula A, 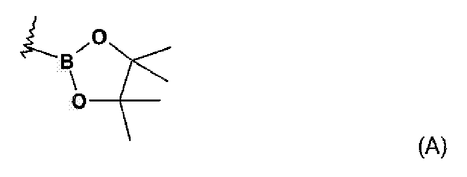 or
b) reacting a boronic acid or boronic acid ester compound of the formula IV, 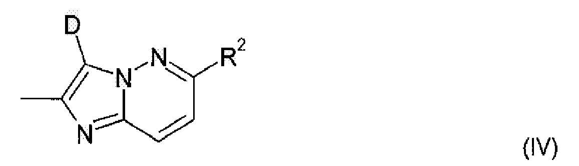 wherein R² is as defined for a compound of the formula I and D is -B(OH₂) in free form or in esterified form, e.g. as a group of the formula A shown under a), under cross-coupling conditions with a compound of the formula V,

   R¹-X (V)

   wherein R¹ is as defined for a compound of the formula I and X is halogen, especially chloro, bromo or iodo, or trifluoromethansulfonyloxy, or
c) reacting a compound of the formula VI, 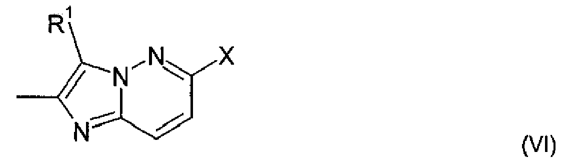 wherein R¹ is as defined for a compound of the formula I and' X is halo, especially chloro, bromo or iodo, or is trifluoromethansulfonyloxy, under cross-coupling conditions with a boronic acid or boronic acid ester of the formula VII,

   R²-D (VII)

   wherein R² is as defined for a compound of the formula I and D is -B(OH₂) in free form or in esterified form, e.g. as a group of the formula A shown under a), or
d) reacting a pyridazine compound of the formula VIII, 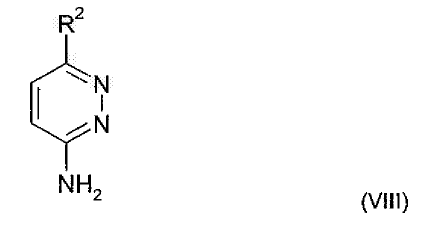
wherein R² is as defined for a compound of the formula I, with a haloketone of the formula IX, 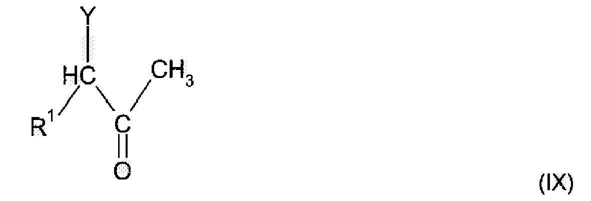 wherein R¹ is as defined for a compound of the formula I and Y is halo, especially chloro or bromo,
and, if desired, a compound of the formula I obtainable according to any one of the reactions a) to d) given above is converted into a different compound of the formula I, an obtainable salt of a compound of the formula I is converted into a different salt thereof, an obtainable free compound of the formula I is converted into a salt thereof, and/or an obtainable isomer of a compound of the formula I is separated from one or more different obtainable isomers of the formula I.

In the following more detailed description of preferred variants of the processes, optional reactions and conversions, synthesis of starting materials and intermediates and the like, R¹ and R² have the meanings given for a compound of the formula I or the compound mentionned specifically, while D is as defined for a compound of the formula III, X as for a compound of the formula II, Y as for a compound of the formula IX, Het as for a compound of the formula X and Hyl for a compound of the formula XI, in each case if not indicated otherwise, respectively.

Where useful or required, the reactions can take place under an inert gas, such as nitrogen or argon.

The reaction given under process variants a), b) and c), respectively, is preferably carried out under the conditions of a Suzuki-reaction, preferably in a mixture of a polar aprotic solvent, such as dimethylformamide (DMF) and optionally water in the presence of a catalyst for the cross-coupling, especially a noble metal catalyst, preferably a palladium catalyst, such as palladium(II) complex, for example bis(triphenylphosphine)palladium (II) dichloride, in the presence of a base, such as potassium carbonate, sodium hydroxide or sodium carbonate, at a preferred temperature in the range from 80 °C to 130 °C; or according to a another preferred method in a cyclic ether solvent, e.g. tetrahydrofurane, in the presence of a catalyst for the cross coupling, especially a noble metal catalyst, preferably a palladium (0) complex, for example tris(dibenzylideneacetone)-dipalladium(0), or of palladium dibenzylideneacetone as precursor, in the presence an appropriate ligand, such as 2-dicyclohexylphosphino-2',6'-dimethoxybiphenyl (SPhos) or 2-dicyclohexylphosphino-2'-(N,N-dimethylamino)-biphenyl (P1), and in the presence of a base, e.g. as mentioned above or potassium phosphate, and at a preferred temperatures in the range from 80 to 150 °C; if required conducting the reaction in a sealed vessel (e.g. a seal reactor) if the boiling point of the reaction mixture is exceeded and especially if (as is a preferred embodiment) the heating is effected by microwave excitation. Where required, other or additional catalyst can be added, e.g. (PdCl₂(PPh₂)˙Fe˙CH₂Cl₂).

The reaction between a compound of the formula VIII and a compound of the formula IX (reaction variant d) above) preferably takes place in an appropriate solvent, such as an alcohol, for example in ethanol, at elevated temperatures, e.g. in the range from 80 to 180 °C, e.g. at 100 to 170 °C, in the absence or if useful presence of a tertiary nitrogen base, such as a tri-(lower alkyl)-amine, for example triethylamine.

### Protecting groups

If one or more other functional groups, for example carboxy, hydroxy, amino, or mercapto, are or need to be protected in a starting material, e.g. in any one or more starting materials of the formula II or III or other starting materials, intermediates and educts mentioned below" because they should not take part in the reaction or disturb the reaction, these are such groups as are usually used in the synthesis of peptide compounds, and also of cephalosporins and penicillins, as well as nucleic acid derivatives and sugars. Protecting groups are such groups that are no longer present in the final compounds once they are removed, while groups that remain as substitutents are not protecting groups in the sense used here which is groups that are added at a certain intermediate stage and removed to obtain a final compound. For example, tert-butoxy if remaining in a compound of the formula I is a substituent, while if it is removed to obtain the final compound of the formula I it is a protecting group.

The protecting groups may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by acetolysis, protonolysis, solvolysis, reduction, photolysis or also by enzyme activity, for example under conditions analogous to physiological conditions, and that they are not present in the end-products. The specialist knows, or can easily establish, which protecting groups are suitable with the reactions mentioned above and below.

The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/I, Georg Thieme Verlag, Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.

### Optional Reactions and Conversions

A compound of the formula I may be converted into a different compounds of the formula I according to standard reaction procedures.

For example, in a compound of the formula I wherein R¹ is aryl, such as phenyl, that is substituted by halo, especially by chloro or bromo, e.g. in the p-position, the halo can be replaced by a unsubstituted or substituted ring nitrogen comprising unsaturated heterocyclyl bound via a ring nitrogen atom by reaction with a compound of the formula X,

H-Het (X)

wherein Het is an unsubstituted or substituted unsaturated heterocyclyl moiety bound to the hydrogen via a ring nitrogen atom, such as 1,2,4-triazol, pyrazole, benzimidazole, 3-trifluoromethyl-pyrazol, under Ullman-type reaction conditions, e.g. as in the reference mentioned in example 1, preferably by reacting the corresponding compound of the formula I and the compound of the formula X in the presence of Cu₂O, a ligand such as salicylaldehyde hydrazone, a base such as caesium carbonate and a solvent such as acetonitrile at preferred temperatures in the range from 100 to 180 °C, e.g. at 160 to 150 °C, for example in a microwave oven. This leads to a compound of the formula I wherein R¹ is aryl, e.g. phenyl, substituted by unsubstituted or substituted ring nitrogen comprising unsaturated heterocyclyl bound via a ring nitrogen atom.

Alternatively, for example, in a compound of the formula I wherein R¹ is aryl, such as phenyl, that is substituted by halo, especially by chloro or bromo, e.g. in the p-position, the halo can be replaced by an unsubstituted or substituted saturated heterocyclyl comprising a nitrogen atom by reaction with a compound of the formula XI,

H-Hyl (XI)

wherein Hyl is an unsubstituted or substituted saturated heterocyclyl moiety bound to the hydrogen via a ring nitrogen atom, such as valerolactame, morpholine, 2-pyrrolidinone or N-methylpiperazine, under reaction conditions such as those described in the reference mentioned in Example 14, e.g. reacting the heterocyclic compound of the formula XI and the corresponding compound of the formula I in the presence of Cul, a base, such as potassium carbonate, and of proline in an appropriate solvent, such as dimethylsulfoxide, preferably at temperatures in the range from 80 to 130 °C.

A compound of the formula I wherein R¹ is aryl, such as phenyl, that is substituted by cyano can be converted to a corresponding compound of the formula I wherein instead of the cyano an 1H-tetrazol-5-yl moiety is present by reaction with an azide salt, such as sodium azide, preferably in the presence of an ammonium salt, such as ammonium chloride, at a temperature e.g. from 120 to 160 °C.

A compound of the formula I wherein R¹ is aryl, such as phenyl, substituted by nitro can be reduced to a corresponding compound of the formula I wherein instead of the nitro an amino group is present, e.g. by reduction by hydrogenation in the presence of a hydrogennation catalyst, e.g. a noble metal catalyst, such as palladium, which can preferably be bound to a carrier, such as charcoal, in an appropriate solvent, such as an alcohol, e.g. methanol, preferably at temperatures in the range from 0 to 50 °C, e.g. at room temperature. As by-product, the alkylation product resulting from the alcohol can be obtained, e.g. in the case of methanol the corresponding methylamino compound of the formula I, which can be isolated according to standard procedures, such as chromatography.

In a compound of the formula I wherein R¹ is aryl, such as phenyl, substituted by chloro, bromo or iodo, the chloro, bromo or iodo can be converted into a group D as described above for a compound of the formula III, for example by reaction first with n-butylllithium (replacing the chloro, bromo or iodo by Li) and subsequent reaction with a corresponding trialkoxyborane, such as triisopropylborane; or by reaction of the chloro, bromo or iodo compound in the presence of a transition metal catalyst (e.g. PdCI(dppf) with alkoxydiborone), or the like. Alternatively, also triflate (trifluoromethanesulfonyloxy) substituents instead of halo can be substituted accordingly in corresponding starting materials.The free boronic acids (unesterified) can be obtained e.g. by working up in the presence of an inorganic acid, such as hydrochloric acid.

The compound of the formula I carrying a group D as just described can then be reacted with an aryl or unsaturated heterocyclyl compound under conditions as described above for reaction a) (e.g. cross coupling, such as Suzuki coupling) to a corresponding compound of the formula I wherein instead of the original chloro, bromo or iodo an aryl or unsaturated heterocyclyl substituent is present (each of which may be substituted as well as described above).

A nitrogen ring atom of the imidazo[1,2-b]pyridazine core or a nitrogen-containing heterocyclyl substituent can form an N-oxide in the presence of a suitable oxidizing agent, e.g. a peroxide, such as m-chloro-perbenzoic acid or hydrogen peroxide.

Also in the optional process steps, carried out "if desired", functional groups of the starting compounds which should not take part in the reaction may be present in unprotected form or may be protected for example by one or more of the protecting groups mentioned hereinabove under "protecting groups". The protecting groups are then wholly or partly removed according to one of the methods described there.

Salts of a compound of formula I with a salt-forming group may be prepared in a manner known *per se.* Acid addition salts of compounds of formula I may thus be obtained by treatment with an acid or with a suitable anion exchange reagent.

Salts can usually be converted to free compounds, e.g. by treating with suitable basic compounds, for example with alkali metal carbonates, alkali metal hydrogencarbonates, or alkali metal hydroxides, typically potassium carbonate or sodium hydroxide.

Mixtures of constitutional isomers or of products and by-products can be separated according to standard procedures, e.g. by distribution, chromatography or the like.

Stereoisomeric mixtures, e.g. mixtures of diastereomers, can be separated into their corresponding isomers in a manner known per se by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula 1 itself. Enantiomers may be separated through the formation of diastereomeric salts, for example by salt formation with an enantiomer-pure chiral acid, or by means of chromatography, for example by HPLC, using chromatographic substrates with chiral ligands.

It should be emphasized that reactions analogous to the conversions mentioned in this chapter may also take place at the level of appropriate intermediates (and are thus useful in the preparation of corresponding starting materials).

### Starting materials:

The starting materials of the formulae II, III, IV, V, VI, VII, VIII, IX and X as well as other starting materials, intermediates or educts mentioned herein, e.g. below, can be prepared according to or in analogy to methods that are known in the art, the materials are known in the art and/or are commercially available, or by or in analogy to methods mentioned in the Examples. Novel starting materials (e.g. in Example 29 the compound of Stage 29.1, 3-bromo-6-(3,4-dimethoxy-phenyl)-2-methyl-imidazo[1,2-b]pyridazine, or analogues wherein instead of the bromo a chloro or iodo or trifluoromethansulfonyloxy is present), as well as processes for the preparation thereof, are likewise an embodiment of the present invention. In the preferred embodiments, such starting materials are used and the reaction chosen are selected so as to enable the preferred compounds to be obtained.

Starting materials of the formula II are known in the art, commercially available or can be prepared according to or in analogy to methods known in the art.

For example, a compound of the formula II can be obtained by reacting a compound of the formula XII, 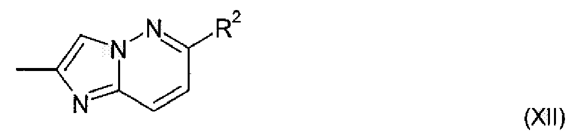 in the presence of a halogenating agent, e.g. N-iodo-, N-bromo- or N-chloro-succinimide (with N-bromosuccinimide being preferred), in an appropriate solvent, such as an alkylated amide, e.g. dimethyl formamide, or a halogenide, methylene chloride, chloroform or the like, e.g. at temperatures in the range from -20 to 50 °C, to the corresponding compound of the formula (II) wherein X is halo (preferably bromo).

A compound of the formula XII can, for example, be obtained by reacting a compound of the formula VIII with a halogenated acetone of the formula XIII, 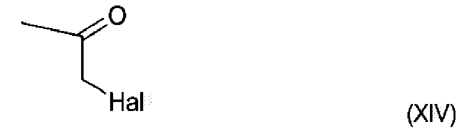 wherein Hal is halo, especially chloro, under conditions analogous to those described above (under process variant d)) for the reaction of a compound of the formula VIII with a haloketone compound of the formula IX.

A compound of the formula VIII can, for example, be obtained by reacting a pyridazine compound of the formula XV, 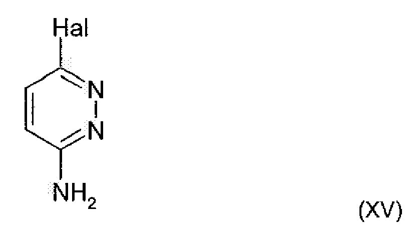 wherein Hal is halo, especially chloro or bromo, with a boronic acid or boronic acid ester of the formula VII mentioned above under conditions analogous to those mentioned above (for process variant c)) for the reaction of a compound of the formula VI and a compound of the formula VII.

A compound of the formula IV can for example be obtained from a compound of the formula Il by replacing the group D with a group -B(OH)₂ in free (obtainable in the presence of an acid, such as hydrochloric acid, from an esterified form) or esterified form e.g. under reaction conditions analogous to those mentioned under the conversions for a compound of the formula I wherein R¹ is aryl, such as phenyl, substituted by chloro, bromo or iodo, the chloro, bromo or iodo, into the corresponding compound wherein the chloro, bromo or iodo is replaced with a group -B(OH)₂ in free or preferably esterified form.

A compound of the formula VI can preferably be obtained by reaction of a compound of the formula XV mentioned above with a compound of the formula IX as defined under process variant d) under reaction conditions analogous to those mentioned above (for process variant d)) for the reaction of a compound of the formula VIII with a compound of the formula IX.

A compound of the formula IX can, for example, be prepared by reacting a compound of the formula XVI, 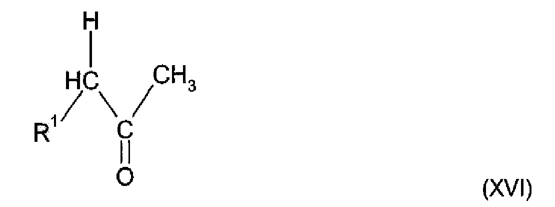 in the presence of a halogenating agent, e.g. an inorganic acid halide, such as a sulfuryl halogenide, preferably sulfurylchloride, in an appropriate solvent, e.g. methylene chloride, e.g. at temperatures in the range from -20 to 50 °C.

A compound of the formula XVI can, for example, be obtained by reacting a compound of the formula XVII,

R¹-Br (XVII)

with isopentyl acetate in the presence of tributyltin methoxide, a catalyst, e.g. Pd₂(dba)₃, and of 2-dicyclohexylphosphino-2'-(N,N-dimethylamino)-biphenyl in an appropriate solvent, e.g. toluene, at elevated temperatures, e.g. under reflux conditions.

Alternatively, a compound of the formula XVI can be obtained by reacting an aldehyde of the formula XVIII,

R¹-CHO (XVIII)

in the presence of nitroethane and ammonium acetate at an elevated temperature, e.g. 60 to 130 °C, followed by conversion of the resulting 2-nitropropenyl intermediate in the presence of iron powder (with or without addition of FeCl₃) and an acid, such as hydrogen chloride or acetic acid, in an aqueous solvent, e.g. at elevated temperature, for example at temperatures between 50 °C and reflux temperature of the reaction mixture.

All remaining starting materials such as starting materials of the formula III, V, VII, X, XI, XIV, XV, XVII or XVIII, are known, capable of being prepared according to known processes, or commercially obtainable; in particular, they can be prepared using processes as described or in analogy to those described in the Examples.

### Examples:

The following examples illustrate the invention without limiting the scope thereof.

Temperatures are given in degree Celsius (°C). Where no temperature is given, the reaction is at room temperature. Ratios of solvents or eluents are given as volume per volume (v/v).

**Abbreviations:**

| | |
|---|---|
| abs | absolute |
| aq | aqueous |
| Boc | tert-butoxycarbonyl |
| brine | sodium chloride solution saturated at RT |
| DMA | N,N-dimethylacetamide |
| DMF | N,N' dimethyl formamide |
| DMSO | dimethylsulfoxide |
| Emrys | Optimizer Emrys^{™} Optimizer, Microwave oven from Personal Chemistry, Biotage AB, Uppsala, Sweden |
| eq. | equivalent(s) |
| ether | diethyl ether |
| EtOH | ethanol |
| EtOAc | ethyl acetate |
| DCM | dichloromethane |
| h | hour(s) |
| HPLC | High Performance Liquid Chromatography |
| Hyflo | Hyflo® Super Cel is a diatomaceous earth used in filtration processes, obtainable e.g. from Fluka, Buchs, Switzerland |
| K₃PO₄ | tripotassium phosphate |
| LC-MS | Liquid Chromatography-Mass Spectrometry |
| min | minute(s) |
| mL | milliliter(s) |
| MS-ES | electrospray mass spectrometry |
| MW | microwave |
| NEt₃ | triethylamine |
| NMP | 1-methyl-2-pyrrolidinone |
| P1 | 2-dicyclohexylphosphino-2'-(N,N-dimethylamino)-biphenyl |
| Pd(dba)₂ | palladium dibenzylidenacetone |
| Pd₂(dba)₃ | palladium trisdibenzylidenacetone |
| Ph | phenyl |
| rotavap | Rotary Evaporator |
| RT | room temperature |
| sat | saturated |
| Sphos | 2-dicyclohexylphosphino-2',6'-dimethoxybiphenyl |
| TBME | tert-butyl methyl ether |
| TFA | trifluoro acetic acid |
| THF | tetrahydrofurane |
| TLC | thin layer chromatography |
| t_{R} | retention time |
| UV | Ultraviolet |

Boronic acid starting materials mentioned in the examples can be obtained as follows:
3,4-dimethoxyphenylboronic acid (Frontier Scientific, Inc., Logan, UT, USA)
3-pyridine boronic acid (= pyridine-3-boronic acid) (Fluorochem Ltd., Derbyshire, United Kingdom)
5-pyrimidinyl boronic acid (= pyrimidin-5-yl boronic acid) (Fluorochem Ltd., Derbyshire, United Kingdom)
4-(aminocarbonyl)-phenyl-boronic acid, 4-(morpholin-4-carbonyl)phenylboronic acid, 4-(N,N-dimethylsulfonamidophenyl)boronic acid, and 4-ethylsulfonylphenyl) boronic acid (Combi-Blocks Inc., San Diego, California, USA)

### Example 1: 6-(3,4-Dimethoxy-phenyl)-2-methyl-3-(4-[1,2,4]triazol-1-yl-phenyl)-imidazo[1,2-b]pyridazine

(For lit. reference see e.g. Chem. Eur. J. (2004), 10, 5607 on the general Ullmann-type arylation of nucleophiles). In a 2 ml vial for microwave with crown cap and magnetic stir bar, a mixture of 50 mg (0.118 mmol) 3-(4-bromo-phenyl)-6-(3,4-dimethoxy-phenyl)-2-methyl-imidazo[1,2-b]pyridazine, 12.2 mg (0.177mmol) 1,2,4-triazole, 0.24 mg Cu₂O, 3.2 mg salicylaldehyde hydrazone, and 77.6 mg (0.236 mmol) Cs₂CO₃ in 2 ml acetonitrile are heated in a microwave oven (Emrys Optimizer,) at 160°C for 20 h. The reaction mixture is diluted with dichloromethane, filtered over hyflo, and the solvent evaporated. Dichloromethane is added again, and the organic phase is washed with water (2x) and brine (2x). After drying over MgSO₄, the reaction mixture is evaporated under reduced pressure. The crude product is purified by chromatography on silica gel (CH₂Cl₂-EtOAc; gradient, 100/0 to 20/80) to give the pure product. LC-MS: t_{R} = 1.63 min; (M+1) = 143; MS-ES.: 143 (M+1).

### The starting material is prepared as follows:

Stage 1.1: 3-(4-Bromo-phenyl)-6-(3,4-dimethoxy-phenyl)-2-methyl-imidazo[1,2-b]pyridazine Note that this compound is also a compound of the formula I according to the invention. In a 6 ml vial for microwave with crown cap and magnetic stir bar, a mixture of 270 mg (1.17mmol) 6-(3,4-dimethoxy-phenyl)-pyridazin-3-ylamine and 433 mg (crude product; ca. 1.15 mmol) of 1-(4-bromo-phenyl)-1-chloro-propan-2-one in 2 ml of ethanol are heated in a microwave oven (Emrys Optimizer) at 170°C for 2 h. To the reaction mixture, a solution of saturated NaHCO₃ is added and thereafter extracted with EtOAc. The organic phase is washed with brine and dried (MgSO₄). The solvent is evaporated, and the residue is purified by chromatography on silica gel (solvent system: CH₂Cl₂-EtOAc 100:0 (start) to 80:20 (end). The yellow solid obtained is suspended in diethyl ether, filtered and dried at 50°C for 2 h to yield the title compound. LC-MS: t_{R} = 1.86 min; (M+1) = 425; HPLC: t_{R} = 5.53 min. (A small amount (ca. 3%) of the isomeric 2-(4-bromo-phenyl)-6-(3,4-dimethoxy-phenyl)-3-methyl-imidazo[1,2-b]pyridazine is equally isolated during the chromatography. LC-MS: t_{R} = 1.86 min; (M+1) = 425; HPLC: t_{R} = 5.46 min.)

### Stage 1.2: 6-(3,4-Dimethoxy-phenyl)-pyridazin-3-ylamine

In a round bottom flask, 500 mg (3.86 mmol) of 3-amino-6-chloropyridazine, 840 mg (2.78 mmol) 3,4-dimethoxyphenylboronic acid, 97.5 mg Pd-catalyst [PdCl₂(PPh₃)₂], and 5.8 ml aqueous K₂CO₃ 1M are heated under inert conditions in 10 ml DMF at 105°C for 20 h. After that time, a saturated solution of NaHCO₃ is added and the mixture is extracted with CH₂Cl₂. The organic phase is dried and the solvent evaporated. The residue is purified by chromatography on silica gel. The beige solid is further suspended in methanol, filtered off and dried under high vacuum to yield the title compound. MS-ES.: (M+1) = 232; (M-1) = 230; HPLC: t_{R} = 2.76 min.; LC-MS: t_{R} = 1.41 min; (M+1) = 232.

### Stage 1.3: 1-(4-Bromo-phenyl)-1-chloro-propan-2-one.

The compound is prepared anaogoulsly to a procedure known in the literature (see J. Org. Chem. (2001), 66,3617). To a solution of 3.5 g (16.4 mmol) of 4-bromophenyl acetone in 10 ml of CH₂Cl₂, a solution of 1.58 ml (19.7 mmol) of sulfuryl chloride in 10 ml of CH₂Cl₂ is added slowly at 0°C. The reaction mixture is stirred overnight at this temperature. To quench the reaction, water is added and the two phases are separated. The aqueous phase is washed twice with CH₂Cl₂, the organic phases are combined, washed with brine and dried. After evaporation of the solvent, the title compound is used without further purification. HPLC; t_{R} = 6.5 min.

### Example 2: 6-(3,4-Dimethoxy-phenyl)-2-methyl-3-(4-nitro-phenyl)-imidazo[1,2-b]pyridazine

In a 20 ml vial for microwave with crown cap and magnetic stir bar, a mixture of 250 mg (0.866 mmol) of 6-chloro-2-methyl-3-(4-nitro-phenyl)-imidazo[1,2-b]pyridazine, 211 mg (1.13 mmol) of 3,4-dimethoxybenzene boronic acid, 20 mg (0.0346 mmol) of Pd(dba)₂, 551 mg (2.6 mmol) of K₃PO₄ and 28 mg (0.0693 mmol) SPhos in 10 ml anhydrous THF are heated under inert conditions in a microwave oven (Emrys Optimizer) at 110°C for 1 h. The reaction mixture is poured into water and extracted with CH₂Cl₂. The organic phase is washed with brine, dried, and evaporated to dryness. Purification is done by chromatography on silica gel with CH₂Cl₂-EtOAc (100/0 to 50/50, gradient) as solvent system to yield the title compound. MS-ES: (M+1) = 391; LC-MS: t_{R} = 1.84 min, (M+1) = 391.

### The starting material is prepared as follows:

### Stage 2.1. 6-Chloro-2-methyl-3-(4-nitro-phenyl)-imidazo[1,2-b] pyridazine

In a 6 ml microwave vial with crown cap and magnetic stir bar, a mixture of 1.1 g (8.49 mmol) of 6-chloropyridazine-3-amine and 2.38 g (crude product; ca. 11.1 mmol) of 1-chloro-1-(4-nitro-phenyl)-propan-2-one in 3.5 ml of ethanol are stirred in a microwave oven (Emrys Optimizer) for 1 h at 170°C. After that time, the reaction is poured into sat. NaHCO₃-solution and extracted with EtOAc. The organic phases are combined and washed with brine, and the solvent is then evaporated. The residue is purified by chromatography (CH₂Cl₂-EtOAc: gradient, 100:0 to 80:20. The product is suspended in methanol, filtered off, and dried under high vacuum. MS-ES.: M+ = 289. HPLC: t_{R} = 5.2 min.

### Stage 2.2: 1-Chloro-1-(4-nitro-phenyl)-propan-2-one,

In analogy to Example 1.3, the title compound is obtained from 1 g (5.58 mmol) of 4-nitrophenyl acetone and sulfuryl chloride in CH₂Cl₂ at 0°C. The crude product is used without purification in the next step.

### Example 3: 6-(3,4-Dimethoxy-phenyl)-2-methyl-3-(4-pyrazol-1-yl-phenyl)-imidazo[1 ,2-b]pyridazine

In analogy to Example 1, the title compound is prepared from 50 mg (0.118mmol) 3-(4-bromo-phenyl)-6-(3,4-dimethoxy-phenyl)-2-methyl-imidazo[1,2-b]pyridazine (stage 1.1), 12.0 mg (0.177mmol) pyrazole, ca. 1 mg Cu₂O, ca. 4 mg salicylaldehyde hydrazone (ligand), and 77.6 mg (0.236mmol) Cs₂CO₃ in 2 ml acetonitrile. Reaction time in the microwave oven is 6 h at 160°C. MS-ES: 412. LC-MS: t_{R} = 1.70 min.

### Example 4: 3-(4-Benzoimidazol-1-yl-phenyl)-6-(3,4-dimethoxy-phenyl)-2-methyl-imidazo[1,2-b]pyridazine

In analogy to Example 1, the title compound is prepared from 75 mg (0.177 mmol) 3-(4-bromo-phenyl)-6-(3,4-dimethoxy-phenyl)-2-methyl-imidazo[1,2-b]pyridazine (stage 1.1), 31.3 mg (0.177mmol) benzimidazole, ca. 1.2 mg Cu₂O, ca. 5 mg salicylaldehyde hydrazone and 116 mg (0.0.354 mmol) Cs₂CO₃ in 2 ml acetonitrile. Reaction time in the microwave oven is 6 h at 160°C. MS-ES: (M+1) = 462;. LC-MS: t_{R} = 1.65 min., (M+1) = 462.

### Example 5 : 4-[6-(3,4-Dimethoxy-phenyl)-2-methyl-imidazo[1,2-b] pyridazin-3-yl]-benzonitrile

In a 20 ml vial for microwave with crown cap and magnetic stir bar, a mixture of 500 mg (1.86 mmol) 4-(6-chloro-2-methyl-imidazo[1,2-b]pyridazin-3-yl)-benzonitrile, 779 mg (4.28 mmol, added in two portions) 3,4-dimethoxyphenyl boronic acid, 42.8 mg Pd(dba)₂, 1.18 g (5.58 mmol) K₃PO₄, and 61.1 mg SPhos in 15 ml anhydrous THF is heated in a microwave oven (Emrys Optimizer) for a total of 11 h at 110°C (2 h), 120°C (2 h), 130°C (5 h), and after addition of 1 more equivalent of the boronic acid, 2 more hours at 110°C (reaction control by HPLC). The reaction mixture is poured into water and extracted with EtOAc. The organic phase is washed with brine and water and then dried, and the solvent is evaporated. The residue is purified by chromatography on silica gel. Solvent system: CH₂Cl₂-EtOAc 100:0 to 230:70 (gradient; first chromatography) and CH₂Cl₂-MeOH 95:5 (second chromatography). LC-MS: t_{R} = 1.81 min., (M+1) = 371.

### Stage 5.1 : 4-(6-Chloro-2-methyl-imid'azo[1,2-b]pyridazin-3-yl)-benzonitrile

In a 20 ml vial for microwave with crown cap and magnetic stir bar, 0.98 g (7.56 mmol) of 6-chloropyridazine-3-amine and 1.9 g (crude product; ca. 9.81 mmol) of 4-(1-chloro-2-oxo-propyl)-benzonitrile in 5 ml EtOH abs. are heated in a microwave oven for a total of 7.5 h at 100°C (3 h 15 min), 120°C (2 h), and 140°C (6 h). After that time, the reaction mixture is poured into aqueous NaHCO₃, and extracted with EtOAc. The organic phase is washed with brine and water. The crude product is purified by chromatography (solvent system: CH₂Cl₂-EtOAC = 100/0 to 50/50; gradient). LC-MS: t_{R} = 1.84 min., (M+1) = 269; HPLC: t_{R} = 4.65 min.

### Stage 5.2: 4-(1-Chloro-2-oxo-propyl)-benzonitrile.

In analogy to Example 1.3. the title compound is prepared from 1.95 g (12.2 mmol) 4-(2-oxopropyl)-benzonitrile and 1.13 ml (14 mmol) sulfuryl chloride in 9.7 ml CH₂Cl₂ at 0°C and 3 h under stirring. HPLC: t_{R} = 5.35 min. The crude product is used in the next step without further purification.

### Stage 5.3: 4-(2-Oxo-propyl)-benzonitrile

(For reference on the general method see Bull. Chem. Soc. Jpn. (1984), 57, 242.) In a round bottom flask equipped with reflux condenser, a mixture of 10 g (52.2 mmol) of 4-bromobenzonitrile, 9.02 ml of isopropenyl acetate, 24 ml of tributyltin methoxide, 502 mg (0.548 mmol) af Pd₂(dba)₃ and 865 mg (2.2 mmol) of P1 in 27.5 ml of toluene is stirred for 16 h at reflux temperature. The reaction mixture is poured into water and the two phases are separated. The organic phase is washed with water, and the aqueous phase is extracted with CH₂Cl₂. The two organic phases are combined and evaporated to dryness. Purification of the product is achieved by chromatography on silica gel. Solvent system: hexane-EtOAc 80:20 to 50:50. Further material can be isolated from the impure fractions by reversed phase chromatography. The title compound is obtained as a yellowish solid. LC-MS: t_{R} = 1.70 min., (M+1) = 160; HPLC: t_{R} = 4.4 min.

### Example 6: 6-(3,4-Dlmethoxy-phenyl)-2-methyl-3-(4-pyridin-3-yl-phenyl)-irnidazo[1 ,2-b]pyridazine

In a 6 ml vial for microwave with crown cap and magnetic stir bar, a mixture of 50 mg (0.118 mmol) of 3-(4-bromo-phenyl)-6-(3,4-dimethoxy-phenyl)-2-methyl-imidazo[1,2-b] pyridazine, 21.7 mg (0.177 mmol) of 3-pyridineboronic acid, 2.71 mg of Pd(dba)₂ and 75 mg of K₃PO₄, 3.87 mg SPhos in 2 ml of anhydrous THF are stirred under inert atmosphere at 110°C for 2 h in a microwave oven (Emrys Optimizer). After that time, the reaction mixture is poured into water and extracted with CH₂Cl₂. The organic phase is washed with brine, dried over MgSO₄ and concentrated to dryness. The residue is purified by chromatography on silica gel. Solvent system: CH₂Cl₂-EtOAc 50:50 to 100:0. The title compound is obtained as a yellow solid. MS-ES: (M+1) = 423; HPLC: t_{R} = 3.4 min.; LC-MS: t_{R} = 1.65 min., (M+1) = 423.

### Example 7: 6-(3,4-Dimethoxy-phenyl)-2-methyl-3-[4-(1H-tetrazol-5-yl)-phenyl]-imidazo[1,2-b]pyridazine

(For reference on the general method see J. Heteroc. Chem. (1999), 36, 1129.) In a 3 ml vial for microwave with crown cap and magnetic stir bar, a mixture of 50 mg (0.135 mmol) of 4-[6-(3,4-dimethoxy-phenyl)-2-methyl-imidazo[1,2-b]pyridazin-3-yl]-benzonitrile, a total of 106 mg (1.63 mmol) of sodium azide, and a total of 88.5 mg (1.65 mmol) of ammonium chloride in 2 ml of DMF is heated in a microwave oven (Emrys Optimizer) at 150°C for 8 h. The progress of the reaction is monitored by HPLC, and every second hour 2 more equivalents of sodium azide and ammonium chloride is added until the cyano-starting material is consumed. The reaction mixture is concentrated under reduced pressure and suspended in 2N HCl and CH₂Cl₂. The title compound precipitates as a yellow powder, which is filtered off, washed with water, and dried at 70°C under high vacuum. HPLC: t_{R} = 2.89 min; ES-MS: 414 (M+1); LC-MS: t_{R} = 1.33 min, (M+1) = 414.

### Example 8: 6-(3,4-Dimethoxy-phenyl)-3-(4-methanesulfonyl-phenyl)-2-methyl-imidazo[1,2-b]pyridazine

The title reaction is run in two portions in 2 ml vial for microwave with crown cap and magnetic stir bar; the relative amounts are given in parenthesis. A mixture of 112 mg (50 and 62mg; 0.48 mmol) of 6-(3,4-dimethoxy-phenyl)-pyridazin-3-ylamine, 181 mg (63 and 118 mg; 0.587 mmol) of 1-chloro-1-(4-methanesulfonyl-phenyl)-propan-2-one, and 109 µl (38 and 71 µl) NEt₃ in 1.5 ml (0.5 and 1 ml) EtOH is heated under Argon (Ar) in a microwave oven (Emrys Optimizer) for 30 min at 170°C. HPLC and TLC shows no more starting material. The reaction mixture is poured into CH₂Cl₂, washed with water and brine, and dried with Na₂SO₄. Purification is achieved by chromatography on silica gel. Solvent system: CH₂Cl₂-MeOH 100/0 to 0/100 (gradient). The title compound is further purified by precipitation from CH₂Cl₂ with diethylether. HPLC: t_{R} = 4.06 min.; ES-MS: 424 (M+1).

The starting material is prepared as follows:

### Stage 8.1: 4-Methanesulfonyl-benzaldehyde

2.5,g (17.4 mmol) of 4-chloro-benzaldehyde and 2.62 g (21.8 mmol) of sodium methane-sulfinate in 25 ml of DMSO are heated under argon for 18 h at 150°C. HPLC shows only minor amount of starting material. The reaction mixture is cooled to RT and poured into ice-water. A precipitate is formed and stirred for 30 min. The solid is filtered, washed with water and dried under high vacuum for 20 h at 50°C. The title compound is used in the next step without further purification. HPLC: t_{R} = 3.04 min.; ES-MS: 183 (M-1).

### Stage 8.2: 1-(4-Methanesulfonyl-phenyl)-propan-2-one

4-Methanesulfonyl-benzaldehyde (1.75 g; 9.02 mmol), nitroethan (5.24 ml; 72.2 mmol) and ammonium acetate (211 mg; 2.71 mmol) are heated for 5 h at 125°C. The excess reagent is removed by evaporation on a rotavap, then CH₂Cl₂ is added, and the mixture is washed with water and brine. After drying over Na₂SO₄, the solvent is removed and thereafter the 1-methanesulfonyl-2-nitro-propenyl)-benzene intermediate is suspended in 11 ml of water. Iron powder (1.98 g; 35.2 mmol) and FeCl₃ · 6H₂O (48.8 mg; 0.18 mmol) are added, and the mixture is heated to reflux. At that temperature, 5 ml of 2M HCl is added slowly during 1 h and the reaction is run for additional 7 h at reflux. To the cold reaction mixture, CH₂Cl₂ is added and filtered over hyflo. The organic phase is separated, washed with water and brine and dried over Na₂SO₄. After evaporation of the solvent, the title compound is obtained. HPLC: t_{R} = 3.21 min.; ES-MS: 211 (M-1).

### Stage 8.3: 1-Chloro-1-(4-methanesulfonyl-phenyl)-propan-2-one

In analogy to Example 1.3. the title compound is prepared from 150 mg (0.67 mmol) 1-(4-methanesulfonyl-phenyl)-propan-2-one and 66 µl (0.8 mmol) sulfuryl chloride in 3 ml CH₂Cl₂ at 0°C and 22 h stirring. HPLC: t_{R} = 4.42 min.; ES-MS: 245 (M-1). The crude product is used in the next step without further purification.

### Example 9: 6-(3,4-Dimethoxy-phenyl)-2-methyl-3-(4-pyrimidin-5-yl-phenyl)-imidazo[1,2-b]pyridazine

In a 6 ml vial for microwave with crown cap and magnetic stir bar, a mixture of 50 mg (0.118 mmol) of 3-(4-bromo-phenyl)-6-(3,4-dimethoxy-phenyl)-2-methyl-imidazo[1,2-b] pyridazine, 21.9 mg (0.177 mmol) of 5-pyrimidinylboronic acid, 2.71 mg of Pd(dba)₂, 75 mg of K₃PO₄ and 3.87 mg SPhos in 3 ml of anhydrous THF is stirred under inert atmosphere at 110°C for 2 h in a microwave oven (Emrys Optimizer). After that time, the reaction is not completed and additional base (K₂CO₃; 49.4 mg) and a different catalyst (PdCl₂(PPh₂)˙Fe˙CH₂Cl₂) are added, and the mixture is heated again at 110°C for 20 h. The mixture is poured into water and extracted with CH₂Cl₂. The organic phase is dried over Na₂SO₄ and concentrated to dryness. The residue is purified by combi-flash chromatography on silica gel. Solvent system: CH₂Cl₂-EtOAc 100:0 to 50:50. The title compound is obtained as a bright-yellow solid. MS-ES.: (M+1) = 424; HPLC: t_{R} = 4.134 min. Traces of debrominated starting material can be found.

### Example 10: 6-(3,4-Dimethoxy-phenyl)-3-(4-iodo-phonyl)-2-methyl-imidazo[1,2-b]pyridazine

In a 20 ml vial for microwave with crown cap and magnetic stir bar, a mixture of 0.6 g ((2.59 mmol) 6-(3,4-dimethoxy-phenyl)-pyridazin-3-ylamine, 2.46 g of 1-chloro-1-(4-iodo-phenyl)-propan-2-one (crude product; ca. 5.19 mmol), and 1.09 ml (7.78 mmol) of Et₃N in 10 ml of ethanol are heated in a microwave (Emrys Optimizer) at 170°C for 30 min. The reaction mixture is poured into a saturated solution of NaHCO₃ and extracted with dichloromethane. The organic phase is washed with brine, dried (Na₂SO₄), filtered and concentrated under reduced pressure. The product is purified by combi-flash chromatography. Solvent system: gradient of CH₂Cl₂/MeOH 100:0 to 95:5. The fractions containing the title compound are collected and concentrated under reduced pressure. The residue is suspended in diethyl ether, filtered and dried under high vacuum at 50°C for 1 hr. A beige solid id obtained. MS: (M+1) =472; HPLC: t_{R} = 5.667 min.

The starting material is obtained as follows:

### Stage 10.1: 1-Chloro-1-(4-iodo-phenyl)-propan-2-one

The title compound is prepared in analogy to the chloro-propanone prepared in stage 1.3, starting from 2 g (7.69 mmol) of 1-(4-iodo-phenyl)-propan-2-one, 1.85 ml (23.1 mmol) sulfuryl chloride and 15 ml CH₂Cl₂. The product is used without purification in the next step. HPLC; t_{R} = 6.78 min.

### Stage 10.2: 1-(4-lodo-phenyl)-propan-2-one

A mixture of 3 g (12.8 mmol) of 4-iodobenzaldehyde (Pfaltz-Bauer) and 302 mg (3.88 mmol) of ammonium acetate in 7.51 ml of nitroethane is stirred under argon for 15 h at 125°C. The reaction mixture is then concentrated under reduced pressure. The residue is dissolved in CH₂Cl₂ and the organic phase washed with water and brine. After drying (Na₂SO₄), the solvent is evaporated and the residue is solubilized in a few ml of glacial acetic acid. This solution is added slowly to a slurry of 2.9 g (51.7 mmol) of iron powder in 8 ml of glacial acetic acid at 60°C. The reaction is stirred overnight at 100°C. The cold reaction mixture is poured into ice water. The brown suspension is filtered over hyflo and the residue on the filter is washed with ample CH₂Cl₂. The filtered phase is separated off, the organic phase is washed successively with 1 M hydrochloric acid, sat. NaHCO₃ solution, water and brine. After drying over MgSO₄, the solvent is evaporated and the residue purified by flash chromatography. Solvent system: hexane-ethylacetate = 90:10. HPLC; t_{R} = 6.14 min.

### Example 11: 6-(3,4-Dimethoxy-phenyl)-3-(3-fluoro-4-methoxy-phenyl)-2-methyl-imidazo[1,2-b]pyridazine

The title compound is prepared in the microwave (30 min at 170°C) in analogy to the compound specified in Example 8, starting from 100 mg (0.411 mmol) of 6-(3,4-dimethoxy-phenyl)-pyridazin-3-ylamine (preparation see Stage 1.2), 167 mg (0.616 mmol) of 1-chloro-1-(3-fluoro-4-methoxy-phenyl)-propan-2-one and 0.115 ml (0.822 mmol) Et₃N in 1.5 ml of ethanol. The purification of the crude product is achieved by chromatography on silica gel. Solvent gradient: hexane-ethylacetate 50:50 (start) to 100% ethylacetate (end). The title compound is isolated as a ochre solid, which is suspended in ethylacetate (few ml) and hexane and stirred while cooling with ice-water. The yellow solid is filtered off and dried to yield the pure title compound. MS: (M+1) =394; HPLC: t_{R} = 4.92 min.

The starting material is prepared as follows:

### Stage 11.1: 1-Chloro-1-(3-fluoro-4-methoxy-phenyl)-propan-2-one

The title compound is prepared in analogy to the chloro-propanone prepared in stage 1.3, starting from 150 mg (0.69 mmol) of 1-(3-fluoro-4-methoxy-phenyl)-propan-2-one, 0.068 ml ml (0.83 mmol) sulfuryl chloride and 3 ml CH₂Cl₂. The product is used without purification in the next step.

### Stage 11.2: 1-(3-Fluoro-4-methoxy-phenyl)-propan-2-one

### Part A:

2.5 g (16.1 mmol) of 3-fluoro-4-methoxy-benzaldehyde (SynChem) and 375 mg (4.82 mmol) of ammonium acetate in 9.32 ml of nitroethane are heated at 125°C for 24 h. The excess reagent is removed by evaporation on a rotavap, CH₂Cl₂ is added and the mixture is washed with water and brine. After drying over Na₂SO₄, the solvent is removed and thereafter the obtained nitro intermediate is suspended in 18 ml of water. Iron powder (3.51 g; 62.6 mmol) and FeCl₃ · 6H₂O (86.8 mg; 0.32 mmol) are added, and the mixture is heated to reflux. At that temperature, 8.83 ml of 1M HCl is added slowly during 1 h and the reaction is run for additional 8 h at reflux. To the cold reaction mixture, CH₂Cl₂ is added and the mixture is filtered over hyflo. The organic phase is separated, washed with water and brine and dried over Na₂SO₄. After evaporation of the solvent, the product obtained is identified as the 2-fluoro-1-methoxy-4-((E)-2-nitro-propenyl)-benzene intermediate, indicating that no reduction took place MS: (M+1) =212; HPLC: t_{R} = 6.42 min.

### Part B:

1 g (4.5 mmol) of the unreduced material obtained above is dissolved in 7 ml of glacial acetic acid and added dropwise during 1 h to a suspension of 2.52 g iron powder in 15 ml of glacial acetic acid at 60°C. Thereafter, the reaction mixture is stirred at 100°C for 22 h. The cold reaction mixture is poured into ice-water and stirred for 15 min. CH₂Cl₂ and water are added and the mixture is filtered over hyflo. The phases are separated, the water phase is extracted again with CH₂Cl₂, and the combined organic phases are washed with 1 M HCl (1x), sat. sodium bicarbonate (1x), water (2x) and brine (1x). After drying and evaporation of the solvent, the crude product is purified by chromatography on silica gel. Solvent: hexane-ethylacetate 90:10. MS: (M+1) =183; HPLC: t_{R} = 4.93 min.

### Example 12a and 12b: 4-[6-(3,4-Dimethoxy-phenyl)-2-methyl-imidazo[1,2-b]pyridazin-3-yl]-phenylamine 12a and {4-[6-(3,4-Dimethoxy-phenyl)-2-methyl-imidazo[1,2-b]pyridazin-3-yl]-phenyl}-methyl-amine 12b

200 mg of 6-(3,4-dimethoxy-phenyl)-2-methyl-3-(4-nitro-phenyl)-imidazo[1,2-b] pyridazine (for preparation see Example 2) in 10 ml of methanol are hydrogenated over Pd-10% C for 37 h at RT (hydrogen balloon). The mixture is filtered over hyflo and the solvent is evaporated. The residue is purified by chromatography on silica gel-C18. Solvent gradient: acetonitrile-water. Analytical data for amine 12a: MS: (M+1) =361; HPLC: t_{R} = 3.22 min.; analytical data for methyl-amine 12b: MS: (M+1) =375; HPLC: t_{R} = 3.87 min. Approximate weight ratio of 12a to 12b obtained: 4 : 3.

### Example 13: 6-(3,4-Dimethoxy-phenyl)-2-methyl-3-[4-(3-trifluoromethyl-pyrazol-1-yl)-phenyl]-imidazo[1,2-b]pyridazine

In analogy to Example 1, the title compound is prepared from 75 mg (0.177mmol) 3-(4-bromo-phenyl)-6-(3,4-dimethoxy-phenyl)-2-methyl-imidazo[1,2-b]pyridazine (for preparation see Stage 1.1), 36 mg (0.265mmol) 3-(trifluormethyl)-pyrazole (Fluka, Buchs, Switzerland), 1.5 mg Cu₂O, 5.5 mg salicylaldehyde hydrazone (Aldrich, Sigma-Aldrich, Buchs, Switzerland)), and 120 mg (0.364 mmol) Cs₂CO₃ in 2 ml acetonitrile. Reaction time in the microwave oven is 4 h at 160°C (1 h) and 150°C (3 h). MS-ES (M+1): 480. HPLC t_{R} = 5.86 min.

### Example 14: 1-{4-[6-(3,4-Dimethoxy-phenyl)-2-methyl-imidazo[1,2-b]pyridazin-3-yl]-phenyl}-piperidin-2-one

(For reference regarding the general method see J. Org. Chem. (2005), 70, 5164.) A mixture of 50 mg (0.106 mmol) of 6-(3,4-dimethoxy-phenyl)-3-(4-iodo-phenyl)-2-methyl-imidazo[1,2-b]pyridazine (for preparation see Example 10), 13.7 mg (0.138 mmol) of valerolactame, 2.02 mg of Cul, 29.6 mg (0.212 mmol) of K₂CO₃ and 3.05 mg of L-proline in 2 ml of DMSO is stirred for 24 h at 130°C. The cooled mixture is partitioned between water and CH₂Cl₂ and filtered over hyflo. The phases are separated, and the aqueous phase is extracted with CH₂Cl₂. The combined organic phases are washed with brine, dried over Na₂SO₄ and evaporated to dryness. The crude product is purified by combi-flash chromatography. Solvent system: hexane-ethylacetate 100/0 (start) to 0/100 (end). The isolated solid is suspended in ether, filtered and dried under high vacuum to yield the title compound as yellow solid. LC-MS: 443 (M+1); t_{R} = 1.43 min. HPLC: t_{R} = 4.2 min.

### Example 15: 6-(3,4-Dimethoxy-phenyl)-2-methyl-3-(4-morphotin-4-yl-phenyl)-imidazo[1,2-b]pyridazine

The title compound is prepared in analogy to Example 14 from 50 mg of 6-(3,4-dimethoxyphenyl)-3-(4-iodo-phenyl)-2-methyl-imidazo[1,2-b]pyridazine (for preparation see Example 10), 19 mg of morpholine, 2 mg of Cul, 30 mg of K₂CO₃ and 3 mg of L-proline in 2 ml of DMSO. Reaction time: 15 h at 100°C. MS: 431 (M+1). HPLC: t_{R} = 4.59 min.

### Example 16: 1-{4-[6-(3,4.Dimethoxy-phenyl)-2-methyl-imidazo[1,2-b]pyridazin-3-yl]-phenyl}-pyrrolidin-2-one

The title compound is prepared in analogy to Example 14 from 50 mg of 6-(3,4-dimethoxyphenyl)-3-(4-iodo-phenyl)-2-methyl-imidazo[1,2-b]pyridazine (for preparation see Example 10), 18 mg of 2-pyrrolidinone, 2 mg of Cul, 30 mg of K₂CO₃ and 3 mg of L-proline in 2 ml of DMSO. Reaction time: 15 h at 100°C. MS: 429 (M+1). HPLC: t_{R} = 4.31 min.

### Example 17: 6-(3,4-Dimethoxy-phenyl)-2-methyl-3-[4-(4-methyl-piperazin-1-yl)-phenyl]-imidazo[1,2-b]pyridazine

The title compound is prepared in analogy to Example 14 from 50 mg of 6-(3,4-dimethoxyphenyl)-3-(4-iodo-phenyl)-2-methyl-imidazo[1,2-b]pyridazine (for preparation see Example 10), 0.024 ml of N-methylpiperazine (Fluka, Buchs, Schweiz), 2 mg of Cul, 30 mg of K₂CO₃, and 3 mg of L-proline in 2 ml of DMSO. Reaction time: 15 h at 100°C. MS: 444 (M+1). HPLC: t_{R} = 3.27 min.

The compounds depicted in the following examples can be prepared in analogy to the methods described herein or as specifically described:

### Example 18:

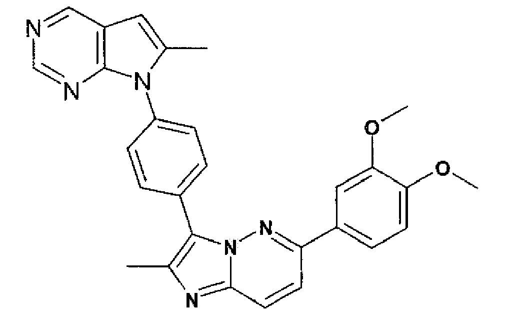

### Example 19:

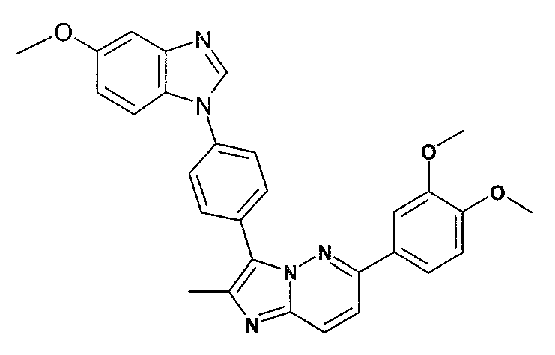

### Example 20:

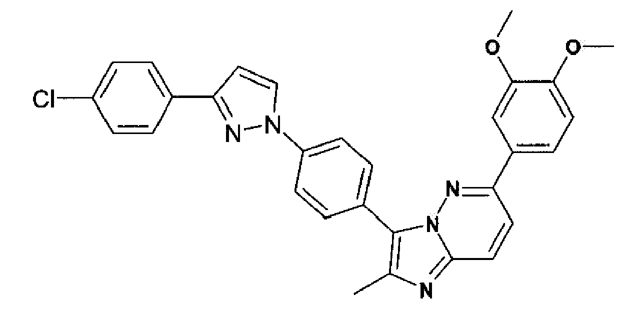

### Example 21:

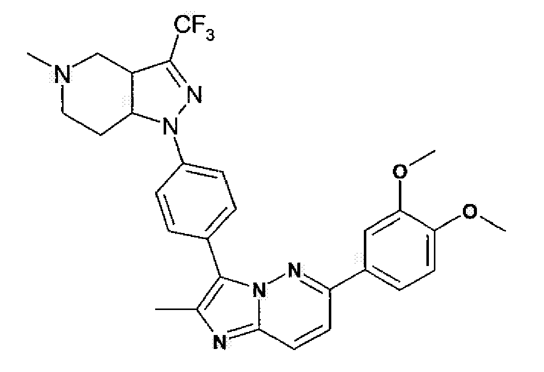

### Example 22:

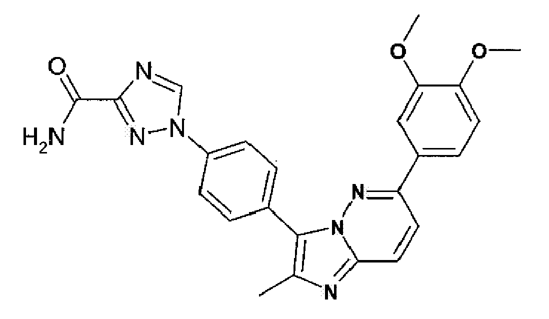

### Example 23:

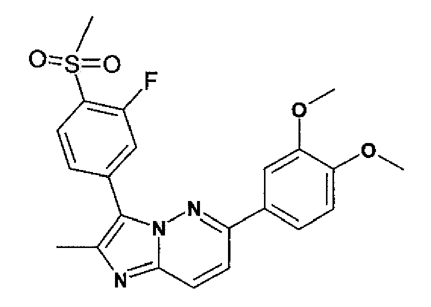

### Example 24: {4-[6-(3,4-Dimethoxy-phenyl)-2-methyl-imidazo[1,2-b]pyridazin-3-yl]-phenyl}-morpholin-4-yl-methanone.

In analogy to Example 27, the title compound is prepared from 80 mg (0.207 mmol) of 3-bromo-6-(3,4-dimethoxy-phenyl)-2-methyl-imidazo[1,2-b]pyridazine (for preparation see Example 29), 58.9 mg (0.248 mmol) of 4-(morpholino-4-carbonyl)phenylboronic acid (from Combi-Blocks), and 0.41 ml 1M aqueous K₂CO₃-solution, 11.9 mg (0.0103 mmol) of tetrakis (triphenylphospine) palladium in 2 ml DMA. The vial is heated in the microwave oven for 20 min at 150°C (no reaction occurs at 100°C). After purification, yellow crystals are obtained. MS.: 459 (M+1).- HPLC.: tR = 3.878 min.

### Example 25:

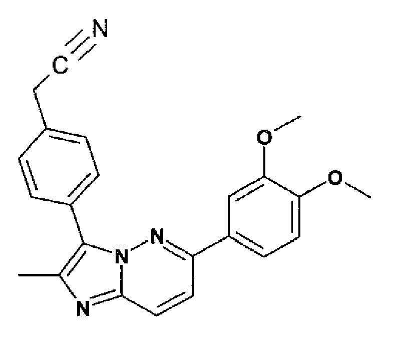

### Example 26: 4-[6-(3,4-Dimethoxy-phenyl)-2-methyl-imidazo[1,2-b]pyridazin-3-yl]-N,N-dimethyl-benzenesulfonamide.

In analogy to Example 27, the title compound is prepared from 80 mg (0.207 mmol) of 3-bromo-6-(3,4-dimethoxy-phenyl)-2-methyl-imidazo[1,2-b]pyridazine (for preparation see Example 29), 71.8 mg (0.31 mmol) of 4-(N,N-dimethylsulfonamidophenyl)boronic acid (from Combi-Blocks), 0.52 ml 1M aqueous K₂CO₃-solution, 11.9 mg (0.0103 mmol) of tetrakis (triphenylphospine) palladium in 2 ml DMA. The vial is heated in the microwave oven for 40 min at 100°C. After purification, yellow crystals are obtained. MS.: 453 (M+1).- HPLC.: t_{R} = 4.691 min.

### Example 27: 4-[6-(3,4-Dimethoxy-phenyl)-2-methyl-imidazo[1,2-b]pyridazin-3-yl]-benzamide.

In a 3 ml vial for microwave with crown cap and magnetic stir bar, a mixture of 80 mg (0.207 mmol) of 3-bromo-6-(3,4-dimethoxy-phenyl)-2-methyl-imidazo[1,2-b]pyridazine (for preparation see Example 29), 41.3 mg (0.248 mmol) of 4-(aminocarbonylphenyl)boronic acid (from Combi-Blooks), 0.52 ml 1M aqueous K₂CO₃-solution and 2 ml DMA (dimethylformamid) is degassed with argon for 5 min. After that time, 11.9 mg (0.0103 mmol) of tetrakis (triphenylphospine) palladium is added and the vial is heated in the microwave oven for 55 min at 150°C (no reaction occurs at 100°C). After that time, no more starting material can be dedected by HPLC. The reaction mixture is evaporated to dryness, the residue dissolved in EtOAc and water. The phases are separated, the water is extracted two more times with EtOAc. After drying with Na₂SO₄, the solvent is evaporated. The purification of the raw material is done by chromatography (CombiFlash; solvent system: from 100% CH₂Cl₂/MeOH 98:2 to 100% CH₂Cl₂/MeOH 95:5. The pure compound is suspended in EtOAc, hexane is added, and after standing for 1 hr at ca. 5°C, the suspension is filtered off, washed with hexane and dried to yield the title compound. MS.: 389 (M+1).- HPLC.: t_{R} = 3.461 min.

### Example 28:

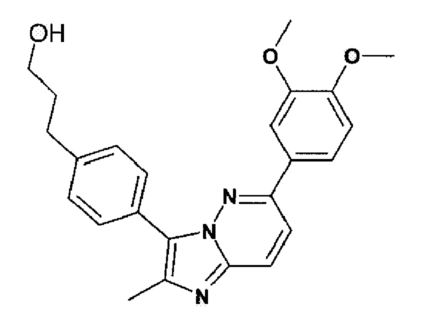

### Example 29:

The following starting material can be used for the synthesis of compounds of the formula I by replacement of the 3-bromo group with a boronic acid of the formula R¹-B(OH)₂:

### Stage 29.1: 3-Bromo-6-(3,4-dimethoxy-phenyl)-2-methyl-imidazo[1,2-b]pyridazine

To an ice-cold solution of 300 mg (1.06 mmol) of 6-(3,4-dimethoxy-phenyl)-2-methyl-imidazo[1,2-b]pyridazinen 3 ml of DMF, 178 mg (1.02 mmol) of N-bromosuccinimide is added. Stirring is continued for 2 h at 0-5°C and then 1 additional h at RT. After that time, the reaction mixture is evaporated to dryness, the residue dissolved in ethylacetate and the organic phase is washed with water (2x) and brine (1x). After drying over Na₂SO₄, the solvent is evaporated. The title compound crystallizes from an ethylacetate-hexane mixture. MS-ES.: 348/350. Rf (CH₂Cl₂-MeOH=95:5) = 0.56.

### Stage 29.2: 6-(3,4-Dimethoxy-phenyl)-2-methyl-imidazo[1,2-b]pyridazine

In a 6 ml vial for microwave with crown cap and magnetic stir bar, a mixture of 500 mg (2.05 mmol) 6-(3,4-dimethoxy-phenyl)-pyridazin-3-ylamine, 0.364 ml (4.11 mmol) of chloroacetone (Fluka), and 0.716 ml of Et₃N in 4 ml of ethanol are heated in a microwave (Emrys Optimizer) at 170°C for 30 min. The reaction mixture is evaporated to dryness and the residue is dissolved in CH₂Cl₂. The organic phase is washed with water (2x) and brine (1x). After drying over Na₂SO₄, the solvent is evaporated and the residue purified by chromatography on silica gel. Solvent system: CH₂Cl₂ (100%; start) to CH₂Cl₂-MeOH 98:2 (end). MS: (M+1) =270; HPLC: t_{R} = 3.37 min.

### Example 30: 3-(2-Chloro-4-methoxy-5-methyl-phenyl)-6-(3,4-dimethoxy-phenyl)-2-methyl-imidazo[1,2-b]pyridazine.

The title compound is prepared in analogy to the compound described in Example 8, starting from 300 mg (1.297 mmol) of 6-(3,4-dimethoxy-phenyl)-pyridazin-3-ylamine (see Stage 1.2), 1.6 g (crude product, ca. 1.95 mmol), 1-chloro-1-(2-chloro-4-methoxy-5-methyl-phenyl)-propan-2-one, and 0.602 ml (4.324 mmol) of NEt₃ in 3 ml dry of EtOH. The mixture is heated in the microwave oven at 170°C for 30 min. After work-up and purification, a yellow solid is obtained. MS.: 424 (M+1).- HPLC.: t_{R} = 5.477 min.

The starting material is obtained as follows:

### Stage 30.1 : 1-chloro-2-chloromethyl-5-methoxy-4-methyl-benzene

For the synthesis of the title compound and for the synthesis of the compounds of Stage 14.2. to Stage 30.4. see Carvalho et.al. in J. Chem. Soc. Perkin Trans. I (1984), 1913. The compound is prepared following the procedure given in above reference, starting from 50 g (319 mmol) of 5-chloro-2-methylanisole (Apollo Chemicals Co., Burlington, North Carolina, USA), 6.22 g (44.7 mmol) of ZnCl₂, 0.466 g of NaCl, 41.4 ml of formaldehyde (37% in water), and HCl gas. The product formed is used without purification in the next step. HPLC.: t_{R} = 5.252 min.

### Stage 30.2. Acetic acid 2-chloro-4-methoxy-5-methyl-benzyl ester

Starting from 62 g (ca. 287 mmol) of 1-chloro-2-chloromethyl-5-methoxy-4-methyl-benzene (see Stage 30.1), 250 g (3.02 Mol) of NaOAc in 300 ml of DMF, the title compound is obtainned as an oil, which is used without further purification in the next step. HPLC.: t_{R} =6.779 min.

### Stage 30.3: 2-Chloro-4-methoxy-5-methyl-phenyl)-methanol

Starting from 70 g (crude product; ca. 275 mmol) Acetic acid 2-chloro-4-methoxy-5-methylbenzyl ester (see Stage 30.2), 29 g (0.71 mmol) of NaOH pellets, 70 ml H₂O and 350 ml MeOH, the title compound is obtained after neutralization with 4N HCl. The product is obtained as a white powder after drying. HPLC.: t_{R} = 5.255 min.

### Stage 30.4: 2-Chloro-4-methoxy-5-methyl-benzaldehyde

Starting from 58.5 g (crude product, ca. 160 mmol) of (2-Chloro-4-methoxy-5-methylphenyl)-methanol (see Stage 24.3), 139 g (1.6 Mol) MnO₂ activated (Merck-Schuchard, Darmsatadt, Germany) in 1 I of toluene, the title compound is obtained. An analytical sample is purified by chromatography on silicagel (solvent system: hexane-EtOAc 100:1 to 50:50). MS.: 185 (M+1).- HPLC.: t_{R} = 6.496 min.

### Stage 30.5: 1-(2-Chloro-4-methoxy-5-methyl-phenyl)-propan-2-one

The title compound is prepared in a slightly different way as the compound prepared in Stage 8.2: 5 g (13.5 mmol) of 2-chloro-4-methoxy-5-methyl-benzaldehyde (see Stage 30.4.) and 316 mg (4.06 mmol) of NH₄OAc in 7.86 ml of nitroethane are heated overnight at 125°C. The reaction mixture is concentrated under reduced pressure. The residue is dissolved in CH₂Cl₂ and extracted with H₂O. The aqueous phase is washed with CH₂Cl₂. The combined organic phases are washed with brine, dried, and evaporated to dryness. A 5-necked flask is charged with 3.04 g (54.2 mmol) of iron powder and 8.5 ml of HOAc. The flask is fitted with a condenser and the mixture is stirred at 60°C to form a grey slurry. To the slurry a suspension of the above intermediate in glacial HOAc is added slowy, and then stirred overnight at 105°C. The reaction is allowed to cool down and is poured onto H₂O. The suspension is filtered over hyflo. The layers are separated and the organic phase washed with 1 N HCl, sat. NaHCO3, water, and brine. The solvent is evaporated, and the residue purified by flash chromatography (solvent system: hexane-EtOAc 100:0 to 50:50). The product is isolated as an oil. MS.: 213 (M+1).- HPLC.: t_{R} = 6.29 min.

### Stage 30.6: 1-(2-Chloro-4-methoxy-5-methyl-phenyl)-propan-2-one

The title compound is prepared in analogy to the compound prepared in Stage 8.3. starting from 1.3 g (3.97 mmol) of 1-(2-chloro-4-methoxy-5-methyl-phenyl)-propan-2-one (see Stage 30.5.) and 0.958 ml of SO₂Cl₂ in 10 ml CH₂Cl₂ at 0°C. The product is used without further purification in Example 30.

### Example 31: 6-(3,4-Dimethoxy-phenyl)-3-(4'-ethanesulfonyl-phehyl)-2-methyl-imidazo[1,2-b]pyridazine.

In analogy to Example 19, the title compound is prepared from 80 mg (0.207 mmol) of 3-Bromo-6-(3,4-dimethoxy-phenyl)-2-methyl-imidazo[1,2-b]pyridazine (for preparation see Example 14), 53.6 mg (0.248 mmol) of (4-Ethylsulfonylphenyl)boronic acid (from Combi-Blocks), 0.52 ml 1M aqueous K2CO3-solution, 11.9 mg (0.0103 mmol) of tetrakis (triphenylphosphine) palladium in 2 ml DMA. The vial is heated in the microwave oven for 40 min at 150°C (no reaction occurs at 100°C). After purification, yellow crystals are obtained. MS.: 438 (M+1).- HPLC.: t_{R} = 4.359 min.

### Example 32:

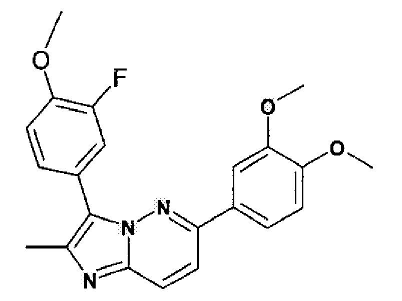

### Example 33:

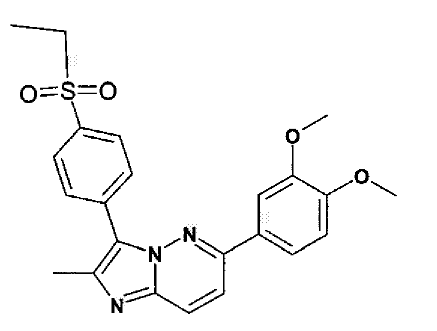

### Example 34:

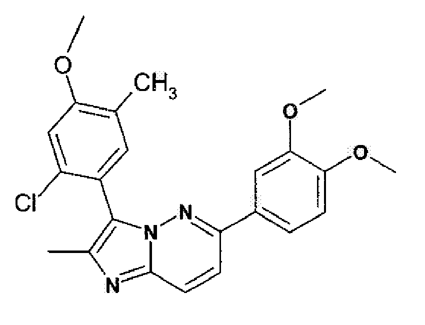

### Example 35: 3-[6-(3,4-Dimethoxy-phenyl)-2-methyl-imidazo[1,2-b]pyridazin-3-yl]-N,N-dimethyl-benzenesulfonamide

In a 3 ml vial for microwave with crown cap and magnetic stir bar, a mixture of 80 mg (0.207 mmol) of 3-Bromo-6-(3,4-dimethoxy-phenyl)-2-methyl-imidazo[1,2-b]pyridazine (preparation see Stage 29.1), 72.5 mg (0.31 mmol) of 3-(N,N-dimethyfsulfonamidophenyl)boronic acid (Combi-Blocks) and 0.52 ml of aqueous 1M K₂CO₃-solution in 2 ml of DMA are degassed with argon for 5 min. Then 11.9 mg of tetrakistriphenylphosphin palladium is added and the mixture is heated in a microwave oven at 150°C for 40 min. The reaction solution is poured into CH2Cl2 and the organic phase is washed with water and brine. The phases are separated, the organic phase is dried (Na2SO4) and evaporated to dryness. The residue is purified by chromatography on silicagel. Solvent system: A = EtOAc; B = EtOAc-MeOH = 98:2. Start with 100% A, then within 30 min to 100% B. The title compound is isolated as a bright yellow solid. MS.: 453.1 (M+1).- HPLC.: t_{R} = 4.635 min.

The compounds in the following Table are prepared in analogy to Example 35:

| **Example** | **Product** | **boronic acid** | **microwave conditions; data** |
|---|---|---|---|
| 36 | 4-[6-(3,4-Dimethoxy-phenyl)-2-methyl-imidazo[1,2-b]pyridazin-3-yl]-N-methyl-benzenesulfonamide | 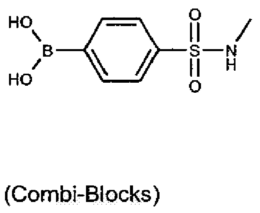 | 110°C 30 min. MS.:439(M+1).-HPLC.: t_{R} = 4.194 min. |
| 37 | 4-[6-(3,4-Dimethoxy-phenyl)-2-methyl-imidazo[1,2-b] pyridazin-3-yl]-benzene-sulfonamide | 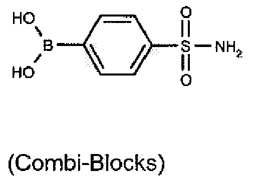 | 110°C 16h MS.: 425 (M+1).-HPLC.: t_{R} = 3.768 min. |
| 38 | {4-[6-(3,4-Dimethoxy-phenyl)-2-methyl-imidazo[1,2-b] pyridezin-3-yl]-phenyl}-acetonitrile | 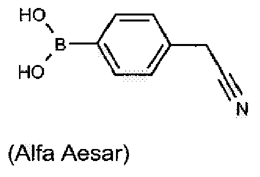 | 110°C 45 min. MS.: 385 (M+1).-HPLC.: t_{R} = 4.47 min. |
| 39 | 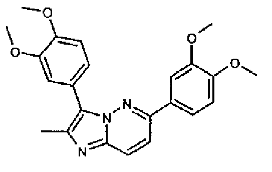 | 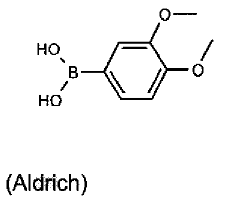 | 110°C 30 min. MS.: 406 (M+1).-HPLC.: t_{R} = 4.508 min. |
| 40 | 3-{4-[6-(3,4-Dimethoxy-phenyl)-2-methyl-imidazo-[1,2-b]pyridazin-3-yl]-phenyl}-propan-1-ol | 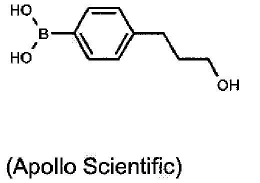 | 110°C 30 min MS.: 404 (M+1).-HPLC.: t_{R} = 4.206 min. |
| 41 | 4-[6-(3,4-Dimethoxy-phenyl)-2-methyl-imidazo[1,2-b]pyridazin-3-yl]-N-ethyl-benzenesulfonamide | 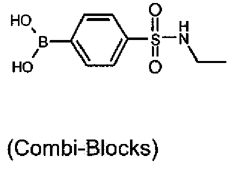 | 110°C 1h LC-MS: 453 (M+1).-HPLC.: t_{R} = 4.463 min. |
| 42 | 6-(3,4-Dimethoxy-phenyl)-2-methyl-3-[4-(propane-2-sulfonyl)-phenyl]-imidazo[1,2-b]pyridazine | 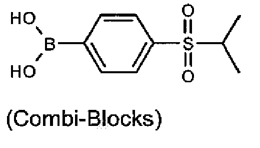 | 110°C 30 min. LC-MS: 452 (M+1); t_{R} = 1.49 min.- |
| 43 | N-Cyclopropyl-4-[6-(3,4-dimethoxy-phenyl)-2-methyl-imidazo[1,2-b]pyridazin-3-yl]-benzenesulfonamide | 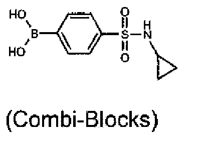 | 110°C 1 ½ h MS.: 465 (M+1).-HPLC.: t_{R} = 4.578 min. |
| 44 | 4-[6-(3,4-Dimethoxy-phenyl)-2-methyl-imidazo[1,2-b]pyridazin-3-yl]-N,N-diethyl-benzenesulfonamide | 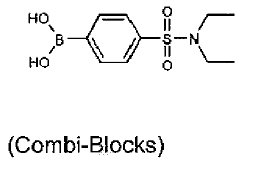 | 110-120°C total of 6 ½ h ; reaction not complete MS.: 481 (M+1).-HPLC.: t_{R}=5.214 min. |
| 45 | 3-[6-(3,4-Dimethoxy-phenyl)-2-methyl-imidazo[1,2-b]-pyridazi n-3-yl]-N-methyl-benzamide | 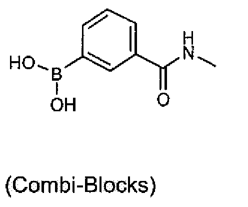 | 110°-120°C 6 ½ h; reaction not complete LC-MS: 403 (M+1).-HPLC.: t_{R} = 3.468 min. |
| 46 | 3-[4-(Azetidine-1-sulfonyl)-phenyl]-6-(3,4-dimethoxy-phenyl)-2-methyl-imidazo[1,2-b]pyridazine | 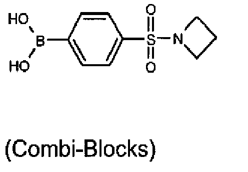 | 110°C 2h MS.: 465 (M+1).-HPLC.: t_{R} = 4.635 min. |
| 47 | 6-(3,4-Dimethoxy-phenyl)-3-(3-methanesulfonyl-phenyl)-2-methyl-imidazo[1,2-b]-pyridazine | 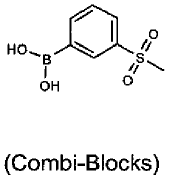 | 110°C 7 ½ h LC-MS: 424 (M+1).-HPLC.: t_{R} = 4.127 min. |
| 48 | 4-[6-(3,4-Dimethoxy-phenyl)-2-methyl-imidazo[1,2-b]-pyridazin-8-yl]-N-(2-hydroxy-ethyl)-benzenesulfonamide- | 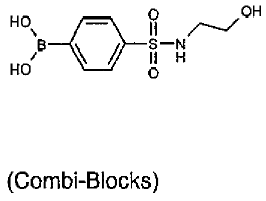 | 140°C 5h MS.: 469 (M+1).-HPLC.: t_{R} = 3.716 min. |
| 49 | 3-[6-(3,4-Dimethoxy-phenyl)-2-methyl-imidazo[1,2-b]pyridazin-3-yl]-benzamide | 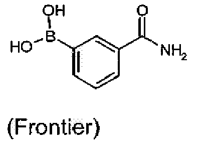 | 140°C 10h ; ionic liquid is added LC-MS: 389 (M+1).-HPLC.: t_{R} = 3.537 min. |

### Example 50: 5-{4-[6-(3,4-Dimethoxy-phenyl)-2-methyl-imidazo[1,2-b]pyridazin-3-yl]-phenyl}-nicotinonitrile

In a 6ml vial for microwave with crown cap and magnetic stir bar, 142 mg (0.301 mmol) of 6-(3,4-Dimethoxy-phenyl)-2-methyl-3-[4-(4,4,5,5-tetramethyl-[1,3,2]dioxaborolan-2-yl)-phenyl]-imidazo[1,2-b]pyridazine, 50 mg of 5-bromo-3-cyanopyridine (Aldrich), 151 mg of K₂CO₃ and 12 mg of PdCl₂(dppf) in 3 ml of toluene and 2 ml of dry EtOH are heated in a microwave oven at 110°C for 1 h. The reaction mixture is poured into CH₂Cl₂, washed with water and brine and dried (NA₂SO₄). After the solvent is evaporated, the residue is purified by chromatography on silicagel. Solvent system: starting with CH₂Cl₂-EtOAc-MeOH = 100-0-0, ending with 80-1.6-0.4. The title compound is isolated as a yellow solid. LC-MS: 448 (M+1).-HPLC.: t_{R} = 4.867 min.

### Stage 50.1: 6-(3,4-Dimethoxy-phenyl)-2-methyl-3-[4-(4,4,5,5-tetramethyl-[1,3,2]dioxaborolan-2-yl)-phenyl]-imidazo[1 ,2-b]pyidazine.

A procedure to prepare the boronic ester can be found in the literature, e.g. WO2005123687. In a 20 ml vial for microwave with crown cap and magnetic stir bar, a solution of 3.5 g (8.249 mmol) of 3-(4-Bromo-phenyl)-6-(3,4-dimethoxy-phenyl)-2-methyl-imidazo[1,2-b]pyridazine (preparation see Stage 1.1.), 3.8 ml of triethyamin puriss (Fluka), 2.8 g of bis(pinacolato)diboron (Aldrich) in 20 ml of dixane are degassed with argon. 371 mg of PdCl2(PPh2)Fe.CH2Cl2 and a few drops of ionic liquid (1-butyl-3-methylimidazolium tetrafluoroborat; Fluka) are added and the mixture is stirred in the microwave oven at 150°C for 4 h (at 130°C the reaction is very slow). The reaction mixture is poured into CH2Cl2, washed with water and dried (Na2SO4). The solvent is evaporated, the residue purified by chromatography on silicagel. Solvent system: CH2Cl2-EtOAc = 100-0 (start) to 70-30 (end). The title compound is isolated as a yellow solid. MS: 472 (M+1).- HPLC.: t_{R} = 6.014 min.

The compounds in the table below are prepared in analogy to Example 50:

| **Example** | **Product** | **Reagent** | **microwave conditions; data** |
|---|---|---|---|
| 51 | 6-(3,4-Dimethoxy-phenyl)-2-methyl-3-(4-pyrazin-2-yl-phenyl)-imidazo[1,2-b]pyridazine | 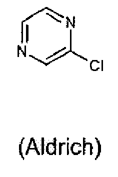 | 110°C 1 h LC-MS: 424 (M+1).-HPLC.: t_{R} = 4.537 min. |
| 52 | {4'-[6-(3,4-Dimethoxy-phenyl)-2-methyl-imidazo[1,2-b]pyridazin-3-yl]-biphenyl-4-yl}-acetonitrile | 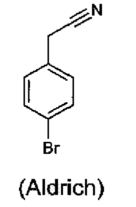 | 110°C 1 h MS: 461 (M+1).-HPLC.: t_{R} = 5.346 min. |
| 53 | 5-{4-[6-(3,4-Dimethoxy-phenyl)-2-methyl-imidazo[1,2-b]pyridazin-3-yl]-phenyl}-pyridine-2-carbonitrile | 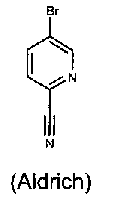 | 110°C 30 min MS: 448 (M+1).-HPLC.: t_{R} = 5.060 min. |
| 54 | 6-(3,4-Dimethoxy-phenyl)-2-methyl-3-[4-(3-methyl-pyridin-2-yl)-phenyl] -imidazo[1,2-b]pyridazine | 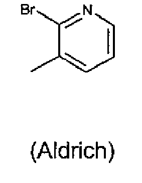 | 110°C 1 h MS: 437 (M+1).-HPLC.: t_{R} = 3.474min. |

### Example 55: 3-(4-Benzothiazol-2-yl-phenyl)-6-(3,4-dimethoxy-phenyl)-2-methyl-imida zo[1,2-b]pyridazine

For reference see J. Org. Chem. (2005), 70, 5164 for similar reaction.
A mixture of 100 mg (0.212 mmol) of 6-(3,4-Dimethoxy-phenyl)-3-(4-iodo-phenyl)-2-methyl-imidazo[1,2-b]pyriDazine (preparation see Example 10), 0.04 ml (1.74 mmol) of benzothiazole (Aldrich), 6 mg of Cul (Fluka), 64 mg of K2CO3, 6 mg of L-proline (Fluka) in 5 ml of DMSO are heated in an oil bath for 18 h. The reaction mixture is poured into CH2Cl2, washed with water and dried (Na2SO4). The solvent is evaporated, the residue purified by double chromatography on silicagel and C18-silicagel to yield the title compound as a yellow solid. MS: 479 (M+1); HPLC.: t_{R} = 6.154min

The compounds in the following Table are prepared in analogy to Example 55:

| **Example** | **Product** | **Reagent** | **reaction conditions; data** |
|---|---|---|---|
| 56 | 1-{4-[6-(3,4-Dimethoxy-phenyl)-2-methyl-imidazo[1,2-b]pyridazin-3-yl]-phenyl}-azetidin-2-one | 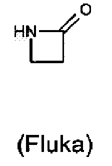 | 100°C 48 h MS: 415 (M+1); HPLC.: t_{R} = 4.369min |
| 57 | (R)-Pyrrolidine-2-carboxylic acid {4-[6-(3,4-dimethoxy-phenyl)-2-methyl-imidazo[1,2-b]pyridazin-3-yl]-phenyl}-amide | 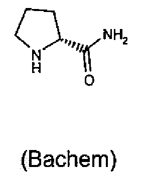 | 100°C 6h LC-MS: 459 (M+1); HPLC.: t_{R} = 3.3min |
| 58 | 1-{4-[6-(3,4-Dimethoxy-phenyl)-2-methyl-imidazo[1,2-b]pyridazin-3-yl]-phenyl}-1H-[1,2,4]triazole-3-carboxylic acid amide | 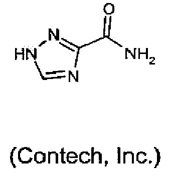 | 100°C 26 h LC-MS: t_{R} = 1.27min ; 456 (M+1).- |
| 59 | 2-{4-[6-(3,4-Dimethoxy-phenyl)-2-methyl-imidaza[1,2-b]pyridazin-3-yl]-phenyl}-benzothiazol-5-ylamine | 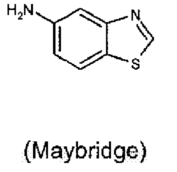 | 100°C 18 h MS: 494 (M+1); HPLC.: t_{R} = 3.939min |

### Example 60 : 5-{4-[6-(3,4-Dimethoxy-phenyl)-2-methyl-imidazo[1,2-b]pyridazin-3-yl]-phenyl}-pyridin-2-ylamine

In a 6ml vial for microwave with crown cap and magnetic stir bar, 100 mg (0.236 mmol) of 3-(4-Bromo-phenyl)-6-(3,4-dimethoxy-phenyl)-2-methyl-imidazo[1,2-b]pyridazine (preparation see Stage 1.1.), 70 mg (0.305 mmol) of 2-aminopyridine-5-boronic acid pinacolester (Aldrich), 130 mg K2CO3 and 10 mg of PdCl2(dppf) in 3 ml of toluene and 2 ml dry EtOH are heated ina microwave oven at 10°C for 8 h. The reaction mixture is poured into CH2Cl2, washed with water and dried (Na2SO4). After the solvent is evaporated, the residue is purified by chromatography on silicagel. Solvent system: CH2Cl2-EtOAc-MeOH = 100-0-0 (start) to 50-40-10 (end).The title compound is isolated as yellow solid. MS: 438 (M+1); HPLC.: t_{R} = 3.498min.

### Example 61: 3,6-Bis-(4-methanesulfonyl-phenyl)-2-methyl-imidazo[1,2-b]pyridazine

A mixture of 50 mg (0.203 mmol) of 3-Bromo-6-chloro-2-methyl-imidazo[1,2-b]pyridazine (BKS422; preparation see Stage 61.1), 92 mg (0.446 mmol) of (4-methylsulfonyl)-phenylboronic acid (Combi-Blocks), 0.51 ml of a aqueous 1M K2CO3-solution, 8 mg of Pd(PPh3)2Cl2 in 1.5 ml of DMF are heated in an oil bath at 105°C for 5 h. The reaction mixture is ppured into CH2Cl2, washed with water and dried (Na2SO4). After the solvent is evaporated, the residue is purified by chromatography on silicagel. Solvent system: CH2Cl2-EtOAc = 100-0 (start) to 0/100 (end). The title compound is isolated as a yellow solid. MS: 442 (M+1); HPLC.: t_{R} = 3.710min.

### Stage 61.1: 3-Bromo-6-chloro-2-methyl-imidazo[1,2-b]pyridazine

6-Chloro-2-methyl-imidaza[1,2-b]pyridazine (NVP-BKS419; preparation see Stage 13.6. Beispiele P2 Anmeldung) (2.4 g; 14.3 mmol) is dissolved in DMF (25 mL) and cooled to 0°C. At this temperature, N-bromo-succinimide (2.82 g; 15 mmol) is added and the yellow solution is stirred at 0°C for 2 h, followed by stirring at RT for 1 h. The yellow solution is taken up into EtOAc (200 mL) and washed with water (2x 100 mL), followed by back extraction of the aqueous layers with EtOAc (1x 200 mL). The combined organic layers are dried (Na₂SO₄), concentrated under reduced pressure, followed by chromatography (120g Redisep, ISCO Sg-100; eluting with TBME), to obtain the title compound as beige crystals; mp. 146-148°C; MS: 247.9 (M+1); HPLC.: t_{R} = 5.81 min. The structure is confirmed by x-ray analysis.

### Example 62: 3,6-Bis-(4'-methoxy-biphenyl-4-yl)-2-methyl-imidazo[1,2-b]pyridazine

The title compound is prepared in analogy to the compound prepared in Example 61, starting from 100 mg (0.406 mmol) of 3-Bromo-6-chloro-2-methyl-imidazo[1,2-b]pyridazine (Stage 61.1) and 400 mg (1.754 mmol) of 4'-methoxybephenyl-4-ylboronic acid (Combi-Blocks). Yellow solid.
MS: 498 (M+1); HPLC.: t_{R} = 6.989min.

### Example 63: 6-(4-Ethoxy-3-methoxy-phenyl)-3-(4-methanesulfonyl-phenyl)-2-methyl-imidazo[1,2-b]pyridazine

50 mg (0.138 mmol) of 3-Bromo-6-(4-ethoxy-3-methoxy-phenyl)-2-methyl-imidezo[1,2-b]pyridazine (preparation see Stage 13.1. in Beispiele Anmeldung P2), 40 mg (0.194 mmol) of (4-methylsulfonyl)phenylboronic acid (Combi-Blocks), 0.35 ml of aqueous 1 M K2CO3-solution and 6 mg of Pd(PPh3)2Cl2 in 1.5 ml DMF are heated in an oil bath at 105°C for 4 ½ h. The reaction mixture is poured into CH2Cl2, the organic phase washed with water and dried (Na2SO4). The organic solution is concentrated and the residue purified by chromatography on silicagel. Solvent system: CH2Cl2-EtOAc = 100-0 (start) to 30-70 (end). The title compound is isolated as a colorless solid. LC-MS: 438 (M+1); HPLC.: t_{R} = 4.499min.

### Example 64: 4-[6-(4-Ethoxy-3-methoxy-phenyl)-2-methyl-imidazo[1,2-b]pyridazin-3-yl]-N-methyl-benzenesulfonamide

The title compound is prepared in analogy to the compound prepared in Example 63, except that a microwave oven is used as a heat source. Colorless solid. LC-MS: 453 (M+1); HPLC.: t_{R} = 4.531 min.

### Example 65: 3-(4-Methanesulfonyl-phenyl)-6-(4'-methoxy-biphenyl-4-yl)-2-methyl-imidazo[1,2-b]pyridazine

50 mg (0.127 mmol) of 3-Bromo-6-(4'-methoxy-biphenyl-4-yl)-2-methyl-imidazo[1,2-b]pyridazine (preparation see Stage 65.1),36 mg (0.18 mmol) of (4-methylsulfonyl)phenylboronic acid (Combi-Blocks), 0.32 ml of aqueous 1 M K2CO3-solution and 5 mg of Pd(PPh3)2CI2 in 1.5 ml DMF are heated in an oil bath at 105°C for 2 ½ h. The solvent is evaporated, the residue dissolved in CH2Cl2 and the organic phase washed with water and dried (Na2SO4). The solvent is evaporated and the residue purified by chromatography on silicagel. Solvent system: CH2Cl2-EtOAc = 100-0 (start) to 0-100 (end). The title compound is isolated as a yellow solid. MS: 470 (M+1); HPLC.: t_{R} = 5.437min.

### Stage 65.1: 3-Bromo-6-(4'-methoxy-biphenyl-4-yl)-2-methyl-imidazo[1,2-b]pyridazine

490 mg (1.554 mmol) of 6-(4'-Methoxy-biphenyl-4-yl)-2-methyl-imidazo[1,2-b]pyridazine (preparation see Stage 65.2) are dissolved in 50 ml of dry DMF. The solution is cooled to 0-5°C and 297 mg (1.58 mmol) of N-bromosuccinimid are added. Stirring is continued for 2 h at 0-5°C, then additional 2 h at RT. The reaction mixture is concentrated under reduced pressure, CH2Cl2 is added and the organic phase is extracted with water and brine. After separation and drying (Na2SO4) of the organic phase, the solvent is evaporated and the residue purified by chromatography. Solvent system: CH2Cl2-EtOAc = 100-0 (start) to 70-30 (end). The title compound is isolated as a yellow solid. MS: 396 (M+1); HPLC.: t_{R} = 6.988min.

### Stage 65.2. 6-(4'-Methoxy-biphenyl-4-yl)-2-methyl-imidazo[1,2-b]pyridazine

In a 20 ml vial for microwave with crown cap and magnetic stir bar, 300 mg (1.79 mmol) of 6-Chloro-2-methyl-imidazo[1,2-b]pyridazine (preparation see Stage 13.6. in Beispiele P2-Anmeldung), 500 mg (2.192 mmol) of 4'-methoxybiphenyl-4-yl-boronic acid (Combi-Blocks), 41 mg Pd (dba)2, 1.15 g (5.418 mmol) of K3PO4 and 59 mg SPhos in 15 ml of dry THF are heated in a microwave oven at 110°C for 30 min. The reaction mixture is poured into CH2Cl2 and washed with water. The organic phase is dried (Na2SO4) and evaporated to dryness. The residue is purified by chromatography. Solvent system: CH2Cl2-EtOAc = 100-0 (start) to 0-100 (end). The title compound is isolated as a yellow solid. MS: 316 (M+1); HPLC.:t_{R}= 5.124min.

### Example 66: 4-(6-(4'-methoxybiphenyl-4-yl)-2-methylimidazo[1,2-b]pyridazin-3-yl)-N-methylbenzenesulfonamide

The title compound is prepared in analogy to the compound prepared in Example 65, starting from 50 mg (0.127 mmol) of 3-Bromo-6-(4'-methoxy-biphenyl-4-yl)-2-methyl-imidazo[1,2-b]pyridazine (Stage 65.1) and 38 mg (0.177 mmol) of methyl-4-boronobenzenesulfonamide (Combi-Blocks). The product is isolated as a yellow solid. MS: 485 (M+1); HPLC.: t_{R} = 5.437 min.

### Example 67: 4-[6-(4-Methanesulfonyl-phenyl)-2-methyl-imidazo[1,2-b]pyridazin-3-yl]-N-methyl-benzenesulfonamide

The title compound is prepared in analogy to the compounds prepared in Example 65, and in analogy to the intermediates prepared in Stage 65.1. and in Stage 65.2. starting from the respective boronic acids. Yellow solid. MS: 457 (M+1); HPLC.: t_{R} = 3.796 min.

### Example 68: 3-{4-[3-(4-Methanesulfonyl-phenyl)-2-methyl-imidazo[1,2-b]pyridazin-6-yl]-2-methoxy-phenoxy}-propylamine

115 mg (0.193 mmol) of (3-{4-[3-(4-Methanesulfonyl-phenyl)-2-methyl-imidazo[1,2-b]pyridazin-6-yl]-2-methoxy-phenoxy}-propyl)-carbamic acid tert-butyl ester (Stage 68.1) are dissolved in 2 ml of CH2Cl2. At RT and under argon, 0.5 ml of TFA-water 9:1 are added. The reaction mixture is stirred for 90 min (control by HPLC and MS). The solution is diluted with CH2Cl2 and the organic phase is washed with sat. NaHCO3-solution, water and brine. After drying the organic phase with Na2SO4, the solvent is evaporated. The residue is purified by chromatography on silicagel. Solvent system: CH2Cl2-MeOH-NH4OH 32% = 90:10:1. MS: 467.1 (M+1); HPLC.: t_{R} = 2.989min.

### Stage 68.1. (3-{4-[3-(4-Methanesulfonyl-phenyl)-2-methyl-imidazo[1,2-b]pyridazin-6-yl]-2-methoxy-phenoxy}-propyl)-carbamic acid tert-butyl ester

In a 6 ml vial for microwave with crown cap and magnetic stir bar, a mixture of 150 mg (0.29 mmol) of {3-[4-(3-Bromo-2-methyl-imidazo[1,2-b]pyridazin-6-yl)-2-methoxy-phenoxy]-propyl}-carbamic acid tert-butyl ester (preparation see Stage 25.2. in Beispiele Anmeldung P2), 71 mg (0.348 mmol) of (4-methylsulfonylphenyl)boronic acid (Combi-Blocks), 0.72 ml of 1 M aqueous K2CO3-solution and 16.8 mg of tetrakis-triphenylphosphin-palladium in 4 ml of DMA are heated in a microwave oven at 150°C for 40 min. The reaction is evaporated under vacuum, the residue is dissolved in CH2Cl2 and extracted with water and brine. The organic phase is dried (Na2SO4) and the solvent is evaporated. The residue is purified by chromatography on silicagel. Solvent system: EtOAc = A; EtOAc-MeOH : 98-2 = B; run: 20 min with 100% A, then in 10 min to 100% B, then 20 min with 100% B. The title compound is isolated as a colorless solid. MS: 567.1 (M+1); HPLC.: t_{R} = 5.077min.

The compounds in the following Table are prepared in analogy to the compounds prepared in Example 68 and in Stage 68.1:

| **Example** | **Product** | **Reagent** | **reaction conditions; data** |
|---|---|---|---|
| 69 | (3-{4-[3-(4-Dimethylsulfamoyl-phenyl)-2-methyl-imidazo[1,2-b]pyridazin-6-yl]-2-methoxy-phenoxy}-propyl)-carbamic acid tert-butyl ester | 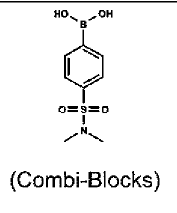 | 150°C 40 min MS: 596.1 (M+1); HPLC.: t_{R} = 5.477min. |
| 70 | 4-{6-[4-(3-Amino-propoxy)-3-methoxy-phenyl]-2-methyl-imidazo[1,2-b]pyridazin-3-yl}-N,N-dimethyl-benzenesulfonamide | TFA | 5 h at RT MS: 496.1 (M+1); HPLC.: t_{R} = 3.483min. |
| 71 | 3-{4-[3-(4-Ethanesulfonyl-phenyl)-2-methyl-imidazo[1,2-b]pyridazin-6-yl]-2-methoxy-phenoxy}-propylamine | TFA | 4 ½ h at RT MS: 481.1 (M+1); HPLC.: t_{R} = 3.214min. |
| 72 | (3-{4-[3-(4-Ethanesulfonyl-phenyl)-2-methyl-imidazo[1,2-b]pyridazin-6-yl]-2-methoxy-phenoxy}-propyl)-carbamic acid tert-butyl ester | 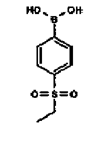 | 150°C 30 min; microwave MS: 581.1 (M+1); HPLC.: t_{R} = 5.248min. |

### Example 73: 2-{4-[3-(4-Ethanesulfonyl-phenyl)-2-methyl-imidazo[1,2-b]pyridazin-6-yl]-2-methoxy-phenoxy}-ethylamine

The title compound is prepared in analogy to the compound prepared in Example 68, starting from 112 mg (0.178 mmol) of (2-{4-[3-(4-Ethanesulfonyl-phenyl)-2-methyl-imidazo[1,2-]pyridazin-6-yl]-2-methoxy-phenoxy}-ethyl)-carbamic acid tert-butyl ester (Stage 73.1) and 0.25 ml of a mixture of TFA-water 9:1. Reaction time: 5 h at RT. Yellow solid. MS:467.1 (M+1); HPLC.; t_{R} = 3.092min.

### Stage 73.1: (2-{4-[3-(4-Ethanesulfonyl-phenyl)-2-methyl-imidazo[1,2-b]pyridazin-6-yl]-2-methoxy-phenoxy}-ethyl)-carbamic acid tert-butyl ester

The title compound is prepared in analogy to the compound prepared in Stage 68.1. starting from 156 mg (0.31 mmol) of {2-[4-(3-Bromo-2-methyl-imidazo[1,2-b]pyridazin-6-yl)-2-methoxy-phenoxy]-ethyl}-carbamic acid tert-butyl ester (preparation see Stage 24.2. in Beispiele Anmeldung P2) and 102 mg (0.466 mmol) of (4-ethylsulfonylphenyl)boronic acid (Combi-Blocks). Reaction time: 40 min at 150°C in the microwave oven. Yelloy crystals. MS: 567.1 (M+1); NPLC.: t_{R} = 5.062min.

The compounds in the following Table are prepared in analogy to the compounds prepared in Example 73 and in Stage 73.1:

| **Example** | **Product** | **Reagent** | **reaction conditions; data** |
|---|---|---|---|
| 74 | 2-{4-[3-(4-Methanesulfonyl-phenyl)- 2-methyl-imidazo[1,2-b]pyndazin-6-yl]-2-methoxy-phenoxy}-ethylamine | TFA | 6.5 h at RT MS:453.1 (M+1); HPLC.: t_{R} = 2.829min. |
| 75 | (2-{4-[3-(4-Methanesulfonyl-phenyl)-2-methyl-imidazo[1,2-b]pyridazin-6-yl]-2-methoxy-phenoxy}-ethyl)-carbamic acid tert-butyl ester | 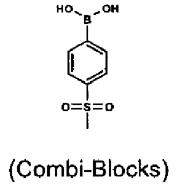 | 150°C 40 min; microwave MS: 553.1 (M+1); HPLC.: t_{R} = 4.875min. |
| 76 | 4-{6-[4-(2-Amino-ethoxy)-3-methoxy-phenyl]-2-methyl-imidazo[1,2-b]pyridazin-3-yl}-N,N-dimethyl-benzenesulfonamide | TFA | 5 h at RT MS: (482.1) (M+1); HPLC.: t_{R} = (3.369)min. |
| 77 | (2-{4-[3-(4-Dimethylsulfamoyl-phe-nyl)-2-methyl-imidazo[1,2-b]pyrida-zin-6-yl]-2-methoxy-phenoxy}-ethyl)-carbamic acid tert-butyl ester | 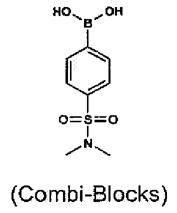 | 150°C 40 min; microwave MS: 582 (M+1); HPLC.: t_{R} = 5.288min. |

### Example 78: Soft capsules

5000 soft gelatin capsules, each comprising as active ingredient 0.05 g of one of the compounds of formula I mentioned in the preceding Examples, are prepared as follows:

| Composition | |
|---|---|
| Active ingredient | 250 g |
| Lauroglycol | 2 litres |

Preparation process: The pulverized active ingredient is suspended in Lauroglykol® (propylene glycol laurate, Gattefossä S.A., Saint Priest, France) and ground in a wet pulverizer to produce a particle size of about 1 to 3 µm. 0.419 g portions of the mixture are then introduced into soft gelatin capsules using a capsule-filling machine.

## Claims

1. A compound of the formula I, 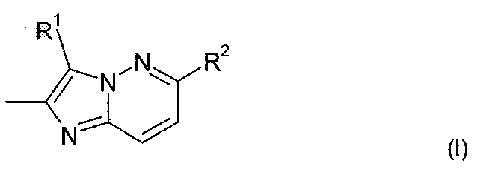 wherein
R¹ is unsubstituted or substituted aryl; and
R² is substituted phenyl or substituted naphthyl;
or an N-oxide thereof, a solvate and/or a (preferably pharmaceutically acceptable) salt thereof.

2. A compound of the formula I according to claim 1, wherein
R¹ is phenyl that is unsubstituted or substituted by by one or more, especially one or two or three, more preferably by two, moieties selected from the group consisting of unsubstituted or substituted alkyl, such as C₁-C₇-alkyl that is unsubstituted or substituted by hydroxyl or cyano-C₁-C₇-alkyl, halo, hydroxyl, alkyloxy, especially C₁-C₇-alkoxy, especially methoxy, amino, mono- or disubstituted amino, preferably N-mono- or N,N-di-(C₁-C₇-alkyl and/or C₃-C₈-cyloalkyl)-amino, especially N-methylamino, C₁-C₇-alkanoylamino, C₁-C₇-alkoxycarbonylamino, phenyl- or naphthyl-C₁-C₇-alkoxycarbonyl-amino, carbamoyl, mono- or disubstituted carbamoyl, preferably N-mono- or N,N-di-(C₁-C₇-alkyl and/or C₃-C₈-cydoalkyl)-carbamoyl, pyrrolidine-2-carbonyl-amino, heterocyclylcarbonyl where heterocyclyl is bound via a ring nitrogen to the carbonyl, especially piperidinocarbonyl, morpholino-carbonyl; thiomorpholinocarbonyl or S-oxo- or S,S-dioxothiomorpholinocarbonyl, C₁-C₇-alkanesulfonyl, sulfamoyl, N-mono- or N,N-disubstituted sulfamoyl, preferably N-mono- or N,N-di-(C₁-C₇-alkyl)-sulfamoyl, C₃-C₈-cycloalkyl-sulfamoyl, azetidine-sulfamoyl, hydroxy-(C₁-C₇-alkyl)-sulfamoyl, cyano, nitro, unsubstituted or substituted heterocyclyl bound via a ring carbon atom or preferably a ring nitrogen atom, especially 1,2,4-triazol-1-yl, carbamoyl-1,2,4-triazol-1-yl, pyrazol-1-yl, 3-trifluoromethyl-pyrazol-1-yl, 3-(halophenyl)-pyrazol-1-yl, e.g. 3-(4-chlorophenyl)-pyrazol-1-yl, pyrrolidin-1-yl, 2-oxo-pyrrolidin-1-yl, piperidin-1-yl, 2-oxopiperidin-1-yl, morpholino, pyridinyl which is unsubstituted or substituted by cyano, C₁-C₇-alkyl or amino, pyrimidinyl, pyrazinyl, benzimidazolyl, C₁-C₇-alkoxy-substituted benzimidazolyl, benzothiazolyl, amino-substituted benzothiazolyl, pyrrolo-pyrimidinyl, especially pyrrolo[2,3-d]pyrimidinyl, C₁-C₇-alkylsubstituted pyrrolo-pyrimidinyl, e.g. 2-C₁-C₇-alkyl-pyrrolo[2,3-d]pyrimidin-yl, and 1H,4H,5H-trihydropyrazolo[2,3-c]piperidin-1-yl which is unsubstituted or substituted by 1 or 2 substituents independently selected from C₁-C₇-alkyl and halo-C₁-C₇-alkyl, and/or further from unsubstituted or substituted aryl, from unsubstituted or substituted cycloalkyl and from unsubstituted or substituted heterocyclyl, especially from phenyl that is unsubstituted or substituted by one or more, e.g. up to 2, moieties independently selected from the group consisting of halo, C₁-C₇-alkoxy and C₁-C₇-alkanesulfonyl, from tetrazol-5-yl, from indolyl, from indazolyl, from C₁-C₇-alkyl-indazoylyl from pyrrolo-pyridinyl and from azetidin-2-one; and
R² is phenyl that is substituted by 1 to 3, preferably 1 or 2 (especially in meta- and/or para-position) substituents selected from the group consisting of halo, especially fluoro, C₁-C₇-alkoxy (very preferred), especially methoxy, hydroxyl, C₁-C₇-alkoxyphenyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, C₁-C₇-alkoxycarbonyl, amino-C₁-C₇-alkoxy, pyrrolidinyl-C₁-C₇-alkoxy, piperidinyl-C₁-C₇-alkoxy, morpholinyl-C₁-C₇-alkoxy, thlomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl and C₃-C₈-cyloalkyl-sulfonyl;
preferably with the proviso that phenyl R² is substituted in meta position by C₁-C₇-alkoxy, especially methoxy, and in para-position by C₁-C₇-alkoxy, especially methoxy, hydroxyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, pyrrolidinyl-C₁-C₇-alkoxy, piperidinyl-C₁-C₇-alkoxy, morpholinyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl or C₃-C₈-cyloalkyl-sulfonyl; where in one more preferred embodiment R² is 3,4-dimethoxyphenyl;
or an N-oxide thereof, a solvate and/or a (preferably pharmaceutically acceptable) salt thereof.

3. A compound of the formula according to claim 1, wherein
R¹ is phenyl that is unsubstituted or substituted (preferably in the 3- (meta) and/or 4- (para) position of the phenyl) by one or more, especially one or two or further three, more preferably by one or two, moieties selected from the group consisting of hydroxyl-C₁-C₇-alkyl, cyano-C₁-C₇-alkyl, halo, C₁-C₇-alkoxy, amino, N-mono- or N,N-di-(C₁-C₇-alkyl and/or C₃-C₈-cyloalkyl)-amino, C₁-C₇-alkanoylamino, C₁-C₇-alkoxycarbonyl-amino, phenyl- or naphthyl-C₁-C₇-alkoxycarbonyl-amino, carbamoyl, N-mono- or N,N-di-(C₁-C₇-alkyl and/or C₃-C₈-cycloalkyl)-carbamoyl, pyrrolidine-2-carbonyl-amino, piperidinocarbonyl; morpholirio-carbonyl, thiomorpholino-carbonyl, S-oxo- or S,S-dioxothiomorpholinocarbonyl, C₁-C₇-alkanesulfonyl, sulfamoyl, N-mono- or N,N-di-(C₁-C₇-alkyl)-sulfamoyl, C₃-C₈-cycloalkyl-sulfamoyl, azetidine-sulfamoyl, hydroxy-(C₁-C₇-alkyl)-sulfamoyl, cyano, nitro, 1,2,4-triazol-1-yl, carbamoyl-1,2,4-triazol-1-yl, such as 3-carbamoyl-1,2,4-triazol-1-yl, pyrazol-1-yl, 3-trifluoromethyl-pyrazol-1-yl, 3-(halophenyl)-pyrazol-1-yl, e.g. 3-(4-chlorophenyl)-pyrazol-1-yl, pyrrolidin-1-yl, 2-oxo-pyrrolidin-1-yl, piperidin-1-yl, 2-oxopiperidin-1-yl, morpholino, pyridine-(2-, 3- or 4-)yl which is unsubstituted or substituted by cyano, C₁-C₇-alkyl or amino, pyrimidin-(2-, 4- or 5-)yl, pyrazinyl, benzimidazalyl, C₁-C₇-alkoxy-substituted benzimidazolyl, benzothiazolyl, amino-substituted benzothiazolyl, pyrrolo-pyrimidinyl, especially pyrrolo[2,3-d]pyrimidin-yl, C₁-C₇-alkyl-substituted pyrrolo-pyrimidinyl and 1H,4H,5H-trihydropyrazolo[2,3-c]piperidin-1-yl which is unsubstituted or substituted by 1 or 2 substituents independently selected from C₁-C₇-alkyl and halo-C₁-C₇-alkyl, or further from phenyl that is unsubstituted or substituted by one or more moieties independently selected from the group consisting of halo, C₁-C₇-alkoxy and C₁-C₇-alkanesulfonyl, from tetrazol-5-yl, from indolyl, from indazolyl, from C₁-C₇-alkyl-indazoylyl, from pyrrolo-pyridinyl and from azetidin-2-one; and
R² is phenyl that is substituted by 1 to 3, preferably 1 or 2 substituents selected from the group consisting of halo, C₁-C₇-alkoxy, hydroxyl, C₁-C₇-alkoxyphenyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, C₁-C₇-alkoxycarbonyl, amino-C₁-C₇-alkoxy, pyrrolidinyl-C₁-C₇-alkoxy, piperidinyl-C₁-C₇-alkoxy, morpholinyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl and C₃-C₈-cyloalkyl-sulfonyl;
preferably with the proviso that phenyl R² is substituted in meta position by C₁-C₇-alkoxy, especially methoxy, and in para-position by C₁-C₇-alkoxy, hydroxyl, C₁-C₇-alkoxy-C₁-C₇-alkoxy, C₁-C₇-alkoxy-C₁-C₇-alkoxy-C₁-C₇-alkoxy, amino-C₁-C₇-alkoxy, N-mono- or N,N-di-(C₁-C₇-alkyl)-amino-C₁-C₇-alkoxy, pyrrolidinyl-C₁-C₇-alkoxy, piperidinyl-C₁-C₇-alkoxy, morpholinyl-C₁-C₇-alkoxy, thiomorpholinyl-C₁-C₇-alkoxy, S-oxo-thiomorpholinyl-C₁-C₇-alkoxy, S,S-dioxothiomorpholinyl-C₁-C₇-alkoxy, piperazinyl-C₁-C₇-alkoxy, N'-C₁-C₇-alkyl-piperazino-C₁-C₇-alkoxy, C₃-C₈-cyloalkoxy, C₁-C₇-alkane-sulfonyl or C₃-C₈-cyloalkyl-sulfonyl; where in one preferred embodiment R² is 3,4-dimethoxyphenyl;
or an N-oxide thereof, a solvate and/or a (preferably pharmaceutically acceptable) salt thereof.

4. A compound of the formula I according to claim 1, wherein
R¹ is phenyl, (especially 3- or 4-) halophenyl, (especially 3- or 4-) C₁-C₇-alkoxyphenyl, halo- and C₁-C₇-alkoxy-substituted phenyl, especially 3-halo-4-C₁-C₇-alkoxyphenyl or 4-halo-3-C₁-C₇-alkoxyphenyl, halo-, C₁-C₇-alkoxy- and C₁-C₇-alkyl-substituted phenyl, such as 2-halo-4-C₁C₇-alkoxy-5-C₁-C₇-alkyl-phenyl, (especially 3- or 4-) aminophenyl, pyrrolidin-2-carbonyl-amino-phenyl, (especially 3- or 4-) mono- or di-(C₁-C₇-alkyl)phenyl, (especially 3- or 4-) C₁-C₇-alkanesulfonyl-phenyl, azetidihe-1-sulfonyl-phenyl, (especially 3- or 4-) sulfamoyl-phenyl, (especially 3- or 4-) N-mono-(C₁-C₇-alkyl)-sulfamoyl-phenyl, cyclopropyl-sulfamoyl-phenyl, 2-hydroxy-ethyl-sulfamoyl-phenyl, (especially 3-or 4-) cyanophenyl, (especially 3- or 4-) nitrophenyl, (especially 4-) 1,2,4-triazol-1-yl-phenyl, (especially 4-) pyrazol-1-yl-phenyl, (especially 4-) (3-trifluoromethyl-pyrazol-1-yl)-phenyl, (especially 4-) 2-oxo-pyrrolidin-1-yl-phenyl, (especially 4-) 2-oxopiperidin-1-yl-phenyl, (especially 4-) morpholino-phenyl, (especially 3- or 4-)piperazin-1-yl-phenyl, (especially 3- or 4-) 4-(C₁-C₇-alkyl)-piperazin-1-yl-phenyl, (especially 4-) pyridine-(2-, 3- or 4-)yl-phenyl, cyano-pyridyl-phenyl, methyl-pyridyl-phenyl, amino-pyridyl-phenyl, (especially 4-) pyrimidin-(2-, 4- or 5-)yl-phenyl, pyrazinyl-phenyl, azetidin-2-one-phenyl, or (especially 3- or 4-)benzimidazol-1-yl-phenyl, and
R² is 4-methane-sulfonylphenyl, 4'-methoxy-biphenyl, 4-(3-amino-propoxy)-3-methoxyphenyl, 3,4-di-C₁-C₇-alkoxy-phenyl, especially 3,4-dimethoxyphenyl or 4-ethoxy-3-methoxyphenyl,
or an N-oxide thereof, a solvate and/or a (preferably pharmaceutically acceptable) salt thereof.

5. A compound of the formula I according to claim 1 selected from the group of compounds with the following names:
6-(3,4-dimethoxy-phenyl)-2-methyl-3-(4-[1,2,4]triazol-1-yl-phenyl)-imidazo[1,2-b]pyridazine;
6-(3,4-dimethoxy-phenyl)-2-methyl-3-(4-nitro-phenyl)-imidazo[1,2-b]pyridazine;
6-(3,4-dimethoxy-phenyl)-2-methyl-3-(4-pyrazol-1-yl-phenyl)-imidazo[1,2-b]pyridazine;
3-(4-benzoimidazol-1-yl-phenyl)-6-(3,4-dimethoxy-phenyl)-2-methyl-imidazo[1,2-b]pyridazine;
4-[6-(3,4-dimethoxy-phenyl)-2-methyl-imidazo[1,2-b] pyridazin-3-yl]-benzonitrile;
6-(3,4-dimethoxy-phenyl)-2-methyl-3-(4-pyridin-3-yl-phenyl)-imidazo[1,2-b]pyridazine;
6-(3,4-dimethoxy-phenyl)-2-methyl-3-[4-(1H-tetrazol-5-yl)-phenyl]-imidazo[1,2-b]pyridazine;
6-(3,4-dimethoxy-phenyl)-3-(4-methanesulfonyl-phenyl)-2-methyl-imidazo[1,2-b]pyridazine;
6-(3,4-dimethoxy-phenyl)-2-methyl-3-(4-pyrimidin-5-yl-phenyl)-imidazo[1,2-b]pyridazine;
6-(3,4-dimethoxy-phenyl)-3-(4-iodo-phenyl)-2-methyl-imidazo[1,2-b]pyridazine;
6-(3,4-dimethoxy-phenyl)-3-(3-fluoro-4-methoxy-phenyl)-2-methyl-imidazo[1,2-b]pyridazine;
4-[6-(3,4-dimethoxy-phenyl)-2-methyl-imidazo[1,2-b]pyridazin-3-yl]-phenylamine;
{4-[6-(3,4-dimethoxy-phenyl)-2-methyl-imidazo[1,2-b]pyridazin-3-yl]-phenyl}-methyl-amine;
6-(3,4-dimethoxy-phenyl)-2-methyl-3-[4-(3-trifluoromethyl-pyrazol-1-yl)-phenyl]-imidaza[1,2-b]pyridazine;
1-{4-[6-(3,4-dimethoxy-phenyl)-2-methyl-imidazo[1,2-b]pyridazin-3-yl]-phenyl}-piperidin-2-one;
6-(3,4-dimethoxy-phenyl)-2-methyl-3-(4-morpholin-4-yl-phenyl)-imidazo[1,2-b]pyridazine;
1-{4-[6-(3,4-dirnethoxy-phenyl)-2-methyl-imidazo[1,2-b]pyridazin-3-yl]-phenyl}-pyrrolidin-2-one;
6-(3,4-dimethoxy-phenyl)-2-methyl-3-[4-(4-methyl-piperazin-1-yl)-phenyl]-imidazo[1,2-b]pyridazine,
{4-[6-(3,4-dimethoxy-phenyl)-2-methyl-imidazo[1,2-b]pyridazin-3-yl]-phenyl}-morpholin-4-yl-methanone
4-[6-(3,4-dimethoxy-phenyl)-2-methyl-imidazo[1,2-b]pyridazin-3-yl]-N,N-dimethyl-benzenesulfonamide
4-[6-(3,4-dimethoxy-phenyl)-2-methyl-imidazo[1,2-b]pyridazin-3-yl]-benzamide
3-(2-chloro-4-methoxy-5-methyl-phenyl)-6-(3,4-dimethoxy-phenyl)-2-methyl-imidazo[1,2-b]pyridazine
6-(3,4-dimethoxy-phenyl)-3-(4-ethanesulfonyl-phenyl)-2-methyl-imidazo[1,2-b]pyridazine
3-[6-(3,4-Dimethoxy-phenyl)-2-methyl-imidazo[1,2-b]pyridazin-3-yl]-N,N-dimethyl-benzenesulfonamide
4-[6-(3,4-Dimethoxy-phenyl)-2-methyl-imidazo[1,2-b]pyridazin-3-yl]-N-methyl-benzenesulfonamide,
4-[6-(3,4-Dimethoxy-phenyl)-2-methyl-imidazo[1,2-b] pyridazin-3-yl]-benzenesulfonamide,
{4-[6-(3,4-Dimethoxy-phenyl)-2-methyl-imidazo[1,2-b] pyridazin-3-yl]-phenyl}-acetonitrile,
3-{4-[6-(3,4-Dimethoxy-phenyl)-2-methyl-imidazo-[1,2-b]pyridazin-3-yl]-phenyl}-propan-1-ol,
4-[6-(3,4-Dimethoxy-phenyl)-2-methyl-imidazo[1,2-b]pyridazin-3-yl]-N-ethyl-benzenesulfonamide,
6-(3,4-Dimethoxy-phenyl)-2-methyl-3-[4-(propane-2-sulfonyl)-phenyl]-imidazo[1,2-b]pyridazine,
N-Cyclopropyl-4-[6-(3,4-dimethoxy-phenyl)-2-methyl-imidazo[1,2-b]pyridazin-3-yl]-benzenesulfonamide,
4-[6-(3,4-Dimethoxy-phenyl)-2-methyl-imidazo[1,2-b]pyridazin-3-yl]-N,N-diethyl-benzenesulfonamide,
3-[B-(3,4-Dimethoxy-phenyl)-2-methyl-imidazo[1,2-b]pyridazin-3-yl]-N-methyl-benzamide,
3-[4-(Azetidine-1-sulfonyl)-phenyl]-6-(3,4-dimethoxy-phenyl)-2-methyl-imidazo[1,2-b]pyridazine
6-(3,4-Dimethoxy-phenyl)-3-(3-methanesulfonyl-phenyl)-2-methyl-imidazo[1,2-b]-pyridazine,
4-[6-(3,4-Dimethoxy-phenyl)-2-methyl-imidazo[1,2-b]-pyridazin-3-yl]-N-(2-hydroxy-ethyl)-benzenesulfonamide,
3-[6-(3,4-Dimethoxy-phenyl)-2-methyl-imidazo[1,2-b]pyridazin-3-yl]-benzamide,
5-{4-[6-(3,4-Dimethoxy-phenyl)-2-methyl-imidazo[1,2-b]pyridazin-3-yl]-phenyl}-nicotinonitrile,
6-(3,4-Dimethoxy-phenyl)-2-methyl-3-(4-pyrazin-2-yl-phenyl)-imidazo[1,2-b]pyridazine,
{4'-[6-(3,4-Dimethoxy-phenyl)-2-methyl-imidazo[1,2-b]pyridazin-3-yl]-biphenyl-4-yl}-acetonitrile,
5-{4-[6-(3,4-Dimethoxy-phenyl)-2-methyl-imidazo[1,2-b]pyridazin-3-yl]-phenyl}-pyridine-2-carbonitrile,
6-(3,4-Dimethoxy-phenyl)-2-methyl-3-[4-(3-methyl-pyridin-2-yl)-phenyl] -imidazo[1,2-b]pyridazine,
3-(4-Benzothiazol-2-yl-phenyl)-6-(3,4-dimethoxy-phenyl)-2-methyl-imida zo[1,2-b]pyridazine,
1-{4-[6-(3,4-Dimethoxy-phenyl)-2-methyl-imidazo[1,2-b]pyridazin-3-yl]-phenyl}-azetidin-2-one,
(R)-Fyrrolidine-2-carboxylic acid {4-[6-(3,4-dimethoxy-phenyl)-2-methyl-imidazo[1,2-b]pyridazin-3-yl]-phenyl}-amide,
1-{4-[6-(3,4-Dimethoxy-phenyl)-2-methyl-imidazo[1,2-b]pyridazin-3-yl]-phenyl}-1H-[1,2,4]triazoEe-3-carboxylic acid amide,
2-{4-[6-(3,4-Dimethoxy-phenyl)-2-methyl-imidazo[1,2-b]pyridazin-3-yl]-phenyl}-benzothiazol-5-ylarnine,
5-{4-[6-(3,4-Dimethoxy-phenyl)-2-methyl-imidazo[1,2-b]pyridazin-3-yl]-phenyl}-pyridin-2-ylamine,
3,6-Bis-(4-methanesulfonyl-phenyl)-2-methyl-imidazo[1,2-b]pyridazine,
3,6-Bis-(4'-methoxy-biphenyl-4-yl)-2-methyl-imidazo[1,2-b]pyridazine,
6-(4-Ethoxy-3-methoxy-phenyl)-3-(4-methanesulfonyl-phenyl)-2-methyl-imidazo[1,2-b]pyridazine,
4-[6-(4-Ethoxy-3-methoxy-phenyl)-2-methyl-imidazo[1,2-b]pyridazin-3-yl]-N-methyl-benzenesulfonamide,
3-(4-Methanesulfonyl-phenyl)-6-(4'-methoxy-biphenyl-4-yl)-2-methyl-imidazo[1,2-b]pyridazine,
4-(6-(4'-methoxybiphenyl-4-yl)-2-methylimidazo[1,2-b]pyridazin-3-yl)-N-methylbenzenesulfonamide,
4-[6-(4-Methanesulfonyl-phenyl)-2-methyl-imidazo[1,2-b]pyridazin-3-yl]-N-methyl-benzenesulfonamide,
3-{4-[3-(4-Methanesulfonyl-phenyl)-2-methyl-imidazo[1,2-b]pyridazin-6-yl]-2-methoxy-phenoxy}-propylamine,
(3-{4-[3-(4-Dimethylsulfamoyl-phenyl)-2-methyl-imidazo[1,2-b]pyridazin-6-yl]-2-methoxy-phenoxy}-propyl)-carbamic acid tert-butyl ester,
4-{6-[4-(3-Amino-propoxy)-3-methoxy-phenyl]-2-methyl-imidazo[1,2-b]pyridazin-3-yl}-N,N-dimethyl-benzenesulfonamide,
3-{4-[3-(4-Ethanesulfonyl-phenyl)-2-methyl-imidazo[1,2-b]pyridazin-6-yl]-2-methoxy-phenoxy}-propylamine,
(3-{4-[3-(4-Ethanesulfonyl-phenyl)-2-methyl-imidazo[1,2-b]pyridazin-6-yl]-2-methoxy-phenoxy}-propyl)-carbamic acid tert-butyl ester,
2-{4-[3-(4-Ethanesulfonyl-phenyl)-2-methyl-imidazo[1,2-b]pyridazin-6-yl]-2-methoxy-phenaxy}-ethylamine,
2-{4-[3-(4-Methanesulfonyl-phenyl)-2-methyl-imidazo[1,2-b]pyridazin-6-yl]-2-methoxy-phenoxy}-ethylamine,
(2-{4-[3-(4-Methanesulfonyl-phenyl)-2-methyl-imidazo[1,2-b]pyridazin-6-yl]-2-methoxy-phenoxy}-ethyl)-carbamic acid tert-butyl ester,
4-{6-[4-(2-Amino-ethoxy)-3-methoxy-phenyl]-2-methyl-imidazo[1,2-b]pyridazin-3-yl}-N,N-dimethyl-benzenesulfonamide,
(2-{4-[3-(4-Dimethylsnlfamoyl-phenyl)-2-methyl-imidazo[1,2-b]pyridazin-6-yl]-2-methoxy-phenoxy}-ethyl)-carbamic acid tert-butyl ester
or an N-oxide thereof, a solvate and/or a pharmaceutically acceptable salt thereof.

6. A compound of the formula I according to claim 1, selected from the group of compounds with the following formulae: 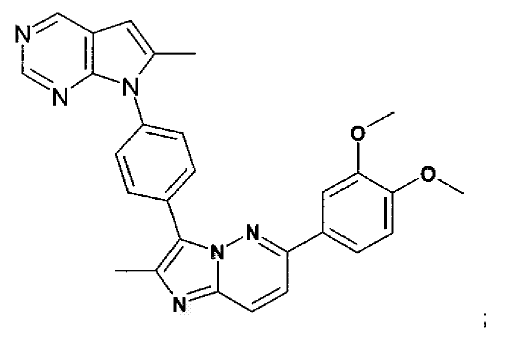 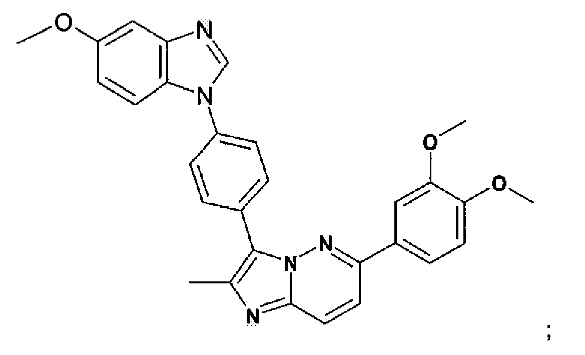 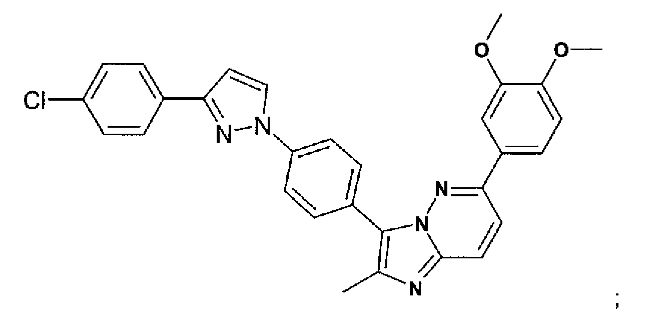 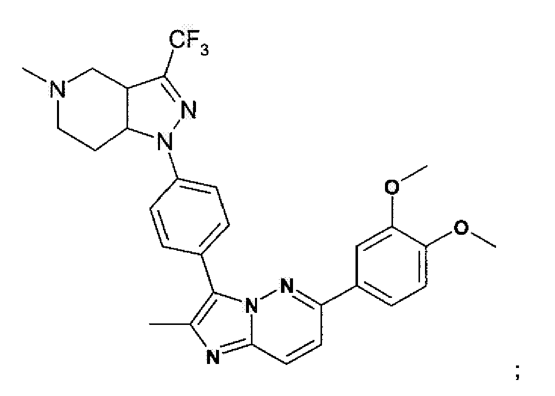 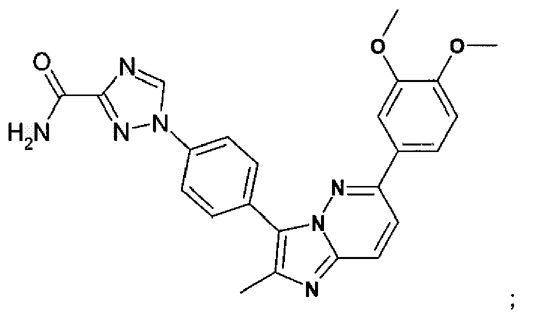 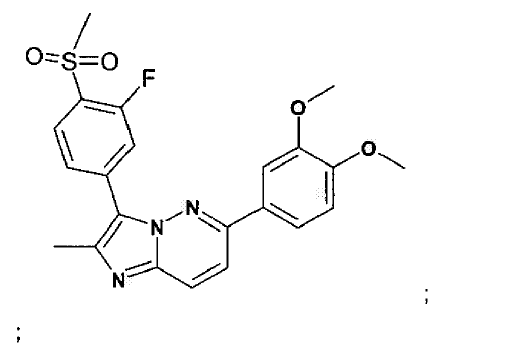 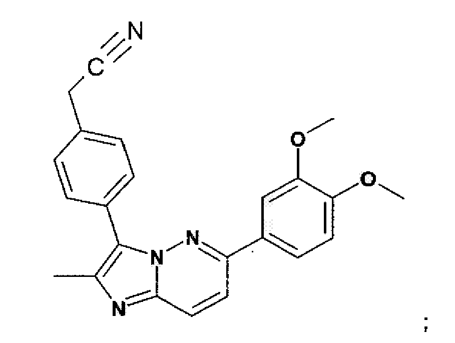 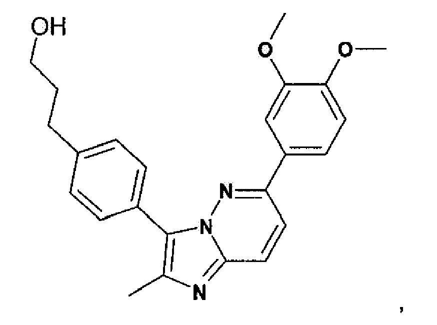 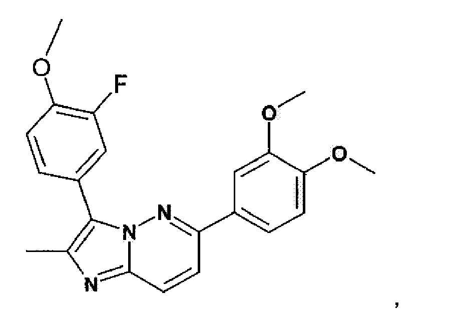 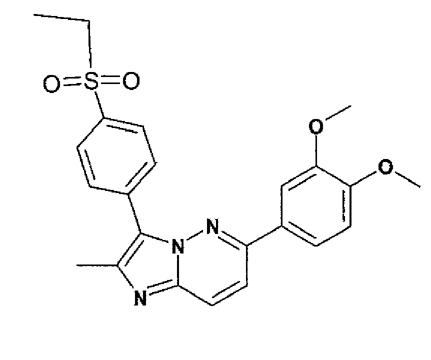 and 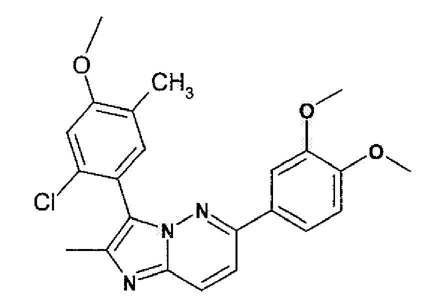 or an N-oxide thereof, a solvate and/or a pharmaceutically acceptable salt thereof.

7. A compound of the formula I, an N-oxide thereof, a tautomer thereof and/or a pharmaceutically acceptable salt thereof, according to any one of claims 1 to 6 for use in the treatment, including prophylactic treatment, of a warm-blooded animal, especially a human.

8. A compound of the formula I, an N-oxide thereof, a tautomer thereof and/or a pharmaceutically acceptable salt thereof, according to claim 7 where the use is against one or more diseases selected from the group consisting of proliferative, inflammatory diseases, allergic diseases, obstructive airways diseases, and disorders commonly occurring in connection with transplantation, especially one or more diseases which respond to an inhibition of kinases of the PI3-kinase-related protein kinase family, especially lipid kinases and/or PI3 kinase (PI3K) and/or mTOR and/or DNA protein kinase and/or ATM and/or ATR and/or hSMG-1 activity.

9. A pharmaceutical preparation, comprising a compound of the formula I, an N-oxide thereof, a tautomer thereof and/or a pharmaceutically acceptable salt thereof, according to any one of claims 1 to 8 and at least one pharmaceutically acceptable carrier.

10. A method or process for the manufacture of a pharmaceutical preparation, comprising mixing a compound of the formula I, an N-oxide thereof, a tautomer thereof and/or a pharmaceutically acceptable salt thereof, according to any one of claims 1 to 8 with at least one pharmaceutically acceptable carrier material.

11. A process for the manufacture of a compound according to any one of claims 1 to 6, said process comprising
a) reacting a compound of the formula II, 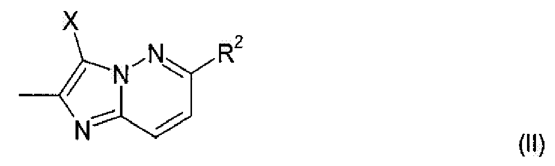 wherein R² is as defined for a compound of the formula I in claim 1 and X is halo, preferably chloro, bromo or iodo, or is trifluoromethansulfonyloxy, under cross-coupling conditions with a boronic acid or boronic acid ester of the formula III,
R¹-D (III)
wherein R¹ is as defined for a compound of the formula I in claim 1 and is bound via a carbon atom to D and D is -B(OH₂) in free form or in esterified form, e.g. as a group of the formula A, 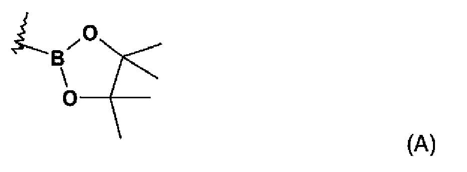 or
b) reacting a boronic acid or boronic acid ester compound of the formula IV, 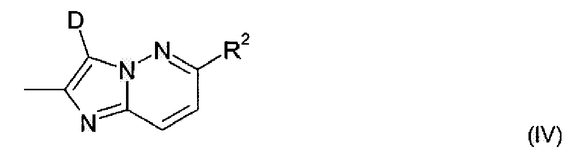 wherein R² is as defined for a compound of the formula I in claim 1 and D is -B(OH₂) in free form or in esterified form, e.g. as a group of the formula A shown under a), under cross-coupling conditions with a compound of the formula V,
R¹-X (V)
wherein R¹ is as defined for a compound of the formula I in claim 1 and X is halogen, especially chloro, bromo or iodo, or trifluoromethansulfonyloxy,
or
c) reacting a compound of the formula VI, 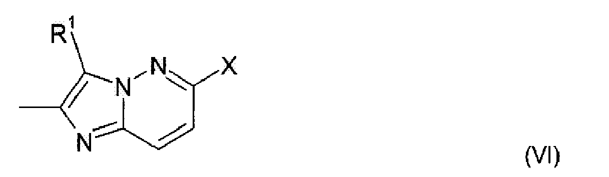 wherein R¹ is as defined for a compound of the formula I in claim 1 and X is halo, especially chloro, bromo or iodo, or is trifluoromethansulfonyloxy, under cross-coupling conditions with a boronic acid or boronic acid ester of the formula VII,
R²-D (VII)
wherein R² is as defined for a compound of the formula I in claim 1 and D is -B(OH₂) in free form or in esterified form, e.g. as a group of the formula A shown under a),
or
d) reacting a pyridazine compound of the formula VIII, 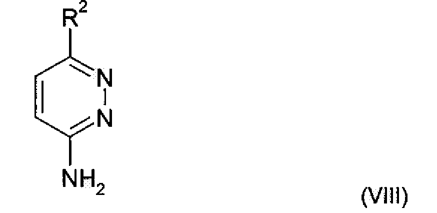
wherein R² is as defined for a compound of the formula I in claim 1, with a haloketone of the formula IX, 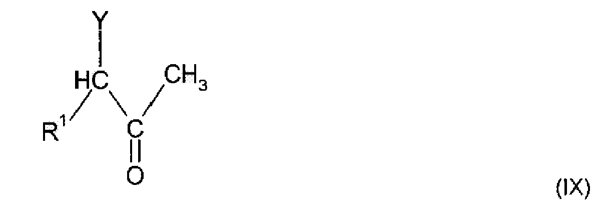 wherein R¹ is as defined for a compound of the formula I in claim 1 and Y is halo, especially chloro or bromo,
and, if desired, a compound of the formula I obtainable according to any one of the reactions a) to d) given above is converted into a different compound of the formula I, an obtainable salt of a compound of the formula I is converted into a different salt thereof, an obtainable free compound of the formula I is converted into a salt thereof, and/or an obtainable isomer of a compound of the formula I is separated from one or more different obtainable isomers of the formula I.
